# EUROPEAN PATENT SPECIFICATION

(11) **EP 2 333 107 B1**
(45) Date of publication and mention of the grant of the patent: **23.04.2014**
(21) Application number: 09800092.0
(22) Date of filing: 22.07.2009
(51) Int. Cl.: C12Q 1/68

(54) **GENE EXPRESSION PROFILE AS AN ENDOMETRIAL RECEPTIVITY MARKER**
GENEXPRESSIONSPROFIL ALS ENDOMETRIALER REZEPTIVITÄTSMARKER
PROFIL D'EXPRESSION GÉNIQUE UTILISÉ COMME MARQUEUR DE LA RÉCEPTIVITÉ ENDOMÉTRIALE

(30) Priority: 22.07.2008 WO PCT/ES2008/000513
(43) Date of publication of application: 15.06.2011
(73) Proprietor: Equipo Ivi Investigacion SL, 46015 Valencia (ES)
(72) Inventor: SIMÓN VALLES, Carlos, Godella 46110 Valencia (ES); HORCAJADAS ALMANSA, JoséAntonio, Valencia 46009 (ES); DIAZ GIMENO, Patricia, 46014 Valencia (ES); PELLICER MARTÍNEZ, Antonio, 46004 Valencia (ES)
(74) Representative: Buchet, Anne
(86) International application number: PCT/ES2009/000386
(87) International publication number: WO 2010/010213

(56) References cited:
- WO-A1-2005/018796
- US-A1- 2004 005 612
- AGILENT TECHNOLOGIES: "Agilent 012391 Whole Genome Oligo Microarray G4112A (Feature Number Version)", GEO , 17 November 2004 (2004-11-17), XP002513552, Retrieved from the Internet: URL:http://www.ncbi.nlm.nih.gov/geo/query/ acc.cgi?acc=GPL1708
- MIRKIN S ET AL: "In search of candidate genes critically expressed in the human endometrium during the window of implantation", HUMAN REPRODUCTION, OXFORD UNIVERSITY PRESS, GB, vol. 20, no. 8, 1 August 2005 (2005-08-01) , pages 2104-2117, XP002595771, ISSN: 0268-1161, DOI: 10.1093/HUMREP/DEI051 [retrieved on 2005-05-05]
- HORCAJADAS J A ET AL: "Wide genomic analysis of human endometrial receptivity: new times, new opportunities", HUMAN REPRODUCTION UPDATE, vol. 13, no. 1, January 2007 (2007-01), pages 77-86, XP002668651, ISSN: 1355-4786
- TAPIA ALEJANDRO ET AL: "Differences in the endometrial transcript profile during the receptive period between women who were refractory to implantation and those who achieved pregnancy", HUMAN REPRODUCTION (OXFORD), vol. 23, no. 2, February 2008 (2008-02), pages 340-351, XP002668652, ISSN: 0268-1161
- RIESEWIJK, A. ET AL.: 'Gene expression profiling of human endometrial receptivity on days LH+2 versus LH+7 by microarray technology.' MOLECULAR HUMAN REPRODUCTION. vol. 9, no. 5, May 2003, pages 253 - 264, XP008041260
- CARSON, D. D. ET AL.: 'Changes in gene expression during the early to mid-luteal (receptive phase) transition in human endometrium detected by high-density microarray screening.' MOLECULAR HUMAN REPRODUCTION. vol. 8, no. 9, September 2002, pages 871 - 879, XP002595770
- BORTHWICK, J. M. ET AL.: 'Determination of the transcript profile of human endometrium.' MOLECULAR HUMAN REPRODUCTION. vol. 9, no. 1, January 2003, pages 19 - 33, XP008142241
- TALBI, S. ET AL.: 'Molecular phenotyping of human endometrium distinguishes menstrual cycle phases and underlying biological processes in normo ovulatory women.' ENDOCRINOLOGY. vol. 147, no. 3, March 2006, pages 1097 - 1121, XP002595772
- BURNEY, R. O. ET AL.: 'Gene expression analysis of endometrium reveals progesterone resistance and candidate susceptibility genes in women with endometriosis.' ENDOCRINOLOGY. vol. 148, no. 8, August 2007, pages 3814 - 3826, XP008142244
- HORCAJADAS, J. A. ET AL.: 'Effect of an intrauterine device on the gene expression profile of the endometrium.' THE JOURNAL OF CLINICAL ENDOCRINOLOGY & METABOLISM. vol. 91, no. 8, August 2006, pages 3199 - 3207, XP008142081

### SEQUENCE LISTING

<110> EQUIPO IVI INVESTIGACION SL
<120> **Gene expression profile as an endometrial receptivity marker**
<130> AX0090139WO
<150> PCT/ES2008/000513
   <151> 2008-07-22
<160> 579
<170> PatentIn version 3.3
<210> 1
   <211> 21
   <212> DNA
   <213> Artificial
<220>
   <223> **Forward primer** CLDN 10
<400> 1
   ctggaaggtg tctaccatcg a 21
<210> 2
   <211> 19
   <212> DNA
   <213> Artificial
<220>
   <223> **Reverse primer** CLDN10
<400> 2
   aaagaagccc aggctgaca 19
<210> 3
   <211> 21
   <212> DNA
   <213> Artificial
<220>
   <223> **Forward primer** SPP1
<400> 3
   taaaccctga cccatctcag a 21
<210> 4
   <211> 21
   <212> DNA
   <213> Artificial
<220>
   <223> **Reverse primer** SPP1
<400> 4
   tgagactcat cagactggtg a 21
<210> 5
   <211> 21
   <212> DNA
   <213> Artificial
<220>
   <223> **Forward primer** FXYD2
<400> 5
   aatgactggg ttgtcgatgg a 21
<210> 6
   <211> 20
   <212> DNA
   <213> Artificial
<220>
   <223> **Reverse primer** FXD2
<400> 6
   acagcggaat cttctgctga 20
<210> 7
   <211> 20
   <212> DNA
   <213> Artificial
<220>
   <223> **Forward primer** MT1G
<400> 7
   tcctgcaagt gcaaagagtg 20
<210> 8
   <211> 20
   <212> DNA
   <213> Artificial
<220>
   <223> **Reverse primer** MT1G
<400> 8
   ggaatgtagc aaaggggtca 20
<210> 9
   <211> 20
   <212> DNA
   <213> Artificial
<220>
   <223> **Forward primer** GPX3
<400> 9
   aggtggaggc tttgtcccta 20
<210> 10
   <211> 20
   <212> DNA
   <213> Artificial
<220>
   <223> **Reverse primer** GPX3
<400> 10
   tataccatct ggccccacca 20
<210> 11
   <211> 19
   <212> DNA
   <213> Artificial
<220>
   <223> **Forward primer** GAPDH
<400> 11
   gaaggtgaag gtcggagtc 19
<210> 12
   <211> 20
   <212> DNA
   <213> Artificial
<220>
   <223> **Reverse primer** GAPDH
<400> 12
   gaagatggtg atgggatttc 20
<210> 13
   <211> 60
   <212> DNA
   <213> Artificial
<220>
   <223> A_23_P207507
<400> 13
   gtaactgtgc tgaatgcttt agatgaggaa atgatcccca agtggtgaat gacacgccta 60
<210> 14
   <211> 60
   <212> DNA
   <213> Artificial
<220>
   <223> A_01_P004704
<400> 14
   ggcgagattg aagggctttt gttattgttg ttggatattt ttgtttccca taaaagcaca 60
<210> 15
   <211> 60
   <212> DNA
   <213> Artificial
<220>
   <223> A_24_P123408
<400> 15
   tttgaccgca gcatgcacaa gctccaaagt ggaattggcc ggctgattct gaaggaagaa 60
<210> 16
   <211> 60
   <212> DNA
   <213> Artificial
<220>
   <223> A_23_P256205
<400> 16
   tttctggaag agattgcatc tgaggaaatt caggaaggat ctttgtagat tggggggaga 60
<210> 17
   <211> 60
   <212> DNA
   <213> Artificial
<220>
   <223> A_23_P256204
<400> 17
   tgtgttctca tgtaggatgt cagccctccc tgcaacttct ctttttggcc aatgtctttt 60
<210> 18
   <211> 60
   <212> DNA
   <213> Artificial
<220>
   <223> A_24_P189516
<400> 18
   aagccattca taaaagaagc gtcaatggcc aaatactatg catcagagat tgcaggacaa 60
<210> 19
   <211> 60
   <212> DNA
   <213> Artificial
<220>
   <223> A_23_P158570
<400> 19
   caacatccag ttgaacacca ttgcaaagca tatcgatgca gaatactgac gtctatagga 60
<210> 20
   <211> 60
   <212> DNA
   <213> Artificial
<220>
   <223> A_32_P31945
<400> 20
   aattcagaaa ttgggttttg gttcagtgat tctcaagaaa aagatctctt gcccattaag 60
<210> 21
   <211> 60
   <212> DNA
   <213> Artificial
<220>
   <223> A_23_P150053
<400> 21
   atcatgaagt gtgatattga catcaggaag gacctctatg ctaacaatgt cctatcaggg 60
<210> 22
   <211> 60
   <212> DNA
   <213> Artificial
<220>
   <223> A_01_P006531
<400> 22
   accgcaaatg cttctaaaac actttcccgc tcctctctgt ctctagcaca caactgtgaa 60
<210> 23
   <211> 60
   <212> DNA
   <213> Artificial
<220>
   <223> A_23_P342275
<400> 23
   acctctgagc agtgatatag cataataaag ccccgggcat tattattatt atttcttttg 60
<210> 24
   <211> 60
   <212> DNA
   <213> Artificial
<220>
   <223> A_23_P211039
<400> 24
   tatgggggta gatagaaaag gagttgaatc atcagagtaa actgccagtt gcaaatttga 60
<210> 25
   <211> 60
   <212> DNA
   <213> Artificial
<220>
   <223> A_01_P013378
<400> 25
   ctttataagt attggtttgg gtgttccttc caagaaggac tatagttagt aataaatgcc 60
<210> 26
   <211> 60
   <212> DNA
   <213> Artificial
<220>
   <223> A_01_P016559
<400> 26
   gaagaggaga ggggatgggg cgacagatcc tatcatcaac tgtccagtgg actggacctt 60
<210> 27
   <211> 60
   <212> DNA
   <213> Artificial
<220>
   <223> A_23_P86956
<400> 27
   caccaattat ggctacaatg acattgtcac catcccagct ggtgccacta atattgacgt 60
<210> 28
   <211> 60
   <212> DNA
   <213> Artificial
<220>
   <223> A_23_P138706
<400> 28
   atcatgtcat tgatgaactg ccaaagtcag gggaggaggg cagagacttt gtgtttacat 60
<210> 29
   <211> 60
   <212> DNA
   <213> Artificial
<220>
   <223> A_01_P002991
<400> 29
   actacaagga catggctcac aaaaggttaa tggatggtta cctagaggat tacctagccc 60
<210> 30
   <211> 60
   <212> DNA
   <213> Artificial
<220>
   <223> A_23_P31407
<400> 30
   ctcctcaatc tggtttatga aacaactgac aaacaccttt ctcctgatgg ccagtatgtc 60
<210> 31
   <211> 60
   <212> DNA
   <213> Artificial
<220>
   <223> A_01_P005774
<400> 31
   ttttttctct ttttggtggt ggttaaaagg gaacacaaaa catttaaata aaactttcca 60
<210> 32
   <211> 60
   <212> DNA
   <213> Artificial
<220>
   <223> A_23_P97043
<400> 32
   tcctcggaag acactctgac cgtggtcact gcggaccatt cccacgtctt cacatttggt 60
<210> 33
   <211> 60
   <212> DNA
   <213> Artificial
<220>
   <223> A_24_P353619
<400> 33
   gctacaaggt ggtgggcggt gaacgagaga atgtctccat ggtggactat gctcacaaca 60
<210> 34
   <211> 60
   <212> DNA
   <213> Artificial
<220>
   <223> A_23_P97046
<400> 34
   acctggaaga gcttcaaacc gagatacaag cactcccact tcatctggaa ccgcacggaa 60
<210> 35
   <211> 60
   <212> DNA
   <213> Artificial
<220>
   <223> A_23_P14083
<400> 35
   tcttagcttt tagcactatt ggtaatttca gagtaggccc aaaggtgata tgactcccat 60
<210> 36
   <211> 60
   <212> DNA
   <213> Artificial
<220>
   <223> A_23_P428738
<400> 36
   ggcccaaaga aagagctacc tggacctttt gttttctgtt tgacaacatg tttaataaat 60
<210> 37
   <211> 60
   <212> DNA
   <213> Artificial
<220>
   <223> A_01_P018201
<400> 37
   agagctacct ggaccttttg ttttctgtct gacaacatgt ttaataaata aaaatgtcac 60
<210> 38
   <211> 60
   <212> DNA
   <213> Artificial
<220>
   <223> A_23_P202269
<400> 38
   gtgtcatatt aggttaataa ggctgctgtg ttttaaaggg catttttatt tgggttttgg 60
<210> 39
   <211> 60
   <212> DNA
   <213> Artificial
<220>
   <223> A_23_P301530
<400> 39
   ctcgtaacag cgaacggtca gtcaagggat cataagtttt tactgccagt attgagaaat 60
<210> 40
   <211> 60
   <212> DNA
   <213> Artificial
<220>
   <223> A_24_P162173
<400> 40
   acttttattc tttcaaagaa aatagactgc cattttccat caagattaga gacaccagcc 60
<210> 41
   <211> 60
   <212> DNA
   <213> Artificial
<220>
   <223> A_01_P007944
<400> 41
   atcagcattc attgacacat agctctaatg acatatgtat gaaaaaccat acactggatg 60
<210> 42
   <211> 60
   <212> DNA
   <213> Artificial
<220>
   <223> A_32_P33304
<400> 42
   tgttggaata ccggcggtga tctgtctttt ataaactcac ctgatttaaa ggaaagatga 60
<210> 43
   <211> 60
   <212> DNA
   <213> Artificial
<220>
   <223> A_32_P33309
<400> 43
   agcacccgta aatggacttt ggtctcaatg ctttgactct ttgccgtggt tttggatgtt 60
<210> 44
   <211> 60
   <212> DNA
   <213> Artificial
<220>
   <223> A_23_P16976
<400> 44
   aattaaaacg cctacagctg cctcctagaa tatagactgt ctgtattatt attcacctat 60
<210> 45
   <211> 60
   <212> DNA
   <213> Artificial
<220>
   <223> A_01_P009551
<400> 45
   ctttctgctg tcaccttcgt cttgtcagaa tgaatataga cactgtatct aagtgggacc 60
<210> 46
   <211> 60
   <212> DNA
   <213> Artificial
<220>
   <223> A_23_P154037
<400> 46
   ttggtttcct ctagggtgat attcgtcatt actctgtctc ttcaatccat ccagctaaat 60
<210> 47
   <211> 60
   <212> DNA
   <213> Artificial
<220>
   <223> A_23_P112481
<400> 47
   ctgtccatct gtgcataagg agaggaaagt tccagggtgt gtatgtttca ggggcttcac 60
<210> 48
   <211> 60
   <212> DNA
   <213> Artificial
<220>
   <223> A_23_P112482
<400> 48
   aatttgggtc aatacatcct tttgtctccc aagggaagag aatgggcagc aggtatgtgt 60
<210> 49
   <211> 60
   <212> DNA
   <213> Artificial
<220>
   <223> A_23_P128728
<400> 49
   tttggtcaga caagagaagg agggcatatt gtctatgacc aacttcctac tcccagttca 60
<210> 50
   <211> 60
   <212> DNA
   <213> Artificial
<220>
   <223> A_23_P151075
<400> 50
   tcaggttcca gtagttcatt ctaatgccta gattcttttg tggttgttgc tggcccaatg 60
<210> 51
   <211> 60
   <212> DNA
   <213> Artificial
<220>
   <223> A_01_P018222
<400> 51
   ctagtcacat cccctgccag atggagttct tcttttgtga gagacactgt aaacaacaca 60
<210> 52
   <211> 60
   <212> DNA
   <213> Artificial
<220>
   <223> A_23_P97871
<400> 52
   aagactggca aacagatggc aagggatgcc cctctttttc ataaaactct ccaaggttca 60
<210>. 53
   <211> 60
   <212> DNA
   <213> Artificial
<220>
   <223> A_01_P007755
<400> 53
   tggtgcatga attctcaagt actgtatttc actgtgttgg tgtgtctgat ggaaatttcg 60
<210> 54
   <211> 60
   <212>. DNA
   <213> Artificial
<220>
   <223> A_32_P18440
<400> 54
   gagcacttaa agtccagtgt tggctgttag tgtatttgat attctgcctg tctcctcatg 60
<210> 55
   <211> 60
   <212> DNA
   <213> Artificial
<220>
   <223> A_24_P170667
<400> 55
   agaacgaata ccctatttag gttttaaaca gattaacctt tggactatgt ttcaagctgc 60
<210> 56
   <211> 60
   <212> DNA
   <213> Artificial
<220>
   <223> A_24_P944437
<400> 56
   atgtagagat ccagtgttaa gagttccatt tgcttcaatt aattatttac cttcctgtgg 60
<210> 57
   <211> 60
   <212> DNA
   <213> Artificial
<220>
   <223> A_01_P007754
<400> 57
   atcagggcat atacaaaagg gtttgttaaa actcgatgtt aactttacaa ctttctgacc 60
<210> 58
   <211> 60
   <212> DNA
   <213> Artificial
<220>
   <223> A_23_P52017
<400> 58
   atcacaaatc ccctgcaagc tattcaaatg gtgatggata cgcttggcat tccttattag 60
<210> 59
   <211> 60
   <212> DNA
   <213> Artificial
<220>
   <223> A_32_P231556
<400> 59
   tctttggaag ataacagtga tgtcacaggg ttggctatgt ttattctgaa tcgcctactt 60
<210> 60
   <211> 60
   <212> DNA
   <213> Artificial
<220>
   <223> A_24_P911179
<400> 60
   aggccgagta agagagatta tgacatttct tgtaaatgat gttctgaaac accaagctat 60
<210> 61
   <211> 60
   <212> DNA
   <213> Artificial
<220>
   <223> A_01_P018684
<400> 61
   atcacaaatc ccctgcaagc cattcaaatg gtgatggata cacttggcat tccttattag 60
<210> 62
   <211> 60
   <212> DNA
   <213> Artificial
<220>
   <223> A_01_P018905
<400> 62
   gtggggggca gctgcggtgg ggagctataa aatgacaatt aaaagataca ctagtctttt 60
<210> 63
   <211> 60
   <212> DNA
   <213> Artificial
<220>
   <223> A_23_P31922
<400> 63
   attgttgtga taatttgtaa ttgtgacttg ttctccccgg ctggcagcgt agtggggctg 60
<210> 64
   <211> 60
   <212> DNA
   <213> Artificial
<220>
   <223> A_23_P31921
<400> 64
   tccctggagg atgcctgaat tctacaaccg gttcaagggc cgcaatgacc tgatggagta 60
<210> 65
   <211> 60
   <212> DNA
   <213> Artificial
<220>
   <223> A_32_P13102
<400> 65
   tgtgaaggtg accaacgtca aggatggcac cacccaccag acctccttgg agctcttcat 60
<210> 66
   <211> 60
   <212> DNA
   <213> Artificial
<220>
   <223> A_01_P014301
<400> 66
   aataattgcc aggagtacag tgctcttgtt gatcttgtat tcagtcaggt taaaacaatg 60
<210> 67
   <211> 60
   <212> DNA
   <213> Artificial
<220>
   <223> A_23_P146943
<400> 67
   aactgtcata cgtatgggac ctacacttaa tctctatgct ttacactagc ttctgcattt 60
<210> 68
   <211> 60
   <212> DNA
   <213> Artificial
<220>
   <223> A_23_P62932
<400> 68
   aataattgcc aggagtacag tgctcttgtt gatcttgtat tcagtcaggt taaaacaacg 60
<210> 69
   <211> 60
   <212> DNA
   <213> Artificial
<220>
   <223> A_01_P010458
<400> 69
   tgggtggggg gttgtcatgg gggaactgcc ctttaaattt taagtgacac tacagaaaaa 60
<210> 70
   <211> 60
   <212> DNA
   <213> Artificial
<220>
   <223> A_23_P61960
<400> 70
   tgaagcagag aaacattcac acacaaaaag caacatagtc atgtgggtcc agatggcctc 60
<210> 71
   <211> 60
   <212> DNA
   <213> Artificial
<220>
   <223> A_23_P61956
<400> 71
   atgtgggtcc agatggcctc agtcctagat gttggcaccc tttgctgtgt ctcctcagag 60
<210> 72
   <211> 60
   <212> DNA
   <213> Artificial
<220>
   <223> A_24_P396994
<400> 72
   tatgctcaga tattcatcgt aagtctccct tcacctgtta cagagtttca gatcggtcac 60
<210> 73
   <211> 60
   <212> DNA
   <213> Artificial
<220>
   <223> A_23_P211965
<400> 73
   tttgtcctag gtgtaccctt tcctcatctc tattaaattg taaacaggac tactgcatgt 60
<210> 74
   <211> 60
   <212> DNA
   <213> Artificial
<220>
   <223> A_01_P010392
<400> 74
   gtagaggaaa gggtgtgtga acatggctaa caatctcaaa tacccaaatt gtgatagcat 60
<210> 75
   <211> 60
   <212> DNA
   <213>' Artificial
<220>
   <223> A_23_P67771
<400> 75
   ttgatggatg ctacttctat ttgtggggaa ccttcaaaca ccatccaaag gacaacctta 60
<210> 76
   <211> 60
   <212> DNA
   <213> Artificial
<220>
   <223> A_01_P005782
<400> 76
   gcacggtttg tgagagccca gtcattgtgc tgtttttaat ttttcacatt tttacaaata 60
<210> 77
   <211> 60
   <212> DNA
   <213> Artificial
<220>
   <223> A_23_P57856
<400> 77
   ctgcgttaaa ggctcgattt tgtatctgca ggcagacacg gatctgagaa tctttattga 60
<210> 78
   <211> 60
   <212> DNA
   <213> Artificial
<220>
   <223> A_01_P001583
<400> 78
   tttggctgtg tctaaactgc atcaccgcgt tgtaaaatat agctgtacaa atatcagaat 60
<210> 79
   <211> 60
   <212> DNA
   <213> Artificial
<220>
   <223> A_01_P001085
<400> 79
   atcagcgtcc tatacattga aggtgtgcat atatgttgaa tgacatttta gggacatggt 60
<210> 80
   <211> 60
   <212> DNA
   <213> Artificial
<220>
   <223> A_23_P98350
<400> 80
   tcctatacat cgaaggtgtg catatatgtt gaatgacatt ttagggacat ggtgttttta 60
<210> 81
   <211> 60
   <212> DNA
   <213> Artificial
<220>
   <223> A_01_P000082
<400> 81
   ctaaacaggc tcattacaaa tggttacctt gttatttaac ccatttgtct ctacttttcc 60
<210> 82
   <211> 60
   <212> DNA
   <213> Artificial
<220>
   <223> A_23_P163481
<400> 82
   cacagtgtct gttcttgggg agcttgcagc agaaatgaat ggggtttttg acactacatt 60
<210> 83
   <211> 60
   <212> DNA
   <213> Artificial
<220>
   <223> A_23_P35595
<400> 83
   cgccccagca gtgtcctcag aacactctac tcgcacctcc cggtgatcca tgaactctga 60
<210> 84
   <211> 60
   <212> DNA
   <213> Artificial
<220>
   <223> A_23_P35597
<400> 84
   aagaccaccg caggatgggc agcagagctc tgacctaaga agctggactt ttgggcagtc 60
<210> 85
   <211> 60
   <212> DNA
   <213> Artificial
<220>
   <223> A_24_P329795
<400> 85
   tgtgttttct ctggagatag aatgtaaacc atattaaaag gaaaaagttt cagacaagca 60
<210> 86
   <211> 60
   <212> DNA
   <213> Artificial
<220>
   <223> A_01_P003476
<400> 86
   gtaaaccaaa aacttttaaa tttcttcagg ttttctaaca tgcttaccac tgggctactg 60
<210> 87
   <211> 60
   <212> DNA
   <213> Artificial
<220>
   <223> A_01_P012231
<400> 87
   tcgctagcac aaaatattgt cgctaatagt catttctgtt ttcccattgt aaatgctgtt 60
<210> 88
   <211> 60
   <212> DNA
   <213> Artificial
<220>
   <223> A_23_P77041
<400> 88
   acacacgctt attgccgtgg caacagcggt agtgctaggg ggattaattt ttatagcatt 60
<210> 89
   <211> 60
   <212> DNA
   <213>. Artificial
<220>
   <223> A_23_P77043
<400> 89
   tcaacttaag cataaagggc tctgaacttt tccactttag agtgaccgtc atttcaggag 60
<210> 90
   <211> 60
   <212> DNA
   <213> Artificial
<220>
   <223> A_01_P000840
<400> 90
   atgccaggac ctcggcacct tcactgagaa catggttgtc tttgggtgcc ccaactgacc 60
<210> 91
   <211> 60
   <212> DNA
   <213> Artificial
<220>
   <223> A_23_P101407
<400> 91
   gtgccgctgt gctgaggaga attgcttcat acaaaagtcg gatgacaagg tcaccctgga 60
<210> 92
   <211> 60
   <212> DNA
   <213> Artificial
<220>
   <223> A_23_P101400
<400> 92
   atgccaagac gaagagaacc agaaacaatg ccaggacctc ggcgccttca ccgagagcat 60
<210> 93
   <211> 60
   <212> DNA
   <213> Artificial
<220>
   <223> A_01_P000020
<400> 93
   accgtgcctt atgcctgtgt gtgatcagtt tctggcacac agatgcctca ataaagattt 60
<210> 94
   <211> 60
   <212> DNA
   <213> Artificial
<220>
   <223> A_01_P005981
<400> 94
   cctcttgcaa atcaatacag atcagtttag caaatctact gtcaatttgg cagtgatatt 60
<210> 95
   <211> 60
   <212> DNA
   <213> Artificial
<220>
   <223> A_23_P97541
<400> 95
   ttctcaaaag aaggaggaaa aggtgtcttg ctggcttgcc tcttgcaatt caatacagat 60
<210> 96
   <211> 60
   <212> DNA
   <213> Artificial
<220>
   <223> A_23_P21092
<400> 96
   gctggatgcc cttttgaagg atctgtacga gaaaaacaaa aaggaaatga atattcaaca 60
<210> 97
   <211> 60
   <212> DNA
   <213> Artificial
<220>
   <223> A_23_P217570
<400> 97
   gtagtactta gcctacctag accagcaagc attcattttt agctcgctca ttttttacca 60
<210> 98
   <211> 60
   <212> DNA
   <213> Artificial
<220>
   <223> A_01_P018103
<400> 98
   cttctgcagt gccttcacta cactgcctta cataaaccaa atcacaataa agttcatatt 60
<210> 99
   <211> 60
   <212> DNA
   <213> Artificial
<220>
   <223> A_23_P40453
<400> 99
   gtggtgaata tcagtagttt gcagtgttta agggcttttg aaaactgcag tgaagatctg 60
<210> 100
   <211> 60
   <212> DNA
   <213> Artificial
<220>
   <223> A_24_P934477
<400> 100
   caggtcatga ggtttcatta tcggcagtaa ctcgttgcac atgtttctag tggcaaaaaa 60
<210> 101
   <211> 60
   <212> DNA
   <213> Artificial
<220>
   <223> A_23_P40445
<400> 101
   ttggtccatg acaaagttgt gcaaaactgg taaacgtctg cttcggagct tgctgcttaa 60
<210> 102
   <211> 60
   <212> DNA
   <213> Artificial
<220>
   <223> A_32_P72822
<400> 102
   tgtgtagtta agcaccaaac agcagagaga acttagacac taccacacca agccttgtga 60
<210> 103
   <211> 60
   <212> DNA
   <213> Artificial
<220>
   <223> A_23_P65757
<400> 103
   ttgattttgt acatagtcct ctggtctatc tcatgaaacc tcttctcaga ccagttttct 60
<210> 104
   <211> 60
   <212> DNA
   <213> Artificial
<220>
   <223> A_01_P000086
<400> 104
   agtcctctgg tctatctcat gaaacctctt ctcagaccaa ttttctaaat atatattgag 60
<210> 105
   <211> 60
   <212> DNA
   <213> Artificial
<220>
   <223> A_23_P118862
<400> 105
   ggcagaggac gggaggagac cagtccccca cccagccgta ccagaaataa aggcttctgt 60
<210> 106
   <211> 60
   <212> DNA
   <213> Artificial
<220>
   <223> A_01_P019708
<400> 106
   catgtcccgc agccgctgca acacgctgtc ctcccccaac cagtaccagt gacccagtgg 60
<210> 107
   <211> 60
   <212> DNA
   <213> Artificial
<220>
   <223> A_23_P431815
<400> 107
   gacaacctga ctatcaccat gcaccgcctg cagctgtcgg acactggcac ctacacctgc 60

<210> 108
   <211> 60
   <212> DNA
   <213> Artificial
<220>
   <223> A_01_P001621
<400> 108
   atcagcctcc ccaaggttct gtcctgttcc gagcaacttt tctaattata aacatcacag 60
<210> 109
   <211> 60
   <212> DNA
   <213> Artificial
<220>
   <223> A_23_P34597
<400> 109
   agaatgccca ctgcctgtaa cagccacctg gagaacttca taaagatgtc tcacagccct 60
<210> 110
   <211> 60
   <212> DNA
   <213> Artificial
<220>
   <223> A_23_P138507
<400> 110
   cccatgtcaa aaacttggat gaaaatggct tggatttgct ctcgaaaatg ttaatctatg 60
<210> 111
   <211> 60
   <212> DNA
   <213> Artificial
<220>
   <223> A_01_P011602
<400> 111
   tatcaaactt aagctgtact tcatcttcta atttcaaaag tataacttaa aaatgtaaat 60
<210> 112
   <211> 60
   <212> DNA
   <213> Artificial
<220>
   <223> A_23_P149195
<400> 112
   atccaccaag gcatccgctg aagaccaacc catcacctca gttgtttttt atttttctaa 60
<210> 113
   <211> 60
   <212> DNA
   <213> Artificial
<220>
   <223> A_01_P005979
<400> 113
   ccgctgaaga ctaacccatc acctcagttg ttttttattt ttctaataaa gtcatgtctc 60
<210> 114
   <211> 60
   <212> DNA
   <213> Artificial
<220>
   <223> A_23_P149200
<400> 114
   ggtaatgata acttggtcaa tgtgtggcct agtgctcctg gagagggtgg ctgggttcct 60
<210> 115
   <211> 60
   <212> DNA
   <213> Artificial
<220>
   <223> A_23_P253524
<400> 115
   ctgagtgcaa aactcagtag actcctcttt gtcacttctc tggagatcca gcattcctta 60
<210> 116
   <211> 60
   <212> DNA
   <213> Artificial
<220>
   <223> A_01_P006340
<400> 116
   ggctttactt tgccactaga gttgaaatat aagggaacag gaaatgaatg cattgtggta 60
<210> 117
   <211> 60
   <212> DNA
   <213> Artificial
<220>
   <223> A_23_P206733
<400> 117
   acctgaagag cttcaagctg aaaggaattt ccacactgtc ccctacatgg tcggaattaa 60
<210> 118
   <211> 60
   <212> DNA
   <213> Artificial
<220>
   <223> A_32_P540234
<400> 118
   gagcaatgaa gtcatccact cctgcatctg gttggtcttt attgagcacc tactatatgc 60
<210> 119
   <211> 60
   <212> DNA
   <213> Artificial
<220>
   <223> A_23_P25674
<400> 119
   aacctgggca agcatgagaa gttctcggag gtgcttaagc ggctgcgact tcagaagcga 60
<210> 120
   <211> 60
   <212> DNA
   <213> Artificial
<220>
   <223> A_24_P61537
<400> 120
   agaaatgaag cccggcccac acccgacacc agccctgctg cttcctaact tattgcctgg 60
<210> 121
   <211> 60
   <212> DNA
   <213> Artificial
<220>
   <223> A_01_P017811
<400> 121
   ctaacttatt gcctgggcag tgcccaccat gcacccctga tgttcgccgc ctggcgagcc 60
<210> 122
   <211> 60
   <212> DNA
   <213> Artificial
<220>
   <223> A_01_P007324
<400> 122
   gtaagaaact gtctgatatg aatcacaaca tggatgaatg tagtattttc ctgaagtgtg 60
<210> 123
   <211> 60
   <212> DNA
   <213> Artificial
<220>
   <223> A_23_P48350
<400> 123
   aaagtatgaa ttaggagccg ctctgtttat tggatgggca ggagcctcac tgtgcataat 60
<210> 124
   <211> 60
   <212> DNA
   <213> Artificial
<220>
   <223> A_23_P19944
<400> 124
   ggaagtcctg gggtttttcc tcttccttct ttgtggtttc tgttttgtaa tttaagaaga 60
<210> 125
   <211> 60
   <212> DNA
   <213> Artificial
<220>
   <223> A_24_P115183
<400> 125
   aacatcatcc aagacttcta caatccgctg gtggcctccg ggcagaagcg ggagatgggt 60
<210> 126
   <211> 60
   <212> DNA
   <213> Artificial
<220>
   <223> A_01_P000670
<400> 126
   gatggctcaa ccgtgacttt gggctctgct tgcatcggtg ttggccactg tccccattta 60
<210> 127
   <211> 60
   <212> DNA
   <213> Artificial
<220>
   <223> A_23_P215918
<400> 127
   tccaggaaga accctaaatt tatggagacc gtggcggaga aagcgctgca ggaataccgc 60
<210> 128
   <211> 60
   <212> DNA
   <213> Artificial
<220>
   <223> A_23_P215913
<400> 128
   cgctgagagg ttgaccagga aatacaacga gctgctaaag tcctaccagt ggaagatgct 60
<210> 129
   <211> 60
   <212> DNA
   <213> Artificial
<220>
   <223> A_23_P313623
<400> 129
   gtctacaggc caaaatgcgc acagttgatt ttcggtgtgt tcctgtataa cggcttgaaa 60
<210> 130
   <211> 60
   <212> DNA
   <213> Artificial
<220>
   <223> A_32_P86173
<400> 130
   gtatttggcc acccagcatt tttgggccga agaaaaaatt caaacctgtt gtccagagac 60
<210> 131
   <211> 60
   <212> DNA
   <213> Artificial
<220>
   <223> A_01_P003861
<400> 131
   aaaccatgaa ggggcctttt ggctgaaatc accacctgcc tttggatgaa ggactccgtt 60
<210> 132
   <211> 60
   <212> DNA
   <213> Artificial
<220>
   <223> A_23_P160318
<400> 132
   tggatgaaag actccgttgg gaataaatgg ccaaagctta taggactctg tgacaggttg 60
<210> 133
   <211> 60
   <212> DNA
   <213> Artificial
<220>
   <223> A_24_P264943
<400> 133
   ttaccgctgc aatgacacca tcccagagga ctatgagacc catcagctgc ggcaagccta 60
<210> 134
   <211> 60
   <212> DNA
   <213> Artificial
<220>
   <223> A_23_P90436
<400> 134
   gctgctgtgg aaggacccgc gaaacgtggg ttggaaggac aagaagtcct atcgttggtt 60
<210> 135
   <211> 60
   <212> DNA
   <213> Artificial
<220>
   <223> A_23_P106761
<400> 135
   tatctctcca tgttcagttc caaggagtcc cagcggggca tgggctacat gcccaaacgt 60
<210> 136
   <211> 60
   <212> DNA
   <213> Artificial
<220>
   <223> A_23_P83620
<400> 136
   tttctttgat cttctttttc ttttctcccc ctcttttttg ttctaaagaa aagtcatttt 60
<210> 137
   <211> 60
   <212> DNA
   <213> Artificial
<220>
   <223> A_24_P416131
<400> 137
   ggatcgtttt gttttgtttt taaagaaagg tgagattggc ttggttcttc atgagcacat 60
<210> 138
   <211> 60
   <212> DNA
   <213> Artificial
<220>
   <223> A_32_P158385
<400> 138
   agagctatat caaatgtgct catgaagaac caagccaatc tcacctttct ttaaaaacaa 60
<210> 139
   <211> 60
   <212> DNA
   <213> Artificial
<220>
   <223> A_23_P3866
<400> 139
   tcatgagcac atttgatata gctctttttc tgtttttcct tgctcatttc gttttgggga 60
<210> 140
   <211> 60
   <212>' DNA
   <213> Artificial
<220>
   <223> A_23_P83624
<400> 140
   agaccctggt gaaggaggtc gtacagaatt tcgctaagga gtttgtgatc agtgatcgga 60
<210> 141
   <211> 60
   <212> DNA
   <213> Artificial
<220>
   <223> A_01_P014841
<400> 141
   aacaatgtat gtgaaagtgt aaaatagaat gttactttag aatgactata aacattaaaa 60
<210> 142
   <211> 60
   <212> DNA
   <213> Artificial
<220>
   <223> A_23_P414793
<400> 142
   tcacggccat agcttccaat acaagcacag gggagtttat agttctgatg tctttgacat 60
<210> 143
   <211> 60
   <212> DNA
   <213> Artificial
<220>
   <223> A_23_P423074
<400> 143
   aagggcttca atcatattgg aggcttcaat catcctttga tttgtactga gtactggttg 60
<210> 144
   <211> 60
   <212> DNA
   <213> Artificial
<220>
   <223> A_32_P84242
<400> 144
   agaaaggctt accttctgtc atcaagtgat tgtatcatcc tggatcgtca tttccaagga 60
<210> 145
   <211> 60
   <212> DNA
   <213> Artificial
<220>
   <223> A_32_P84241
<400> 145
   tggatcgtca tttccaagga actagccttt cttttcctaa gcgtctgtat gtgttctaaa 60
<210> 146
   <211> 60
   <212> DNA
   <213> Artificial
<220>
   <223> A_23_P405064
<400> 146
   cccaacttct ctggcaactg gaaaatcatc cgatcggaaa acttcgagga attgctcaaa 60
<210> 147
   <211> 60
   <212> DNA
   <213> Artificial
<220>
   <223> A_01_P005286
<400> 147
   tgagggcctg agcaggaaag actggccctc tggcttctac actttgtccc tgtagcctat 60
<210> 148
   <211> 60
   <212> DNA
   <213> Artificial
<220>
   <223> A_23_P115064
<400> 148
   ccctacacca acaaagagga atggctgcaa gagcccagat cacccattcc gggttcactc 60
<210> 149
   <211> 60
   <212> DNA
   <213> Artificial
<220>
   <223> A_01_P003175
<400> 149
   cgttccagga gaaaaggctc tacttcccag cctttccttg cccctgacat ctggactctt 60
<210> 150
   <211> 60
   <212> DNA
   <213> Artificial
<220>
   <223> A_23_P150407
<400> 150
   ttcgcccctc ccttgtttta tattttatga agttagtgcg ggctttgctg ctccctggcc 60
<210> 151
   <211> 60
   <212> DNA
   <213> Artificial
<220>
   <223> A_23_P419760
<400> 151
   ataacaacaa ctttggattt ttatatataa actttgtgat ttaaatttac tgaatttaat 60
<210> 152
   <211> 60
   <212> DNA
   <213> Artificial
<220>.
   <223> A_01_P010473
<400> 152
   ctgtacttgg ataggctggc taacttgtag gaagagagca ctgtatcgta tccttttgct 60
<210> 153
   <211> 60
   <212> DNA
   <213> Artificial
<220>
   <223> A_23_P44724
<400> 153
   tgtacttgga taggctggct aactcgtagg aagagagcac tgtatggtat ccttttgctt 60
<210> 154
   <211> 60
   <212> DNA
   <213> Artificial
<220>
   <223> A_24_P33477
<400> 154
   cttatccagg cagctaaaaa cctgatgaat gctgttgtcc tcacggtgaa agcatcctat 60
<210> 155
   <211>' 60
   <212> DNA
   <213> Artificial
<220>
   <223> A_32_P14986
<400> 155
   ataggtgtag cttggagtgc tggtatctaa tataccattg tattcactaa ctaactcaaa 60
<210> 156
   <211> 60
   <212> DNA
   <213> Artificial
<220>
   <223> A_23_P84736
<400> 156
   gtgaaaaatc tggaagtgta atggtagaac ataaaacttg tattgcttct gtttcagtgc 60
<210> 157
   <211> 60
   <212> DNA
   <213> Artificial
<220>
   <223> A_24_P396167
<400> 157
   gacttcatca tgctgcagaa caacgagcac agaattgcgc agtacctggc cacttatggc 60
<210> 158
   <211> 60
   <212> DNA
   <213> Artificial
<220>
   <223> A_23_P13031
<400> 158
   tgaccatcaa catgaagccc cttcagctat accggaaagg tgtgatcaag gccacaccca 60
<210> 159
   <211> 60
   <212> DNA
   <213> Artificial
<220>
   <223> A_23_P13027
<400> 159
   tagagaccct gtggcgcatc agtttctggg attttgtgga cgtctccgtg catgaactgc 60
<210> 160
   <211> 60
   <212> DNA
   <213> Artificial
<220>
   <223> A_23_P121692
<400> 160
   ggcggggccg gggggactct ggtatctaat tctttaatga ttcctataaa tctaatgaca 60
<210> 161
   <211> 60
   <212> DNA
   <213> Artificial
<220>
   <223> A_01_P003023
<400> 161
   aaatttgctg tctaagatta atagcattca aagatcccca gacttcatag aatactcagg 60
<210> 162
   <211> 60
   <212> DNA
   <213> Artificial
<220>
   <223> A_23_P121695
<400> 162
   caagaggcaa aggaatccat gtagtagata tcctctgctt aaaaactcac tacggaggag 60
<210> 163
   <211> 60
   <212> DNA
   <213> Artificial
<220>
   <223> A_01_P019786
<400> 163
   cgtacatgtc agaaaccatt agcattgcat gcaggtttca tattctttct aagatggaaa 60
<210> 164
   <211> 60
   <212> DNA
   <213> Artificial
<220>
   <223> A_23_P213745
<400> 164
   gtggtacaac gcctggaacg agaagcgcag ggtctacgaa gaatagggtg aaaaacctca 60
<210> 165
   <211> 60
   <212> DNA
   <213> Artificial
<220>
   <223> A_24_P345451
<400> 165
   ccctcatttt ttggatagtc accagaccgc aatggaaacg tcctaaggag ccaaattcta 60
<210> 166
   <211> 60
   <212> DNA
   <213> Artificial
<220>
   <223> A_01_P005800
<400> 166
   ttagataact ccagcagaaa actgtaactg ctatgtcttc aggaaaatgt agaagaaaga 60
<210> 167
   <211> 60
   <212> DNA
   <213> Artificial
<220>
   <223> A_23_P209564
<400> 167
   cttcctgtgc tgcttgtgtc aaatggaacc tgccctctaa agcactttct ttcctttact 60
<210> 168
   <211> 60
   <212> DNA
   <213>' Artificial
<220>
   <223> A_23_P103486
<400> 168
   cacgtgttct gaaaccactg gtgtctgctc agatgtgttg ggacaaaatg aaagtgactt 60
<210> 169
   <211>. 60
   <212> DNA
   <213> Artificial
<220>
   <223> A_24_P916916
<400> 169
   atgtggctcc ctcctgctgt cctacagtca caacatggag tttgtctttt tctctgacag 60
<210> 170
   <211> 60
   <212> DNA
   <213> Artificial
<220>
   <223> A_23_P8807
<400> 170
   aggatttcta ctttggtctt caagaaagct gtgccccaga acaccagaga tttcaactta 60
<210> 171
   <211> 60
   <212> DNA
   <213> Artificial
<220>
   <223> A_23_P8801
<400> 171
   ttcaagagat ggaaccctaa gtggagaatg agttattcta aggatttcta ctttggtctt 60
<210> 172
   <211> 60
   <212> DNA
   <213> Artificial
<220>
   <223> A_32_P153423
<400> 172
   agtacacctt ggagtttttt cgaaatatgg gttgggtttt tgggctcttg gttgatttaa 60
<210> 173
   <211> 60
   <212> DNA
   <213> Artificial
<220>
   <223> A_23_P118660
<400> 173
   gtttatgcta aaaataacac caaaatgtgg tgaactctta aggacttttc ccttcaagtg 60
<210> 174
   <211> 60
   <212> DNA
   <213> Artificial
<220>
   <223> A_24_P1929
<400> 174
   ttcactgagg atgataagcc attattaaga agcgttgcta atgttataca gcaggctggg 60
<210> 175
   <211> 60
   <212> DNA
   <213> Artificial
<220>
   <223> A_01_P010027
<400> 175
   actttttata agcattcttt taataaagga aaattgtttt tgaagtatac ctcctttggg 60
<210> 176
   <211> 60
   <212> DNA
   <213> Artificial
<220>
   <223> A_23_P71480
<400> 176
   gcacctgtta cagagggaag gccaagtgct gcaagtgagc tgagagtgac cagaagaaat 60
<210> 177
   <211> 60
   <212> DNA
   <213> Artificial
<220>
   <223> A_23_P60166
<400> 177
   gggggtagtt agggagagac tacatgaaat ggtgtgcccc tattttcttt ctgatcctaa 60
<210> 178
   <211> 60
   <212> DNA
   <213> Artificial
<220>
   <223> A_01_P001685
<400> 178
   agcaataaag tcatccactc ctgcatctgg ttggtcttta ttgagcacct actatatgca 60
<210> 179
   <211> 60
   <212> DNA
   <213> Artificial
<220>
   <223> A_01_P000551
<400> 179
   ctctgcaccc ccagcctatc ccagaggcct tgcaggtgac cagcagtgtc attgtattta 60
<210> 180
   <211> 60
   <212> DNA
   <213> Artificial
<220>
   <223> A_24_P376129
<400> 180
   tgtgtagtgt atgttataat acaggctaag ctgccacaat aaaagagctt aaatactgtg 60
<210> 181
   <211> 60
   <212> DNA
   <213> Artificial
<220>
   <223> A_23_P83351
<400> 181
   agatggggcc aacaaaccgc ctggacttct ggagcccacg tccactctgg tccgtgtgaa 60
<210> 182
   <211> 60
   <212> DNA
   <213> Artificial
<220>
   <223> A_23_P33759
<400> 182
   aggcaggaaa acagccagaa gccaccttga cacttttgaa catttccagt tctgtagagt 60
<210> 183
   <211> 60
   <212> DNA
   <213> Artificial
<220>
   <223> A_23_P24129
<400> 183
   aataacacct tccaaaaacc tggagtgtaa gagctttgtt tctttatgga actcccctgt 60
<210> 184
   <211> 60
   <212> DNA
   <213> Artificial
<220>
   <223> A_23_P88331
<400> 184
   atccatttac tcagctggag aggagacatc aagaacatgc cagacacatt tcttttggtg 60
<210> 185
   <211> 60
   <212> DNA
   <213> Artificial
<220>
   <223> A_24_P97104
<400> 185
   agtcatgagc agagctgaaa attttaaaca agttgagtac ctccttattc atggaacagc 60
<210> 186
   <211> 60
   <212> DNA
   <213> Artificial
<220>
   <223> A_23_P39885
<400> 186
   gtgccagtta tggctatagg tgctacaaaa acacagcaag ggtgatggga aagcattgta 60
<210> 187
   <211> 60
   <212> DNA
   <213> Artificial
<220>
   <223> A_01_P008447
<400> 187
   aaatcatttc cgcatcagct gctgaaacaa caaataggaa ttgtttttat ggaagctttg 60
<210> 188
   <211> 60
   <212> DNA
   <213> Artificial
<220>
   <223> A_23_P54291
<400> 188
   agtctaacac cacaactaat ttcacccaag gttttaagca cgttctttca tcagaccctg 60
<210> 189
   <211> 60
   <212> DNA
   <213> Artificial
<220>
   <223> A_24_P316586
<400> 189
   aaagcaaagg agtgaacttc taatgctgta atttcagact cacatggttg cgcacatgga 60
<210> 190
   <211> 60
   <212> DNA
   <213> Artificial
<220>
   <223> A_23_P160559
<400> 190
   gatgaacagg tcaactgctt caacatcaat tatctgagga acgtggctct agtgtctgga 60
<210> 191
   <211> 60
   <212> DNA
   <213> Artificial
<220>
   <223> A_01_P019449
<400> 191
   tggagggtca aatgggggga accccaccct accccacccc tttgaacact cattacagta 60
<210> 192
   <211> 60
   <212> DNA
   <213> Artificial
<220>
   <223> A_23_P149180
<400> 192
   gaacagaagg aaggaatgcc agctccattt ggggaccaga gccatccaga acctgagtcc 60
<210> 193
   <211> 60
   <212> DNA
   <213> Artificial
<220>
   <223> A_01_P007491
<400> 193
   cattgcgcaa acactcagaa agtactgcca aaagcctaat aaaaaatcta aagtttgctc 60
<210> 194
   <211> 60
   <212> DNA
   <213> Artificial
<220>
   <223> A_01_P017090
<400> 194
   cattgcgcaa acactcagaa agtactgcca aaagcctaat aaaaaatcta aagtttgctc 60
<210> 195
   <211> 60
   <212> DNA
   <213> Artificial
<220>
   <223> A_01_P013980
<400> 195
   cattgcgcaa acactcagaa agtactgcca aaagcctaat aaaaaatcta aagtttgctc 60
<210> 196
   <211> 60
   <212> DNA
   <213> Artificial
<220>
   <223> A_23_P17438
<400> 196
   aagttattac aggtataaaa gtgatgacct atcatgagga aatgaaagtg gctgatttgc 60
<210> 197
   <211> 60
   <212> DNA
   <213> Artificial
<220>
   <223> A_01_P010992
<400> 197
   cagaaagaaa gagcaataat aattaattca catgccatgt ggattctatt tataaatcac 60
<210> 198
   <211> 60
   <212> DNA
   <213> Artificial
<220>
   <223> A_23_P2831
<400> 198
   gcttcactga attcctgcat taacccaatt gctctgtatt tggtgagcaa aagattcaaa 60
<210> 199
   <211> 60
   <212> DNA
   <213> Artificial
<220>
   <223> A_24_P330263
<400> 199
   gtaaaccaaa acccaacaat gtggccagaa agaaagagca ataataatta attcacacac 60
<210> 200
   <211> 60
   <212> DNA
   <213> Artificial
<220>
   <223> A_01_P015376
<400> 200
   tgtgacaagt ggacactatt tatgttaaat atacaatcat caaggaagta tgaagttatt 60
<210> 201
   <211> 60
   <212> DNA
   <213> Artificial
<220>
   <223> A_23_P501007
<400> 201
   aggcagccat cataaccatt gaatagcatg caagggtaag aatgagtttt taactgcttt 60
<210> 202
   <211> 60
   <212> DNA
   <213> Artificial
<220>
   <223> A_01_P017336
<400> 202
   ggaggcagcc atcataacca ttgaatagca tgcaagggta agaatgagtt tttaactgct 60
<210> 203
   <211> 60
   <212> DNA
   <213> Artificial
<220>
   <223> A_01_P016340
<400> 203
   ggaaccactc ccaccacagg cacaagctgt cacctagcag cctcaaaacg ggtcagtatt 60
<210> 204
   <211> 60
   <212> DNA
   <213> Artificial
<220>
   <223> A_23_P254512
<400> 204
   taaagggcag ggcccacgtg tatagtatct gtatataagt tgctgtgtgt ctgtcctgat 60
<210> 205
   <211> 60
   <212> DNA
   <213> Artificial
<220>
   <223> A_23_P113005
<400> 205
   atggagaaag aagtggagac agtcctttcc caccattcct gcctttaagc caaagaaaca 60
<210> 206
   <211> 60
   <212> DNA
   <213> Artificial
<220>
   <223> A_23_P382065
<400> 206
   agttttccgg gccaagaatt tttatccatg aagactttcc tacttttctc ggtgttctta 60
<210> 207
   <211> 60
   <212> DNA
   <213> Artificial
<220>
   <223> A_23_P333605
<400> 207
   tgtatggatt agcatcagtg aagaacgtta ctcttttgtc aaggtatttg gatttgctca 60
<210> 208
   <211> 60
   <212> DNA
   <213> Artificial
<220>
   <223> A_23_P144596
<400> 208
   ttgtgaatct attgtttctc ctctgaagca tttggtggcc taatttacaa gcacgatgga 60
<210> 209
   <211> 60
   <212> DNA
   <213> Artificial
<220>
   <223> A_23_P259220
<400> 209
   agattacaca accttcacga cagttggtga ttggctggat gccatcaaga tggggcggta 60
<210> 210
   <211> 60
   <212> DNA
   <213> Artificial
<220>
   <223> A_23_P95060
<400> 210
   aactggagga ggggactcca ggaatgggga aatgtgacac caccatcctg aagccagctt 60
<210> 211
   <211> 60
   <212> DNA
   <213> Artificial
<220>
   <223> A_23_P167051
<400> 211
   ccaaacgagc tgttcataac agcgattttg cctaacatta tatcataagc gtgttccaat 60
<210> 212
   <211> 60
   <212> DNA
   <213> Artificial
<220>
   <223> A_24_P60441
<400> 212
   ttgctccctg attaaaatga gatatggcta tttggaagac actgcatttt agccagtgta 60
<210> 213
   <211> 60
   <212> DNA
   <213> Artificial
<220>
   <223> A_24_P370042
<400> 213
   taaataggga taagagaaac tcttactatg cagattacgt ttttgaatgg tgaacaggct 60
<210> 214
   <211> 60
   <212> DNA
   <213> Artificial
<220>
   <223> A_23_P66948
<400> 214
   aacacttgga ggtgtgcctt gtacgtcact caacaaacac tcagcagctg ctaaaagaaa 60
<210> 215
   <211> 60
   <212> DNA
   <213> Artificial
<220>
   <223> A_23_P136125
<400> 215
   gagttggtgt tcataatttc agttctagtt gattgcgaga attttcaaat aaggaagagg 60
<210> 216
   <211> 60
   <212> DNA
   <213> Artificial

<220>
   <223> A_23_P114011
<400> 216
   gtgaatagca atatcccaac taaccttcgt gtgcttcgtt caatcctgga aaacctgaga 60
<210> 217
   <211> 60
   <212> DNA
   <213> Artificial
<220>
   <223> A_01_P018037
<400> 217
   tgagcaaagg agcttaatgc taaggtcaaa aaggagagtg aaaggttgag aacaattgtc 60
<210> 218
   <211> 60
   <212> DNA
   <213> Artificial
<220>
   <223> A_24_P266048
<400> 218
   tgtgatcatc agcaataaag atataataac tctgttttct tagcctgtat agaggagagg 60
<210> 219
   <211> 60
   <212> DNA
   <213> Artificial
<220>
   <223> A_23_P167599
<400> 219
   actgaagact ttgaacactt gctttttgtg attgcttatg tcattagtgc ctcatgactg 60
<210> 220
   <211> 60
   <212> DNA
   <213> Artificial
<220>
   <223> A_01_P009941
<400> 220
   actcagctct cacaggggta atcatctcaa gtggtatttg tagccaagtg ggagctattt 60
<210> 221
   <211> 60
   <212> DNA
   <213> Artificial
<220>
   <223> A_23_P218555
<400> 221
   tccctcattc atcttgcaag caaatcccat ttcttgaaaa gccttggaga actcggtttg 60
<210> 222
   <211> 60
   <212> DNA
   <213> Artificial
<220>
   <223> A_23_P348121
<400> 222
   tgggactgga agtgacctgt acaagtgatg cagaaaggag ggtttcaaag aaaaaggatt 60
<210> 223
   <211> 60
   <212> DNA
   <213> Artificial
<220>
   <223> A_23_P218553
<400> 223
   tattggaaga cttgggttga tctcttagaa gccatgggac ctcctccctc attcatcttg 60
<210> 224
   <211> 60
   <212> DNA
   <213> Artificial
<220>
   <223> A_23_P345581
<400> 224
   tgctgtcact ccgggcacct cgaacctcgt cttcacctat cctagcgtcc tggagcagga 60
<210> 225
   <211> 60
   <212> DNA
   <213> Artificial
<220>
   <223> A_24_P196562
<400> 225
   tgactatgag accgttcgca atgggggcct gatcttcgct ggactggcct tcatcgtggg 60
<210> 226
   <211> 60
   <212> DNA
   <213> Artificial
<220>
   <223> A_23_P161769
<400> 226
   ctttagacct ttgtccccgt cactgccagc gcttgggctg aaggaagctc cagactcaat 60
<210> 227
   <211> 60
   <212> DNA
   <213> Artificial
<220>
   <223> A_23_P74609
<400> 227
   ccaacactgt gtgaattatc taaatgcgtc taccattttg cactagggag gaaggataaa 60
<210> 228
   <211> 60
   <212> DNA
   <213> Artificial
<220>
   <223> A_01_P004760
<400> 228
   gacgcgtcca ctactgtgtc aattatctaa atacgtctac cattttgcgc tagggaggaa 60
<210> 229
   <211> 60
   <212> DNA
   <213> Artificial
<220>
   <223> A_23_P162640
<400> 229
   gatctcttac ctttggaaaa taggggttag gcatgaaggt ggttgtgatt aagaagatgg 60
<210> 230
   <211> 60
   <212> DNA
   <213> Artificial
<220>
   <223> A_24_P4816
<400> 230
   ggattggctt tgatagagga atggggatga tgtaagttta cagtattcct ggggtttaat 60
<210> 231
   <211> 60
   <212> DNA
   <213> Artificial
<220>
   <223> A_23_P65817
<400> 231
   tgtgtgtcag ttctgtcagc tgcaagttct tgtgtaatga agtcaatgct gtcaggccaa 60
<210> 232
   <211> 60
   <212> DNA
   <213> Artificial
<220>
   <223> A_23_P23221
<400> 232
   cctgccttaa gtcaacttat ttgtttttgc cgggaaagtc gctacatgga tcaatgggtt 60
<210> 233
   <211> 60
   <212> DNA
   <213> Artificial
<220>
   <223> A_01_P005123
<400> 233
   ttgttccact cttacgagac tgcaccaact cggatcatca gaaatggttc ttcaaggagc 60
<210> 234
   <211> 60
   <212> DNA
   <213> Artificial
<220>
   <223> A_23_P257731
<400> 234
   gatttgtgaa tgatcccaga ccaaccctga gattttgtca acctgattaa gtcaatatga 60
<210> 235
   <211> 60
   <212> DNA
   <213> Artificial
<220>
   <223> A_23_P415652
<400> 235
   gagaatcaga agttcatctt gcaggaggat ggatctttat ttcacgaaca gtccaagaaa 60
<210> 236
   <211> 60
   <212> DNA
   <213> Artificial
<220>
   <223> A_24_P942370
<400> 236
   tccctataac tcaaaataac ttgtttgtaa aagaaaattt gtttacttac ccattagtaa 60
<210> 237
   <211> 60
   <212> DNA
   <213> Artificial
<220>
   <223> A_01_P010093
<400> 237
   acttttctag gatgaagaca gcttattttt aagttgtatg gtcttagttg gtttagggtc 60
<210> 238
   <211> 60
   <212> DNA
   <213> Artificial
<220>
   <223> A_23_P413576
<400> 238
   ggtatttgaa aaacgttttt cctaatttac atgttccaga ggatagacca ggctggcatg 60
<210> 239
   <211> 60
   <212> DNA
   <213> Artificial
<220>
   <223> A_23_P116922
<400> 239
   aaagctgggg ttcattttgg tatatcacac tgaaactggg tacccagagt gctgctgttt 60
<210> 240
   <211> 60
   <212> DNA
   <213> Artificial
<220>
   <223> A_01_P005076
<400> 240
   cttggggaca gatagagggg atggttgggg atacttccca aaactttttc aagtcaactt 60
<210> 241
   <211> 60
   <212> DNA
   <213> Artificial
<220>
   <223> A_23_P83134
<400> 241
   ggattaaggg tccaaaaatg ctgatctaag gggttgccat ggtgttgaac aatgcaactt 60
<210> 242
   <211> 60
   <212> DNA
   <213> Artificial
<220>
   <223> A_23_P159190
<400> 242
   atggatggat ggacttcggc cgccgcagtg ctgaggatga gaactaacaa tcctagaacc 60
<210> 243
   <211> 60
   <212> DNA
   <213> Artificial
<220>
   <223> A_23_P159191
<400> 243
   agcctatgga tggatggact tcggccgccg cagtgctgag gatgagaact aacaatccta 60
<210> 244
   <211> 60
   <212> DNA
   <213> Artificial
<220>
   <223> A_01_P012911
<400> 244
   gctgaagtgt ggtgaatatg atgaaaatct gatgagacca aacaagccat ggggcacagt 60
<210> 245
   <211> 60
   <212> DNA
   <213> Artificial
<220>
   <223> A_23_P85693
<400> 245
   atacaggcca aagaggtgct gaaaaaatat ttggagtcca aggaggatgt ggctgatgca 60
<210> 246
   <211> 60
   <212> DNA
   <213> Artificial
<220>
   <223> A_23_P16523
<400> 246
   tattggggtg accttcttgg ggactcgggg gctggtctga tggaactgtg tatttattta 60
<210> 247
   <211> 60
   <212> DNA
   <213> Artificial
<220>
   <223> A_01_P016695
<400> 247
   ttggggtgac cttcctggga actggggggc tggtctgatg gaactgtgta tttatttaaa 60
<210> 248
   <211> 60
   <212> DNA
   <213> Artificial
<220>
   <223> A_24_P390096
<400> 248
   aagggataaa cctaaatatt tacttgttat cattagagag ggaacatcaa atgctgggac 60
<210> 249
   <211> 60
   <212> DNA
   <213> Artificial
<220>
   <223> A_23_P364024
<400> 249
   cagctcaagt accctaattt agttcttttg gactaataca attcaggaaa gaaaaaaccc 60
<210> 250
   <211> 60
   <212> DNA
   <213> Artificial
<220>
   <223> A_23_P209954
<400> 250
   gtgtgtagga cggggaggtc acgatggcgc gacgtctgca gaaatttcat gaggaggtat 60
<210> 251
   <211> 60
   <212> DNA
   <213> Artificial
<220>
   <223> A_24_P932547
<400> 251
   tgtggaattc atccatccaa ttaattccag cttataatat ttctggtcca catttgatgc 60
<210> 252
   <211> 60
   <212> DNA
   <213> Artificial
<220>
   <223> A_01_P016209
<400> 252
   ccatagtaat aatacacatt tctgtgagtg ctgacttgtc tttgcaatat ttcttctctg 60
<210> 253
   <211> 60
   <212> DNA
   <213> Artificial
<220>
   <223> A_23_P253692
<400> 253
   acccctttta gttaagtctt tcactaaggt tctcttgcat atatttcaag tgaatgttgg 60
<210> 254
   <211> 60
   <212> DNA
   <213> Artificial
<220>
   <223> A_23_P253695
<400> 254
   agggaactgt cctacactgc tattgttgct acatgtatcg agccttgatt gctcctagtt 60
<210> 255
   <211> 60
   <212> DNA
   <213> Artificial
<220>
   <223> A_32_P109029
<400> 255
   atgagaccat cctgaaagag cagaagggtc agagcatgtt cgtggagaac aaggcctttt 60
<210> 256
   <211> 60
   <212> DNA
   <213> Artificial
<220>
   <223> A_32_P544510
<400> 256
   cctgaaagag cagaagggtc agagcatgtt cgtggagaac aaggcctttt ccatggatga 60
<210> 257
   <211> 60
   <212> DNA
   <213> Artificial
<220>
   <223> A_23_P38167
<400> 257
   tccagccaaa tagtgttctc ggggtggtgg ctgggcagcg cctatgtttc tctggagatt 60
<210> 258
   <211> 60
   <212> DNA
   <213> Artificial
<220>
   <223> A_23_P346673
<400> 258
   gcaggaacag gtgtccatga ccctgattac ctggatctca caggttctgg gaatgcaata 60
<210> 259
   <211> 60
   <212> DNA
   <213> Artificial
<220>
   <223> A_23_P346670
<400> 259
   gggcctgctg tggctattcc aacatctctg gtcagggcct ggaagtacct taggacacat 60
<210> 260
   <211> 60
   <212> DNA
   <213> Artificial
<220>
   <223> A_01_P006986
<400> 260
   aggcctggat cttgctcctc tgtgaggaac aagggtgcct aataaaaaca tttctgcttt 60
<210> 261
   <211> 60
   <212> DNA
   <213> Artificial
<220>
   <223> A_23_P133474
<400> 261
   tgggtgtggg tgcatgtggg tgtttacaca catgcctaca ggtatgcgtg attgtgtgtg 60
<210> 262
   <211> 60
   <212> DNA
   <213> Artificial
<220>
   <223> A_23_P133475
<400> 262
   cactcaaggc cccagcctgg cacaaatgga tgcatacagt tctgtgtact gccaggcatg 60
<210> 263
   <211> 60
   <212> DNA
   <213> Artificial
<220>
   <223> A_23_P329821
<400> 263
   tcgttgggaa attgatgacc ggaaactaga aagcccctat aatgtggaat atgcatatgt 60
<210> 264
   <211> 60
   <212> DNA
   <213> Artificial
<220>.
   <223> A_23_P329822
<400> 264
   ctgacgttac taagcattgc agcacaatgt agaaattggc ttgggatgga taggtatagg 60
<210> 265
   <211> 60
   <212> DNA
   <213> Artificial
<220>
   <223> A_24_P40626
<400> 265
   gcaaacacga gcacactctc ttcgaaccca attgtgggtg tagcaatgaa agcaatatga 60
<210> 266
   <211> 60
   <212> DNA
   <213> Artificial
<220>
   <223> A_23_P97181
<400> 266
   aagtccactg ttagtctctc cgttagcacc agggacacac ttgttctgag ttttgttcat 60
<210> 267
   <211> 60
   <212> DNA
   <213> Artificial
<220>
   <223> A_01_P017852
<400> 267
   ctccttctgg gggttatcgt atgtacaaag tttaccttat aatggctcaa attgtattta 60
<210> 268
   <211> 60
   <212> DNA
   <213> Artificial
<220>
   <223> A_23_P133445
<400> 268
   gaatgaatat ggtttgtgct ggaagcctcc gaggtggaaa aaactcgtgc aatggagatt 60
<210> 269
   <211> 60
   <212> DNA
   <213> Artificial
<220>
   <223> A_01_P014820
<400> 269
   aaattaggcg ccttgtttga gctgcatttc acacttcttt agagctagct gacctttggc 60
<210> 270
   <211> 60
   <212> DNA
   <213> Artificial
<220>
   <223> A_23_P1173
<400> 270
   aacccaagga atgatggaat caacacaaca tagtatgttt gctttcctta cccaattgta 60
<210> 271
   <211> 60
   <212> DNA
   <213> Artificial
<220>
   <223> A_23_P61643
<400> 271
   ttctgcacaa gaaatccacc aaaatcccgg agtctgagga cctttaatgg gctttgtcat 60
<210> 272
   <211> 60
   <212> DNA
   <213> Artificial
<220>
   <223> A_23_P61637
<400> 272
   agatcaatcc attgtatcat tcagttcttc taaagcctac gttggttagg ctgatggcag 60
<210> 273
   <211> 60
   <212> DNA
   <213> Artificial
<220>
   <223> A_01_P003831
<400> 273
   ccaggtattt tcagattgta ggagttttct ttcttaacaa tttcaacagg ccactcactc 60
<210> 274
   <211> 60
   <212> DNA
   <213> Artificial
<220>
   <223> A_32_P502420
<400> 274
   gcaacagata atcctatggt ctttgccaat aggggagaga caatttctgg aggaaacttc 60
<210> 275
   <211> 60
   <212> DNA
   <213> Artificial
<220>
   <223> A_23_P7154
<400> 275
   ttggggtcgg tatctagtgc tatccattca tctgtggtcg ttccctcttt gaagatgttt 60
<210> 276
   <211> 60
   <212> DNA
   <213> Artificial
<220>
   <223> A_23_P373521
<400> 276
   tctgaggact ccttgcattt ggaatcatcc ggtttattta tgtgcaattt ccttcccctc 60
<210> 277
   <211> 60
   <212> DNA
   <213> Artificial
<220>
   <223> A_23_P157659
<400> 277
   ataggtttgt actgtgccta atttactttg taaaccagaa tgattccgtt tttgcctcac 60
<210> 278
   <211> 60
   <212> DNA
   <213> Artificial
<220>
   <223> A_01_P001077
<400> 278
   aaacgacctc cactgaactg ggtttgacct ctgttgtact gatgtgtcgt gactaaataa 60
<210> 279
   <211> 60
   <212> DNA
   <213> Artificial
<220>
   <223> A_32_P83845
<400> 279
   gggctataaa atatcatttt tcaggtttat tcttttagca ggtgtagtta aacgacctcc 60
<210> 280
   <211> 60
   <212> DNA
   <213> Artificial
<220>
   <223> A-01-P012352
<400> 280
   taaaagataa gagacacaac atgtattatg cacttcattt ctctactgtg tggagaaagc 60
<210> 281
   <211> 60
   <212> DNA
   <213> Artificial
<220>
   <223> A_23_P168351
<400> 281
   aaaggagagg gattatagag aaaaggcgtc gggatcggat aaataacagt ttatctgagt 60
<210> 282
   <211> 60
   <212> DNA
   <213> Artificial
<220>
   <223> A_24_P363408
<400> 282
   aagctctttg tcaagttagt gattgcattt gatcccaaaa caagatgaat gtatgcaatg 60
<210> 283
   <211> 60
   <212> DNA
   <213> Artificial
<220>
   <223> A_23_P168354
<400> 283
   tactcggggc aaagtactag ctctgtgatt agattgaatt ctccaacaac aacatctcag 60
<210> 284
   <211> 60
   <212> DNA
   <213> Artificial
<220>
   <223> A_32_P477383
<400> 284
   ttccagtccc aaatcattta cttttctgtg gtccagccct actcctataa gtcatgatct 60
<210> 285
   <211> 60
   <212> DNA
   <213> Artificial
<220>
   <223> A_24_P911788
<400> 285
   ggtagggagg caggaagagg gaaacattgt gtcttgttta ggatccttat tgtgtgtatc 60
<210> 286
   <211> 60
   <212> DNA
   <213> Artificial
<220>
   <223> A_23_P30736
<400> 286
   ccaaatcatt tacttttctg tggtccagcc ctactcctat aagtcatgat ctccaaagct 60
<210> 287
   <211> 60
   <212> DNA
   <213> Artificial
<220>
   <223> A_32_P343332
<400> 287
   ccaaatcatt tacttttctg tggtccagcc ctactcctat aagtcatgat ctccaaagct 60
<210> 288
   <211> 60
   <212> DNA
   <213> Artificial
<220>
   <223> A_01_P015398
<400> 288
   tgtcttccaa ctgcagtctc cacagtcttc agaagacaaa tgctcaggta gtcactgttt 60
<210> 289
   <211> 60
   <212> DNA
   <213> Artificial
<220>
   <223> A_23_P374053
<400> 289
   ggtctctcgg aagccaccgt gtggttcttt cacaggcacg tttattttgc tgaaataaaa 60
<210> 290
   <211> 60
   <212> DNA
   <213> Artificial
<220>
   <223> A_24_P71904
<400> 290
   agacaaagta gtgaacatca atgaacatct gatagagata aactgtaatc aggcataagc 60
<210> 291
   <211> 60
   <212> DNA
   <213> Artificial
<220>
   <223> A_23_P213050
<400> 291
   catcattatc aatatgtcat ctttagcagg actcatgccc gttgcacagc agccggttta 60
<210> 292
   <211> 60
   <212> DNA
   <213> Artificial
<220>
   <223> A_01_P012816
<400> 292
   cctctgctca ctttaagaac tttaactgac tccaaaaatc tcaggaatta aactgttaac 60
<210> 293
   <211> 60
   <212> DNA
   <213> Artificial
<220>
   <223> A_23_P256107
<400> 293
   agtggatgaa aacttcgatc ctttacctga ttattggcta tctcttctgt tcaagaaatt 60
<210> 294
   <211> 60
   <212> DNA
   <213> Artificial
<220>
   <223> A_23_P116414
<400> 294
   catcaagggg gtcttgtttt gctagagagt ttggggtttg gtttgtggat ttcattgtga 60
<210> 295
   <211> 60
   <212> DNA
   <213> Artificial
<220>
   <223> A_23_P14986
<400> 295
   tgcagatagt gcctctgcaa actaaggagt gactaggtgg gttggggacc ccctcaggat 60
<210> 296
   <211> 60
   <212> DNA
   <213> Artificial
<220>
   <223> A_23_P59375
<400> 296
   aaagccaccg gaggaaagga aaaaacatcg gccaacctag aaacgttttc attcgtcatt 60
<210> 297
   <211> 60
   <212> DNA
   <213> Artificial
<220>
   <223> A_32_P45009
<400> 297
   gttctggtgt catagatgtc ccattttgtg aggtagagct gtgcattaaa cttgcacatg 60
<210> 298
   <211>' 60
   <212> DNA
   <213> Artificial
<220>
   <223> A_23_P5376
<400> 298
   gcacagggaa gttctggtgt catagatatc ccgttttgtg aggtagagct gtgcattaaa 60
<210> 299
   <211> 60
   <212> DNA
   <213> Artificial
<220>
   <223> A_01_P006229
<400> 299
   gcacagggaa gttctggtgt catagatatc ccgttttgtg aggtagagct gtgcattaaa 60
<210> 300
   <211> 60
   <212> DNA
   <213> Artificial
<220>
   <223> A_01_P015343
<400> 300
   cgactgtcga gattgcccag tatgttctgt gaacacaaat aaacttgatt tactgtctgc 60
<210> 301
   <211> 60
   <212> DNA
   <213> Artificial
<220>
   <223> A_23_P42257
<400> 301
   tgtgagatgt tccccctgct gtaaatgcag gtctcttggt atttattgag ctttgtggga 60
<210> 302
   <211> 60
   <212> DNA
   <213> Artificial
<220>
   <223> A_01_P000583
<400> 302
   cgactgtcga gattgcccag tatgttctgt gaacacaaat aaacttgatt tactgtctgc 60
<210> 303
   <211> 60
   <212> DNA
   <213> Artificial
<220>
   <223> A_32_P419552
<400> 303
   gtacgtaata tttattttaa cttatgcaag ggtgtgagat gttccccctg ctgtaaatgc 60
<210> 304
   <211> 60
   <212> DNA
   <213> Artificial
<220>
   <223> A_23_P203458
<400> 304
   gccacaacaa ccctcttaaa actaattggc tttttagaaa caccccacaa aagctcagaa 60
<210> 305
   <211> 60
   <212> DNA
   <213> Artificial
<220>
   <223> A_23_P421379
<400> 305
   tgcttccgga caacttcccc agataccccg tgggcaagtt cttccaatat gacacctgga 60
<210> 306
   <211> 60
   <212> DNA
   <213> Artificial
<220>
   <223> A_23_P150609
<400> 306
   ctcaactcag ctcctttaac gctaatattt ccggcaaaat cccatgcttg ggttttgtct 60
<210> 307
   <211> 60
   <212> DNA
   <213> Artificial
<220>
   <223> A_23_P42869
<400> 307
   catgaaacac ttctcatcat attgtatgta agtaattgca tttctgctct tccaaagctc 60
<210> 308
   <211> 60
   <212> DNA
   <213> Artificial
<220>
   <223> A_23_P42868
<400> 308
   tttctgctct tccaaagctc ctgcgtctgt ttttaaagag catggaaaaa tactgcctag 60
<210> 309
   <211> 60
   <212> DNA
   <213> Artificial
<220>
   <223> A_01_P002590
<400> 309
   aatcaaagct acctgtggtg atgttgccac cggttaaaat gtacactgga tatgttgtta 60
<210> 310
   <211> 60
   <212> DNA
   <213> Artificial
<220>
   <223> A_23_P29953
<400> 310
   ctgtgtggaa ccactgacta ctggctctca ttgacttcct tactaagcat agcaaacaga 60
<210> 311
   <211> 60
   <212> DNA
   <213> Artificial
<220>
   <223> A_24_P203000
<400> 311
   ttgaggttgt ctgagtcttg ggtctatgcc ttgaaaaaag ctgaattatt ggacagtctc 60
<210> 312
   <211> 60
   <212> DNA
   <213> Artificial
<220>
   <223> A_01_P020028
<400> 312
   ccacaaagtg gacgcctgct gtatcttccc aacagtggct tcacagaccc acaagagaag 60
<210> 313
   <211> 60
   <212> DNA
   <213> Artificial
<220>
   <223> A_23_P255430
<400> 313
   ctcccggatg ccttggagat agaggcctgc caggtgtact ttacttacga cccctactca 60
<210> 314
   <211> 60
   <212> DNA
   <213> Artificial
<220>
   <223> A_01_P011439
<400> 314
   tccccccaga atataaatct caggtaataa ggctttagaa ctgctgataa agcggatcgt 60
<210> 315
   <211> 60
   <212> DNA
   <213> Artificial
<220>
   <223> A_23_P50081
<400> 315
   tagctgtttc tctctttaat ctcacgtagc ctttttcagg ttagtacgtg ttcttctgtc 60
<210> 316
   <211> 60
   <212> DNA
   <213> Artificial
<220>
   <223> A_23_P112026
<400> 316
   atctgcaaat cgtgactaag tacatcctga ttcctgcaag ccagcagcca aaggagaata 60
<210> 317
   <211> 60
   <212> DNA
   <213> Artificial
<220>
   <223> A_01_P006886
<400> 317
   acccgcgagt tcgggctcct gctgctcttc ctctgcgtgg ccatcgccct cttcgcgccc 60
<210> 318
   <211> 60
   <212> DNA
   <213> Artificial
<220>
   <223> A_23_P210581
<400> 318
   ccctatgtgt gtttccctca ataaggagat gccttgttct tttcaccatg caaataacat 60
<210> 319
   <211> 60
   <212> DNA
   <213> Artificial
<220>
   <223> A_24_P237117
<400> 319
   cctgtcggtg ctcttcgtga ccgtcaccgc cgtcaacctc tccgtcagca ccttgcccag 60
<210> 320
   <211> 60
   <212> DNA
   <213> Artificial
<220>
   <223> A_23_P210580
<400> 320
   ccagccccta tgtgtgtttc cctcaataag gagatgcctt gttcttttca ccatgcaaat 60
<210> 321
   <211> 60
   <212> DNA
   <213> Artificial
<220>
   <223> A_01_P002764
<400> 321
   ccgccgatgc acccgcgagt tcgggctcct gctgctcttc ctctgcgtgg ccatcgccct 60
<210> 322
   <211> 60
   <212> DNA
   <213> Artificial
<220>
   <223> A_23_P329261
<400> 322
   aagttgaaca atcctagcca ttgacaatcg tgatagttat tattttccca tttgctgtct 60
<210> 323
   <211> 60
   <212> DNA
   <213> Artificial
<220>
   <223> A_23_P55219
<400> 323
   tcgttttgtg tttttcccaa aacttgaact tgcaggcaag ccttggttgg gtatttgatt 60
<210> 324
   <211> 60
   <212> DNA
   <213> Artificial
<220>
   <223> A_23_P86874

<400> 324
   cctgcgccat tgcctgctgc ctgtgctcag ccgcccacgt gcctgggtag cggtccactg 60
<210> 325
   <211> 60
   <212> DNA
   <213> Artificial
<220>
   <223> A_01_P003027
<400> 325
   gcttgctgaa gcgtcaggtg accgagttca gctcccataa ggtggcggca cctaaggagg 60
<210> 326
   <211> 60
   <212> DNA
   <213> Artificial
<220>
   <223> A_01_P016715
<400> 326
   cttttgtaac tcaagtcttg aaatgttctg tagtgttaag caaagtctcc tcttgcttga 60
<210> 327
   <211> 60
   <212> DNA
   <213> Artificial
<220>
   <223> A_23_P257335
<400> 327
   atgatgaaca gagttatgat tcctatgata acagctatag caccccagcc caaagtggtg 60
<210> 328
   <211> 60
   <212> DNA
   <213> Artificial
<220>
   <223> A_01_P004436
<400> 328
   aaacacacag ctgctgaatg ttcaacctgt gaaactgaga tgtttctaga atgaaacagt 60
<210> 329
   <211> 60
   <212> DNA
   <213> Artificial
<220>
   <223> A_32_P38763
<400> 329
   aggataccaa atggaaacac atgatgatgc ctctgggtct gtatgagacc gtgatgaagt 60
<210> 330
   <211> 60
   <212> DNA
   <213> Artificial
<220>
   <223>. A_23_P360605
<400> 330
   ctcaaagttt tcttaaggga aaacactaca aaaagtcaca aggataccaa atggaaacac 60
<210> 331
   <211> 60
   <212> DNA
   <213> Artificial
<220>
   <223> A_01_P007808
<400> 331
   cacatagtcc tttgtgaact tgtttgtgaa ggaagttcac tttttgtgta catacgtgta 60
<210> 332
   <211> 60
   <212> DNA
   <213> Artificial
<220>
   <223> A_01_P007810
<400> 332
   ttgcaaggca ttagaaaaaa tttcacaatt acaggggact gaaaatgtga tttcaaccag 60
<210> 333
   <211> 60
   <212> DNA
   <213> Artificial
<220>
   <223> A_24_P227091
<400> 333
   acactccaag acctgtgcct tttagagaag ctcacaatga tttaaggact gtttgaaact 60
<210> 334
   <211> 60
   <212> DNA
   <213> Artificial
<220>
   <223> A_23_P52278
<400> 334
   aacacactgg agaggtctaa agtggaagaa actacagagc acttggttac aaagagcaga 60
<210> 335
   <211> 60
   <212> DNA
   <213> Artificial
<220>
   <223> A_23_P256956
<400> 335
   tcaagccttg accacttgtg atgacatctt aatcaaacag gaccagactc tggctgaact 60
<210> 336
   <211> 60
   <212> DNA
   <213> Artificial
<220>
   <223> A_23_P148475
<400> 336
   gcttgggaga tgctttcagg ttgcagccag aaggggtttt ttaaatgact tctctggatt 60
<210> 337
   <211> 60
   <212> DNA
   <213> Artificial
<220>
   <223> A_23_P151046
<400> 337
   ggttgaaata ggagatgacc tctaactgat agaacgttac tttgtgtcgt gatgaaaact 60
<210> 338
   <211> 60
   <212> DNA
   <213> Artificial
<220>
   <223> A_23_P139654
<400> 338
   attgttggga tcctgggaat tatctgtctt atcttaatgg cctctgtggt aacgatagtt 60
<210> 339
   <211> 60
   <212> DNA
   <213> Artificial
<220>
   <223> A_24_P387926
<400> 339
   agtggaatat taagtaaaag ttgggcacta atctggatta acattcgagg aaatcagttg 60
<210> 340
   <211> 60
   <212> DNA
   <213> Artificial
<220>
   <223> A_23_P200838
<400> 340
   ataggacaga tcccatctcc tccacccaat acattattag actgaactgt gacctgaaat 60
<210> 341
   <211> 60
   <212> DNA
   <213> Artificial
<220>
   <223> A_24_P77082
<400> 341
   tcatgcgatt atgccatcga cctttatccc tctctataca atggtcactt tttccagaat 60
<210> 342
   <211> 60
   <212> DNA
   <213> Artificial
<220>
   <223> A_23_P50108
<400> 342
   aaagtgggaa ataacttgca acgtctgtta gagatggttg ctacacatgt tgggtctgta 60
<210> 343
   <211> 60
   <212> DNA
   <213> Artificial
<220>
   <223> A_01_P010921
<400> 343
   agaatgaaga taaaatgttg atcatgtata tatatccata gtgaataaaa ttgtctcagt 60
<210> 344
   <211> 60
   <212> DNA
   <213> Artificial
<220>
   <223> A_24_P14156
<400> 344
   agtgtattcg acaactctgt gagtttctta cagaaaatgg ttatgcacat aatgtgtcca 60
<210> 345
   <211> 60
   <212> DNA
   <213> Artificial
<220>
   <223> A_23_P313591
<400> 345
   gaagatgaaa ttaaccgccg cacagctgct gagaatgagt ttgtggtgct gaagaaggat 60
<210> 346
   <211> 60
   <212> DNA
   <213> Artificial
<220>
   <223> A_23_P381945
<400> 346
   gaagatgaaa ttaaccaccg cacagctgct gagaatgagt ttgtggtgct gaagaaggat 60
<210> 347
   <211> 60
   <212> DNA
   <213> Artificial
<220>
   <223> A_23_P86012
<400> 347
   gcctttagtt ctccactggg gaggaatcct•ggaccaagca caaaaactta acaaaagtga 60
<210> 348
   <211> 60
   <212> DNA
   <213> Artificial
<220>
   <223> A_01_P018078
<400> 348
   atggccaggt tgcctacgag tggggtccac tgatgaaaag aggttttttg tacttacata 60
<210> 349
   <211> 60
   <212> DNA
   <213> Artificial
<220>
   <223> A_23_P69179
<400> 349
   ccaggttgcc taggagtggg gtccactgat gaaaagaggt gttttgtact tacataagaa 60
<210> 350
   <211> 60
   <212> DNA
   <213> Artificial
<220>
   <223> A_24_P122137
<400> 350
   catttccctg cagatggtac agatgttcct gccttagagt catctctagt tccccacctc 60
<210> 351
   <211> 60
   <212> DNA
   <213> Artificial
<220>
   <223> A_24_P233488
<400> 351
   gtcttccaga agaagaagct gggctgtcaa ctcctgggga agtataagca gatcatcgcc 60
<210> 352
   <211> 60
   <212> DNA
   <213> Artificial
<220>
   <223> A_23_P155017
<400> 352
   tctggcaccc ggggaggtag catttccctg cagatggtac agatgttcct gccttagagt 60
<210> 353
   <211> 60
   <212> DNA
   <213> Artificial
<220>
   <223> A_01_P019499
<400> 353
   aggagcaggc tgggatccca actatcgctt gttgcctctt ttcaagtgga atttgaattt 60
<210> 354
   <211> 60
   <212> DNA
   <213> Artificial
<220>
   <223> A_23_P6771
<400> 354
   aggagcaggc tgggatccca actatcgctt gttgcctctt tttcaagtgg aatttgaatt 60
<210> 355
   <211> 60
   <212> DNA
   <213> Artificial
<220>
   <223> A_01_P000575
<400> 355
   acacactggc tcctagacct aaagggtatg agctggagct aaggccagct agagcttcca 60
<210> 356
   <211> 60
   <212> DNA
   <213> Artificial
<220>
   <223> A_23_P201940
<400> 356
   acttgaaggt ttgatgccaa agcagacatt ttcctcacac ccacctgctg ctgtatgaat 60
<210> 357
   <211> 60
   <212> DNA
   <213> Artificial
<220>
   <223> A_23_P63980
<400> 357
   ggtgtgatgg acgggcagct tcctgtgtgc tccaagggat gagcctcgtg gggcagaggg 60
<210> 358
   <211> 60
   <212> DNA
   <213> Artificial
<220>
   <223> A_01_P007807
<400> 358
   ctttcctcgg ttctggcctc cagaccagag taaggggcag gtccctccaa caggtgctca 60
<210> 359
   <211> 60
   <212> DNA
   <213> Artificial
<220>
   <223> A_24_P403561
<400> 359
   agcactgatt tactctgtaa aaagcaaaat ctctctgtcc taaactaatg gaagcgattc 60
<210> 360
   <211> 60
   <212> DNA
   <213> Artificial
<220>
   <223> A_23_P159349
<400> 360
   tctggctccc tggatgacac agagacggag cagctgttac aggaagagca gtctgagtgt 60
<210> 361
   <211> 60
   <212> DNA
   <213> Artificial
<220>
   <223> A_23_P215024
<400> 361
   ttttttctga ataagtctct cataatgagt gcagtgtcag actgtgccta ctctgatggt 60
<210> 362
   <211> 60
   <212> DNA
   <213> Artificial
<220>
   <223> A_01_P020198
<400> 362
   ttaagggata ttgggaaaag ttttggtgtg tttctgttga cttctttttt gtatgctgtg 60
<210> 363
   <211> 60
   <212> DNA
   <213> Artificial
<220>
   <223> A_01_P016431
<400> 363
   aggaagctta tagtgggtca aacaaggagg tgtttagtgt gttgtttaaa aagaaggctc 60
<210> 364
   <211> 60
   <212> DNA
   <213> Artificial
<220>
   <223> A_23_P253958
<400> 364
   gcaccactac aatgtccatt ttaatggcct ggaatgcaaa acgcctgaag aatacaaagg 60
<210> 365
   <211> 60
   <212> DNA
   <213> Artificial
<220>
   <223> A_23_P83857
<400> 365
   ctgctgaaga tcattggatt ttccacatca gtaactgccc tggggtttgt gctgtacaaa 60
<210> 366
   <211> 60
   <212> DNA
   <213> Artificial
<220>
   <223> A_23_P96410
<400> 366
   ccccagtgct acacgttgga gtatacctat gtgtgtgctt tgccactgaa gtaagatttt 60
<210> 367
   <211> 60
   <212> DNA
   <213> Artificial
<220>
   <223> A_01_P016743
<400> 367
   ttttctttct tggcctcaag ttcaatatgg agagggattg cttccctgaa tcctctcttc 60
<210> 368
   <211> 60
   <212> DNA
   <213> Artificial
<220>
   <223> A_23_P207445
<400> 368
   acagcatcaa tagaaagtca tctttgagat aatttaaccc tgcctctcag agggttttct 60
<210> 369
   <211> 60
   <212> DNA
   <213> Artificial
<220>
   <223> A_23_P1021
<400> 369
   gtctgcttgg atttcctaca gcccccgtgg gcatggacca cctttatttt atacaaaatt 60
<210> 370
   <211> 60
   <212> DNA
   <213> Artificial
<220>
   <223> A_23_P1029
<400> 370
   ccgcctctac tccatacaca ggccttgcaa acagtgtctc aacgaggtct gcttctacag 60
<210> 371
   <211> 60
   <212> DNA
   <213> Artificial
<220>
   <223> A_23_P87700
<400> 371
   tgagaggaag gaggatctcc ttcttctcca accattgaca gctaaccctt agacagtatt 60
<210> 372
   <211> 60
   <212> DNA
   <213> Artificial
<220>
   <223> A_01_P003020
<400> 372
   ttgaccctcc tactccacat tgcaacattt gcatcagaca gcatttcaat tccagtatta 60
<210> 373
   <211> 60
   <212> DNA
   <213> Artificial
<220>
   <223> A_01_P013777
<400> 373
   agatgactgt ttctcatgcc tttatcttcc ttcatgtaag taaagtggac ctttgtgctc 60
<210> 374
   <211> 60
   <212> DNA
   <213> Artificial
<220>
   <223> A_23_P2052
<400> 374
   tattcaacct gtcctttcag ggagtttatt ggaggatcaa agaactgaaa gcactagagc 60
<210> 375
   <211> 60
   <212> DNA
   <213> Artificial
<220>
   <223> A_23_P135104
<400> 375
   ttctcaatct aaatgccttt caggtgggcc gcttccttgg ctacctggtt ccagggggct 60
<210> 376
   <211> 60
   <212> DNA
   <213> Artificial
<220>
   <223> A_23_P110430
<400> 376
   aagtggctgg aagagtccct tagtactctt ctagcattta gatctacact ctcgagttaa 60
<210> 377
   <211> 60
   <212> DNA
   <213> Artificial
<220>
   <223> A_01_P001372
<400> 377
   aacatttgct ctgggggggc agggaataca cagatgcgtt gcaaaggtag gttgaaggga 60
<210> 378
   <211> 60
   <212> DNA
   <213> Artificial
<220>
   <223> A_24_P345837
<400> 378
   accgccgcgc caaggcaaag agactacaag aggcagagct ggagaagctg aagatggccg 60
<210> 379
   <211> 60
   <212> DNA
   <213> Artificial
<220>
   <223> A_23_P60933
<400> 379
   agctgctgtg cctgatgtcg ggacagccct gctcccaagt acaaatagag tgacccgtaa 60
<210> 380
   <211> 60
   <212> DNA
   <213> Artificial
<220>
   <223> A_23_P206707
<400> 380
   ttgggaactc tagtctcgcc tcgggttgca atggacccca actgctcctg tgccgctggt 60
<210> 381
   <211> 60
   <212> DNA
   <213> Artificial
<220>
   <223> A_23_P206701
<400> 381
   gcatcggaga agtgcagctg ctgcgcctga tgtcgggaca gccctgctcc caagtacaaa 60
<210> 382
   <211> 60
   <212> DNA
   <213> Artificial
<220>
   <223> A_23_P414343
<400> 382
   tgtgccaagt gtgcccacgg ctgcatctgc aaagggacgt cggagaagtg cagctgctgt 60
<210> 383
   <211> 60
   <212> DNA
   <213> Artificial
<220>
   <223> A_23_P106844
<400> 383
   caaccctgac cgtgaccgtt tgctatattc ctttttctat gaaataatgt gaatgataat 60
<210> 384
   <211> 60
   <212> DNA
   <213> Artificial
<220>
   <223> A_24_P361896
<400> 384
   cgctcccaga tgtaaagaac gcgacttcca caaacctgga ttttttatgt acaaccctga 60
<210> 385
   <211> 60
   <212> DNA
   <213> Artificial
<220>
   <223> A_32_P306874
<400> 385
   cacaatgatt ccaacttcaa ctctttccct tggtttacta gagactacag gcttactggc 60
<210> 386
   <211> 60
   <212> DNA
   <213> Artificial
<220>
   <223> A_32_P889536
<400> 386
   gagcacacgc tagttcagaa agtccaagca ccatcaaact taccatggct tcagtagtaa 60
<210> 387
   <211> 60
   <212> DNA
   <213> Artificial
<220>
   <223> A_32_P307960
<400> 387
   tggtgtcttt atctgcagta acctcacctt cgccacttta ttccacacca tctgagagta 60
<210> 388
   <211> 60
   <212> DNA
   <213> Artificial
<220>
   <223> A_32_P393950
<400> 388
   ccaagacctc cttggttgaa acaactgatg gaacgctagt gaccaccata aagatgtcaa 60
<210> 389
   <211> 60
   <212> DNA
   <213> Artificial
<220>
   <223> A_32_P309031
<400> 389
   cagaatcagc agaaatgatg atcaagacac aaacagatcc tcctgggtct acaccagaga 60
<210> 390
   <211> 60
   <212> DNA
   <213> Artificial
<220>
   <223> A_32_P424560
<400> 390
   cagggatcca ctcagctatg actcatggat tttcacaatt ggatgtgacc actcttatga 60
<210> 391
   <211> 60
   <212> DNA
   <213> Artificial
<220>
   <223> A_32_P378729
<400> 391
   tatccctttt tccatgacac taagcaatgc agaaacaagt gccgaaaggg tcagaagcac 60
<210> 392
   <211> 60
   <212> DNA
   <213> Artificial
<220>
   <223> A_23_P5211
<400> 392
   agcatcaaga gttatttttc tgactgtcaa gtttcaacat tcaggtctgt ccccaacagg 60
<210> 393
   <211> 60
   <212> DNA
   <213> Artificial
<220>
   <223> A_23_P210428
<400> 393
   tgagtcctgg gatcagacac cccttcacgt gtatccccac acaaatgcaa gctcaccaag 60
<210> 394
   <211> 60
   <212> DNA
   <213> Artificial
<220>
   <223> A_23_P210425
<400> 394
   tgaggaagtg gacgagatgt accgggaggc acccattgat aagaaaggca acttcaacta 60
<210> 395
   <211> 60
   <212> DNA
   <213> Artificial
<220>
   <223> A_01_P014392
<400> 395
   tgtgctcagg agttgcgggc agcatggaca tctgtcccag aggaggcaga atctccaata 60
<210> 396
   <211> 60
   <212> DNA
   <213> Artificial
<220>
   <223> A_24_P244800
<400> 396
   ttttcagcca ggaagagctc tctggaaatt ctgagttgat acaaaagtac agaaatatca 60
<210> 397
   <211> 60
   <212> DNA
   <213> Artificial
<220>
   <223> A_23_ P37205
<400> 397
   cgtttggctg cactaacttt ggtagctcag tgtgcatcta gagtgggact ggggagggag 60
<210> 398
   <211> 60
   <212> DNA
   <213> Artificial
<220>
   <223> A_23_P119042
<400> 398
   gttttgagcg ttgtattcca aaggcctcat ctggagcctc gggaaagtct ggtcccacat 60
<210> 399
   <211> 60
   <212> DNA
   <213> Artificial
<220>
   <223> A_01_P015240
<400> 399
   aggaagctga gcagatccct gtgatgcctg tgacctcaat taaagcaatt cctttgacct 60
<210> 400
   <211> 60
   <212> DNA
   <213> Artificial
<220>
   <223> A_23_P127584
<400> 400
   ctgctgtgaa agaggctggc tacacaatcg aatggtttga ggtgatctcg caaagttatt 60
<210> 401
   <211> 60
   <212> DNA
   <213> Artificial
<220>
   <223> A_23_P140256
<400> 401
   ctactagctc tttgagataa tacattccga ggggctcagt tctgccttat ctaaatcacc 60
<210> 402
   <211> 60
   <212> DNA
   <213> Artificial
<220>
   <223> A_01_P012780
<400> 402
   agcagctttt gactgtttcc agagtgctta taatatacat aactccctgg aaattactga 60
<210> 403
   <211> 60
   <212> DNA
   <213> Artificial
<220>
   <223> A_23_P131207
<400> 403
   ctaaatgttg cgtgggtggc atgagttgaa gaaggcaaag gcttgtaaat ttacccaatg 60
<210> 404
   <211> 60
   <212> DNA
   <213> Artificial
<220>
   <223> A_23_P131208
<400> 404
   aagttttctg ctgtaaagaa agctgtaata tatagtaaaa ctaaatgttg cgtgggtggc 60
<210> 405
   <211> 60
   <212> DNA
   <213> Artificial
<220>
   <223> A_23_P213695
<400> 405
   tataacaaaa tgttttattt tcattttagc aaaaattgtc ttataatact agctaacggc 60
<210> 406
   <211> 60
   <212> DNA
   <213> Artificial
<220>
   <223> A_01_P018499
<400> 406
   gctaacggca aagacgtttt tatagggaaa ctatttatat gtaacatcct gatttacagc 60
<210> 407
   <211> 60
   <212> DNA
   <213> Artificial
<220>
   <223> A_01_P009356
<400> 407
   catctgcgtg gctctgctgg tcgtgggcat cgtctgtgtg gtggcctact gcaagaccaa 60
<210> 408
   <211> 60
   <212> DNA
   <213> Artificial
<220>
   <223> A_23_P349857
<400> 408
   ggacagagat gtttggagaa actgcctttg cgattgtaca tgccagatcc taagcaaagt 60
<210> 409
   <211> 60
   <212> DNA
   <213> Artificial
<220>
   <223> A_23_P213699
<400> 409
   ctagctaacg gcaaaggcgt ttttataggg aaactattta tatgtaacat cctgatttac 60
<210> 410
   <211> 60
   <212> DNA
   <213> Artificial
<220>
   <223> A_24_P134653
<400> 410
   ctgcaatcta gtgacaaagt cgaaagttta acaggctttt ctcatgaaga actagacgac 60
<210> 411
   <211> 60
   <212> DNA
   <213> Artificial
<220>
   <223> A_23_P254226
<400> 411
   tagttcccct gattctgacc ttgagtttgt agccaatact aaggcaaggg tcaaagagct 60
<210> 412
   <211> 60
   <212> DNA
   <213> Artificial
<220>
   <223> A_01_P014581
<400> 412
   ttttctcacg aagaaccaga tgactcttgg taaccatgtt tgctgcccag cttctaactt 60
<210> 413
   <211> 60
   <212> DNA
   <213> Artificial
<220>
   <223> A_01_P010547
<400> 413
   aagggccctc caagccttaa tggcaccctt aagcctccat gcccaggcca aaagatgctt 60
<210> 414
   <211> 60
   <212> DNA
   <213> Artificial
<220>
   <223> A_23_P219161
<400> 414
   gggctaactt aaaagagttt tttcaatgct gcagtgactg aagaagcagt ccactcccat 60
<210> 415
   <211> 60
   <212> DNA
   <213> Artificial
<220>
   <223> A_23_P304311
<400> 415
   agggctatgc ctgtgtctta ttgagacacc ttggcaaaga gatggctgat tctgggtggt 60
<210> 416
   <211> 60
   <212> DNA
   <213> Artificial
<220>
   <223> A_24_P406601
<400> 416
   tcaaaggaat tactctcttc ttgttaaatt agctaaatca tgtaaccgca gataggaagg 60
<210> 417
   <211> 60
   <212> DNA
   <213> Artificial
<220>
   <223> A_24_P181254
<400> 417
   tttttccttt gatgttcaag tcctagtcta taggattggc agtttaaatg ctttactccc 60
<210> 418
   <211> 60
   <212> DNA
   <213> Artificial
<220>
   <223> A_23_P2789
<400> 418
   ttagatatct gcaggggtgt ctaaaagtgt gttcattttg cagcaatgtt taggtgcata 60
<210> 419
   <211> 60
   <212> DNA
   <213> Artificial
<220>
   <223> A_23_P169061
<400> 419
   ttgaaggcaa agatcatcaa tatctgcatc tggctgctgt cgtcatctgt tggcatctct 60
<210> 420
   <211> 60
   <212> DNA
   <213> Artificial
<220>
   <223> A_32_P33576
<400> 420
   cagttcttaa acgtgtataa ctattgtcag acaatttata ggtgtttcat ctagtcctgg 60
<210> 421
   <211> 60
   <212> DNA
   <213> Artificial
<220>
   <223> A_32_P33572
<400> 421
   gaaaatcaca tccaaaaacg gtataaccca gcttccttaa ggcaattttc ttctctgaaa 60
<210> 422
   <211> 60
   <212> DNA
   <213> Artificial
<220>
   <223> A_01_P003195
<400> 422
   tagtcgtgga gatgtcttcg tacagttctt caggaagaga ggagttcaat gatctgggtt 60
<210> 423
   <211> 60
   <212> DNA
   <213> Artificial
<220>
   <223> A_23_P257129
<400> 423
   tacggtggcg aacgaggcca cgctgctcga tactgactac gacaatttcc tgtttctctg 60
<210> 424
   <211> 60
   <212> DNA
   <213> Artificial
<220>
   <223> A_23_P397334
<400> 424
   caaggccccc tctgtccttt tcagaacaca tggacttgga ggcagatttg aaataaactt 60
<210> 425
   <211> 60
   <212> DNA
   <213> Artificial
<220>
   <223> A_23_P397341
<400> 425
   gccatgttgc cacatgagca agcttgggtg ctcccaaggt tcaaatactt tttattagac 60
<210> 426
   <211> 60
   <212> DNA
   <213> Artificial
<220>
   <223> A_23_P66213
<400> 426
   tttccctcca agctcctatt ttactgtgtc agctggaagg aaacctttcc ctcttgggac 60
<210> 427
   <211> 60
   <212> DNA
   <213> Artificial
<220>
   <223> A_23_P66211
<400> 427
   ttactgtgtc agctggaagg aaacctttcc ctcttgggac ctctttaccc tctgtgacct 60
<210> 428
   <211> 60
   <212> DNA
   <213> Artificial
<220>
   <223> A_32_P62997
<400> 428
   agctctggaa acagatgtct agtgatcatc tcagctgaag tgtggcttgc gtaaataact 60
<210> 429
   <211> 60
   <212> DNA
   <213> Artificial
<220>
   <223> A_23_P82699
<400> 429
   attggctata agcacttgga attgtactgg gttttctgta aagttttaga aactagctac 60
<210> 430
   <211> 60
   <212> DNA
   <213> Artificial
<220>
   <223> A_23_P156852
<400> 430
   cttcagggaa gatggctatc agatgaatgc acaaatgctg tggtgaactt cttatccaga 60
<210> 431
   <211> 60
   <212> DNA
   <213> Artificial
<220>
   <223> A_23_P254584
<400> 431
   ttcattgtct ggataactat acaacctgaa aactgtcatt tcaggttctg tgctcttttt 60
<210> 432
   <211> 60
   <212> DNA
   <213> Artificial
<220>
   <223> A_23_P417918
<400> 432
   tttgctagcc aaaaggtatg ggggcttcat gaaaaggtat ggaggcttca tgaagaaaat 60

<210> 433
   <211> 60
   <212> DNA
   <213> Artificial
<220>
   <223> A_01_P015221
<400> 433
   catttcaggt tctgtgctct ttttggagtc cttaagctca gtattggtct gttgcagcta 60
<210> 434
   <211> 60
   <212> DNA
   <213> Artificial
<220>
   <223> A_23_P29816
<400> 434
   gagaaaagca aagctctttc ttattttcct cataatcagc taccctggag gggagggaga 60
<210> 435
   <211> 60
   <212> DNA
   <213> Artificial
<220>
   <223> A_24_P294408
<400> 435
   ttacctgaac cagtgaactt acaagcaagt gtgactgttt cctgtgacct gaagatagcc 60
<210> 436
   <211> 60
   <212> DNA
   <213> Artificial
<220>
   <223> A_23_P127367
<400> 436
   ctgctgcctg tctccctgac ccatgatctg gcaagttagg cacagtcaga catggacagt 60
<210> 437
   <211> 60
   <212> DNA
   <213> Artificial
<220>
   <223> A_01_P004358
<400> 437
   ggagagtggt cactggggaa aaggacctgg ccatcacctt ccagtacctg ctgcctgtct 60
<210> 438
   <211> 60
   <212> DNA
   <213> Artificial
<220>
   <223> A_23_P360215
<400> 438
   ctatgaggca ccacgtaaga cctcctgccc ttagctctct tgctcaccac ccaagaacct 60
<210> 439
   <211> 60
   <212> DNA
   <213> Artificial
<220>
   <223> A_24_P347411
<400> 439
   agaatctgac atcatgacaa caaatggtgt aattcatgtt gtagataaac tcctctatcc 60
<210> 440
   <211> 60
   <212> DNA
   <213> Artificial
<220>
   <223> A_23_P205111
<400> 440
   aggaagttgc aagccaacaa aaaagttcaa ggatctagaa gacgattaag ggaaggtcgt 60
<210> 441
   <211> 60
   <212> DNA
   <213> Artificial
<220>
   <223> A_23_P18443
<400> 441
   ttgagccctt gccgggcctt ttttccacct gccaattcta catgtattgt tgtggtttta 60
<210> 442
   <211> 60
   <212> DNA
   <213> Artificial
<220>
   <223> A_24_P303052
<400> 442
   tctccaggca gtagatcctc ttcaagatcc tgctattact atgagtcaag ccactacaga 60
<210> 443
   <211> 60
   <212> DNA
   <213> Artificial
<220>
   <223> A_23_P18447
<400> 443
   aatcactgta gtctaagacc tgatctatag atgacctaga atagccatgt actataatgt 60
<210> 444
   <211> 60
   <212> DNA
   <213> Artificial
<220>
   <223> A_01_P016440
<400> 444
   ttttttaaat gtgcagtaca catcagcctc actgagctaa taaagggaaa cgaatgtttc 60
<210> 445
   <211> 60
   <212> DNA
   <213> Artificial
<220>
   <223> A_23_P206059
<400> 445
   tctcaatcac tactcttctt gaagcactat tatttattct tccgctgtct gcctgcagca 60
<210> 446
   <211> 60
   <212> DNA
   <213> Artificial
<220>
   <223> A_23_P1374
<400> 446
   actgatcaac agcatggacc agaatatgtt caggaacttt tccttcatga accccgggat 60
<210> 447
   <211> 60
   <212> DNA
   <213> Artificial
<220>
   <223> A_23_P258463
<400> 447
   tggggtgttt gttcccattg gatgcatttc tatcaaaact ctatcaaatg tgatggctag 60
<210> 448
   <211> 60
   <212> DNA
   <213> Artificial
<220>
   <223> A_01_P010711
<400> 448
   tcaaatgtga tggctagatt ctaacatatt gccatgtgtg gagtgtgctg aacacacacc 60
<210> 449
   <211> 60
   <212> DNA
   <213> Artificial
<220>
   <223> A_23_P258462
<400> 449
   tcaaatgtga tggctagatt ctaacatatt gccatgtgtg gagtgtgctg aacacacacc 60
<210> 450
   <211> 60
   <212> DNA
   <213> Artificial
<220>
   <223> A_24_P119141
<400> 450
   agcagcacag gaatcttact tcttggcagc tgcagtctgt caagatgaga catcagatta 60
<210> 451
   <211> 60
   <212> DNA
   <213> Artificial
<220>
   <223> A_23_P73114
<400> 451
   ccagaacaaa ttttaacaaa aggacaacca cagagggata tagtgaatat cgtatcattg 60
<210> 452
   <211> 60
   <212> DNA
   <213> Artificial
<220>
   <223> A_32_P500684
<400> 452
   tatacgaagc tggaatttga tgaagcaagg agcttctgga ataaaggaaa ttattcaaga 60
<210> 453
   <211> 60
   <212> DNA
   <213> Artificial
<220>
   <223> A_23_P84510
<400> 453
   tggtactgtg atgcatttca agtggcagtt ttatcacgtt tgaatctacc attcatagcc 60
<210> 454
   <211> 60
   <212> DNA
   <213> Artificial
<220>
   <223> A_24_P383480
<400> 454
   atttgatttc cggacatatg attcagaagg cgtgatactg tacgcagaat ctatcgatca 60
<210> 455
   <211> 60
   <212> DNA
   <213> Artificial
<220>
   <223> A_23_P140805
<400> 455
   cagtgaagcc actaaccctg gagctagtgg aggaaactgt gcaggctatg gaggtggagt 60
<210> 456
   <211> 60
   <212> DNA
   <213> Artificial
<220>
   <223> A_23_P140807
<400> 456
   acactgagct cacccacaga gcccgtgaag aggtctggcc gctaccactt tgtgcctgga 60
<210> 457
   <211> 60
   <212> DNA
   <213> Artificial
<220>
   <223> A_23_P151710
<400> 457
   ccagctgcct attgatttaa gctttcctgt tgaatgacaa agtatgtggt tttgtaattt 60
<210> 458
   <211> 60
   <212> DNA
   <213> Artificial
<220>
   <223> A_01_P012436
<400> 458
   gaacatggtt tgactcatct tatatgggaa accatgtagc agtgagtcat atcttaatat 60
<210> 459
   <211> 60
   <212> DNA
   <213> Artificial
<220>
   <223> A_23_P47924
<400> 459
   tcttgtgaat ggactgtcag ctgttaaact gttcctgttt tgaagtgcta ttacctttct 60
<210> 460
   <211> 60
   <212> DNA
   <213> Artificial
<220>
   <223> A_32_P770346
<400> 460
   tgatcatttt ttggcaatta atcagaagaa gagtgggaag ccggtattca tttataagca 60
<210> 461
   <211> 60
   <212> DNA
   <213> Artificial
<220>
   <223> A_32_P461885
<400> 461
   agaccaaaaa atgtaaccga ttcattgagc acctacatta atgctaatta tattaggggc 60
<210> 462
   <211> 60
   <212> DNA
   <213> Artificial
<220>
   <223> A_32_P480310
<400> 462
   gttttaacta cttgtctctc ttttgctaag aagggatttt tgaatatgct atctacctgg 60
<210> 463
   <211> 60
   <212> DNA
   <213> Artificial
<220>
   <223> A_01_P017426
<400> 463
   actcaagcta ctggcacata atgaaagatt acttcatgac attccattgc tcttcttttg 60
<210> 464
   <211> 60
   <212> DNA
   <213> Artificial
<220>
   <223> A_23_P82738
<400> 464
   caagctactg gcacatagtg aaagattact tctgacattc cattgctctt cttttgaaaa 60
<210> 465
   <211> 60
   <212> DNA
   <213> Artificial
<220>
   <223> A_23_P94141
<400> 465
   aaattttgga ttgtatgttc aggagaagag ggatggattg aaaagaaggc agcagctaga 60
<210> 466
   <211> 60
   <212> DNA
   <213> Artificial
<220>
   <223> A_23_P58819
<400> 466
   tctgggtctc aggacagtga tgttggctag cccaggggaa tgtatttttc aaaacataca 60
<210> 467
   <211> 60
   <212> DNA
   <213> Artificial
<220>
   <223> A_01_P013828
<400> 467
   taagacatga aaggttggcc ttactgttga acaagaagta aatccacagg ctcctgttat 60
<210> 468
   <211> 60
   <212> DNA
   <213> Artificial
<220>
   <223> A_23_P18078
<400> 468
   tgagactcat ctgggatttg gctttccttg gaagctctta cgtgatgtgg gaaatgacaa 60
<210> 469
   <211> 60
   <212> DNA
   <213> Artificial
<220>
   <223> A_23_P1962
<400> 469
   aggcgttctc tagatccttt cctctgtttc cctctctcgc tggcaaaagt atgatctaat 60
<210> 470
   <211> 60
   <212> DNA
   <213> Artificial
<220>
   <223> A_23_P166087
<400> 470
   gatggtacat gacttgattc aacgtttggt tctgaactta cacactgatg cgtttactca 60
<210> 471
   <211> 60
   <212> DNA
   <213> Artificial
<220>
   <223> A_23_P205531
<400> 471
   ggttagatgc caccatgtag ggattatcgc gagtggttga ccttacactt actccttaaa 60
<210> 472
   <211> 60
   <212> DNA
   <213> Artificial
<220>
   <223> A_01_P017372
<400> 472
   gcttttctgt aataagcttc cttttataat agtgctcagc ttagctctct cagatcctat 60
<210> 473
   <211> 60
   <212> DNA
   <213> Artificial
<220>
   <223> A_23_P5365
<400> 473
   tgcacggttt acgccacaaa agtgctcttg acatccgtga caccgttttg actttttgtt 60
<210> 474
   <211> 60
   <212> DNA
   <213> Artificial
<220>
   <223> A_01_P012092
<400> 474
   gggctactgc cactgtgtgc cttccgccaa cacctcctgt ccccacctaa gcctggtggg 60
<210> 475
   <211> 60
   <212> DNA
   <213> Artificial
<220>
   <223> A_23_P39076
<400> 475
   tagctgcctt cgcaccttgc tgtgtgacct gaggccctca ctgagcctca atttcctcat 60
<210> 476
   <211> 60
   <212> DNA
   <213> Artificial
<220>
   <223> A_23_P39074
<400> 476
   gtgggggtga ggggctccgg gtcactgctg tatataactc ccctccccca gaaaaataaa 60
<210> 477
   <211> 60
   <212> DNA
   <213> Artificial
<220>
   <223> A_23_P383227
<400> 477
   ctcacagtgg cctgtaacaa tttcttctgg gagaacagtt gagcagacag ccacattggg 60
<210> 478
   <211> 60
   <212> DNA
   <213> Artificial
<220>
   <223> A_23_P94800
<400> 478
   ataagcagcc caggaagaaa tgaaaactcc tctgatgtgg ttggggggtc tgccagctgg 60
<210> 479
   <211> 60
   <212> DNA
   <213> Artificial
<220>
   <223> A_32_P900698
<400> 479
   cttccacaag tactcgggca aagagggtga caagttcaag ctcaacaagt cagaactaaa 60
<210> 480
   <211> 60
   <212> DNA
   <213> Artificial
<220>
   <223> A_23_P58266
<400> 480
   ccaaaagtgt ttgttggcaa ttattcccct aggctgagcc tgctcatgta cctctgatta 60
<210> 481
   <211> 60
   <212> DNA
   <213> Artificial
<220>
   <223> A_23_P161940
<400> 481
   cttaaccaaa cggatgaaac tctgagcaat gttgaggtgt ttatgcaatt aatatatgac 60
<210> 482
   <211> 60
   <212> DNA
   <213> Artificial
<220>
   <223> A_01_P016031
<400> 482
   cttaaccaaa cggatgaaac tctgagcaat gttgaggtgt ttatgcaatt aatatatgac 60
<210> 483
   <211> 60
   <212> DNA
   <213> Artificial
<220>
   <223> A_01_P002141
<400> 483
   aacataaaag ctaggagatg tggcatctga acatttttgc tttgctgcca gagtaaccct 60
<210> 484
   <211> 60
   <212> DNA
   <213> Artificial
<220>
   <223> A_23_P121686
<400> 484
   ggtcatgtgt gctagttcac cagagaaaat tgaaatcttg gctcctccaa atgggtctgt 60
<210> 485
   <211> 60
   <212> DNA
   <213> Artificial
<220>
   <223> A_23_P205355
<400> 485
   aacaactgtc agttcatcct gcatgggaaa aatgttggaa tgggagtctg aaatggggct 60
<210> 486
   <211> 60
   <212> DNA
   <213> Artificial
<220>
   <223> A_24_P321766
<400> 486
   gtatgctggt agctagtgat ttacacaggt ttagttgact aatgaggcat tacaaataat 60
<210> 487
   <211> 60
   <212> DNA
   <213> Artificial
<220>
   <223> A_23_P139114
<400> 487
   catgctctcc aaaccacttt ttgcagcttt ctctagttca agttcaccag actctataaa 60
<210> 488
   <211> 60
   <212> DNA
   <213> Artificial
<220>
   <223> A_23_P139123
<400> 488
   gacaacattt gatcccaaga aaaccagaat ggaacccttt cacttcaaaa actcagttat 60
<210> 489
   <211> 60
   <212> DNA
   <213> Artificial
<220>
   <223> A_23_P215328
<400> 489
   tgtgtgcata ctctagaaga gtagggaaaa taatgcttgt tacaattcga cctaatatgt 60
<210> 490
   <211> 60
   <212> DNA
   <213> Artificial
<220>
   <223> A_24_P8165
<400> 490
   tgaccactgg agagtctaag ccatccttag catgggcatc catcttccta aactgttatt 60
<210> 491
   <211> 60
   <212> DNA
   <213> Artificial
<220>
   <223> A_23_P128609
<400> 491
   gtagtgctta tacctatata gtccaaagga agaatgacag ctgccctgaa gtgaaggtgt 60
<210> 492
   <211> 60
   <212> DNA
   <213> Artificial
<220>
   <223> A_23_P92107
<400> 492
   ataccctgca gtgcactgta gaacagaaga taagaacttt tctctgaatt tgggtcttct 60
<210> 493
   <211> 60
   <212> DNA
   <213> Artificial
<220>
   <223> A_01_P004018
<400>. 493
   tatgtatgat gtgatctggt ccagccaggg cctggtttgt cagctatcta ggtttgataa 60
<210> 494
   <211> 60
   <212> DNA
   <213> Artificial
<220>
   <223> A_23_P152791
<400> 494
   gagatatgta gaaagactct ttggttcaca ttccgatatt aaaatagtga catgaactgg 60
<210> 495
   <211> 60
   <212> DNA
   <213> Artificial
<220>
   <223> A_01_P007822
<400> 495
   aatagttttt gttaaacctt ttgtaaagta ccaaggctcc cattaacaaa ttacggcctc 60
<210> 496
   <211> 60
   <212> DNA
   <213> Artificial
<220>
   <223> A_24_P731648
<400> 496
   actacatttt aaagggaatg tgtatgtgaa gagcactacc aacatcgctt ttgttttaag 60
<210> 497
   <211> 60
   <212> DNA
   <213> Artificial
<220>
   <223> A_01_P003853
<400> 497
   agatagcaga agagtaaata agtactcagt attgaccacc tacatctgaa atctacaaca 60
<210> 498
   <211> 60
   <212> DNA
   <213> Artificial
<220>
   <223> A_23_P216468
<400> 498
   gaaatccaaa atagtcatgt ttctgcagta ttctgtagcc aacttaaacc tgtgctttca 60
<210> 499
   <211> 60
   <212> DNA
   <213> Artificial
<220>
   <223> A_24_P232252
<400> 499
   cattgtgaat ccctttgcct tggaatccac aatccttgac aacgaagact cagacaccaa 60
<210> 500
   <211> 60
   <212> DNA
   <213> Artificial
<220>
   <223> A_23_P363399
<400> 500
   ttggggatga ttcttacctt ggtaattaaa tgaagctaca catttgggta atctagcaaa 60
<210> 501
   <211> 60
   <212> DNA
   <213> Artificial
<220>
   <223> A_01_P001379
<400> 501
   gtacctaagt aaggatgatc taggataagt aactcctgtt ttatattgag tactttaggg 60
<210> 502
   <211> 60
   <212> DNA
   <213> Artificial
<220>
   <223> A_23_P117242
<400> 502
   gtgaacattt caaccagcct tatagctgtt ctcatcatca ccttctgcat tgtgaccgtg 60
<210> 503
   <211> 60
   <212> DNA
   <213> Artificial
<220>
   <223> A_24_P253251
<400> 503
   caaggcaaat gattttgtgt ttcttgatga cagactatta agtttgggac ttattttccc 60
<210> 504
   <211> 60
   <212> DNA
   <213> Artificial
<220>
   <223> A_23_P91230
<400> 504
   aggtcctttc caccctgaga cttggctcca ccactgatat cctcctttgg ggaaaggctt 60
<210> 505
   <211> 60
   <212> DNA
   <213> Artificial
<220>
   <223> A_01_P005468
<400> 505
   acagcaggat ttcaggaagt gccagttgat caatgaataa ataaatgagc ctatttctct 60
<210> 506
   <211> 60
   <212> DNA
   <213> Artificial
<220>
   <223> A_24_P190472
<400> 506
   aagagatgtt gtcctgacac ttgtggcatc aaatgcctgg atcctgttga caccccaaac 60
<210> 507
   <211> 60
   <212> DNA
   <213> Artificial
<220>
   <223> A_23_P8513
<400> 507
   tttaagagcg atcctcatcc cttcagcaat atgtatttga gttcacacta tttctgtttt 60
<210> 508
   <211> 60
   <212> DNA
   <213> Artificial
<220>
   <223> A_24_P98109
<400> 508
   aagagtggca gaggctacta caaaaagcaa ccttttcatt ttcactaaga gtttaaaagc 60
<210> 509
   <211> 60
   <212> DNA
   <213> Artificial
<220>
   <223> A_24_P935819
<400> 509
   gaagataatc gatagtcatg ttttttagac tctctgtatt gcttggtaag ctacgtagta 60
<210> 510
   <211> 60
   <212> DNA
   <213> Artificial
<220>
   <223> A_23_P134176
<400> 510
   ttgatgtgtg ggagcacgct tactaccttc agtataaaaa tgtcaggcct gattatctaa 60
<210> 511
   <211> 60
   <212> DNA
   <213> Artificial
<220>
   <223> A_32_P89691
<400> 511
   cgtctgggat gaaggcttgt cagcacttcc agtttagaac gcaatgtttc tagagacata 60
<210> 512
   <211> 60
   <212> DNA
   <213> Artificial
<220>
   <223> A_32_P85311
<400> 512
   ctctggagaa agctctggag gcctttgaaa catttaaaaa gggattgggg ttgaaaatca 60
<210>' 513
   <211> 60
   <212> DNA
   <213> Artificial
<220>
   <223> A_32_P127153
<400> 513
   gtagccatga catagcttga gctatagcct ttaattcctt actttggcta tgggtggagg 60
<210> 514
   <211> 60
   <212> DNA
   <213> Artificial
<220>
   <223> A_23_P77103
<400> 514
   cagcacttcc agtttagaac gcaatgtttc tagagacata ttggctgttt gttttgatga 60
<210> 515
   <211> 60
   <212> DNA
   <213> Artificial
<220>
   <223> A_01_P000174
<400> 515
   atgagggctg agttatgaaa agataacttc tgaagactta actggcccag aagctgattt 60
<210> 516
   <211> 60
   <212> DNA
   <213> Artificial
<220>
   <223> A_23_P82775
<400> 516
   accctatttc caagttcaag ttaactagct ttgaatgtgt cccaaaacag cttcctccat 60
<210> 517
   <211> 60
   <212> DNA
   <213> Artificial
<220>
   <223> A_01_P001801
<400> 517
   gtcccaaaac agcttcctcc atttcctgaa agtttattga tcaaagaaat gttgtcccgg 60
<210> 518
   <211> 60
   <212> DNA
   <213> Artificial
<220>
   <223> A_24_P46946
<400> 518
   ctgggcgcgc ctccgctcga cggctacccg ttgcccacgc ccgacacgtc cccgctggac 60
<210> 519
   <211> 60
   <212> DNA
   <213> Artificial
<220>
   <223> A_23_P111194
<400> 519
   ccgccatgaa ctacgacaag ctgagccgct ccatccgcca gtattacaag aagggcatca 60
<210> 520
   <211> 60
   <212> DNA
   <213> Artificial
<220>
   <223> A_23_P7313
<400> 520
   ttccacagcc atgaatttca cagccatgaa gatatgctgg ttgtagaccc caaaagtaag 60
<210> 521
   <211> 60
   <212> DNA
   <213> Artificial
<220>
   <223> A_01_P017618
<400> 521
   gaatgtaata agaatctggt ggtgtcaatt gcttacttgt tttcccacag ttgtccagca 60
<210> 522
   <211> 60
   <212> DNA
   <213> Artificial
<220>
   <223> A_32_P527817
<400> 522
   gaaatatagt ccaagctttc tctgtggaaa aagacaaaac tcattagtag acatgtttcc 60
<210> 523
   <211> 60
   <212> DNA
   <213> Artificial
<220>
   <223> A_23_P8981
<400> 523
   tcattagtag acatgtttcc ctattgcttt cataggcacc agtcagaata aagaatcata 60
<210> 524
   <211> 60
   <212> DNA
   <213> Artificial
<220>
   <223> A_24_P351906
<400> 524
   atatcccctt ggatttcact tgcattgtgc aataagcaaa gaagggttga taaaagttct 60
<210> 525
   <211> 60
   <212> DNA
   <213> Artificial
<220>
   <223> A_23_P255231
<400> 525
   ctggaagaga acaccatttt atctcaggtt agtgaagaat cagtgcaggt ccctgactct 60
<210> 526
   <211> 60
   <212> DNA
   <213> Artificial
<220>
   <223> A_32_P109727
<400> 526
   aattaaagag gtaccctttg agacactagc ccagtggaat ctagaacacg ctactttaaa 60
<210> 527
   <211> 60
   <212> DNA
   <213> Artificial
<220>
   <223> A_23_P205553
<400> 527
   agggaactaa tgcaactgga aaaggagctg gtagaacgtc aacctcaagt ggacatgtta 60
<210> 528
   <211> 60
   <212> DNA
   <213> Artificial
<220>
   <223> A_23_P128887
<400> 528
   aacctcaagt ggacatgtta caggagattt caaacagcct tctcattaag ggacatggag 60
<210> 529
   <211> 60
   <212> DNA
   <213> Artificial
<220>
   <223> A_23_P140277
<400> 529
   aatctgggag gcagaagcca aatctgtttt ggatcaagat gatgtggaca cctcaatgga 60
<210> 530
   <211> 60
   <212> DNA
   <213> Artificial
<220>
   <223> A_24_P68311
<400> 530
   caatgcgcca cctatgagtc tgtcaatgtg accgatttta agtcaagttg gagaaatggg 60
<210> 531
   <211> 60
   <212> DNA
   <213> Artificial
<220>
   <223> A_24_P307759
<400> 531
   aagaagctaa agagaaagtc cagatcaatg tggtaaaact cattgcagcg ttgaagaact 60
<210> 532
   <211> 60
   <212> DNA
   <213> Artificial
<220>
   <223> A_23_P212844
<400> 532
   agctccacgg gaagaacctg gaactgggga agatcatgga caggttcgaa gaggttgtgt 60
<210> 533
   <211> 60
   <212> DNA
   <213> Artificial
<220>
   <223> A_01_P002824
<400> 533
   ttgaagaaga accagcccag cctgcctcct atcttttcct ggaatatttt tggggttgga 60
<210> 534
   <211> 60
   <212> DNA
   <213> Artificial
<220>
   <223> A_23_P87011
<400> 534
   acccgtgtgg taccttcagc cctggccaag ctttgaggct ctgtcactga gcaatggtaa 60
<210> 535
   <211> 60
   <212> DNA
   <213> Artificial
<220>
   <223> A_23_P87013
<400> 535
   ccgtggagat cccaactggt ttatgaagaa agcgcaggag cataagaggg aattcacaga 60
<210> 536
   <211> 60
   <212> DNA
   <213> Artificial
<220>
   <223> A_23_P123662
<400> 536
   caagcaagag cactgcctct atagggtaac ctggaacatt ctctaggtta tatcaatata 60
<210> 537
   <211> 60
   <212> DNA
   <213> Artificial
<220>
   <223> A_23_P123666
<400> 537
   gccggaagct gatgaacatc gccttcaatg acatgaaccc cttccgcatg aaacagctgc 60
<210> 538
   <211> 60
   <212> DNA
   <213> Artificial
<220>
   <223> A_23_P64372
<400> 538
   ccagaaaatg aatgatacta tatttggttt cacaatggag gagcgctcat gggggcccta 60
<210> 539
   <211> 60
   <212> DNA
   <213> Artificial
<220>
   <223> A_01_P009817
<400> 539
   tccgcccttc cctgacactg tctgctgccc caatcgccgt cacaataaaa gaaagtgtgg 60
<210> 540
   <211> 60
   <212> DNA
   <213> Artificial
<220>
   <223> A_24_P924602
<400> 540
   tcacaggctc tccgtggcct ggaactgcag ccccagctgc atcctacacc cccaccccaa 60
<210> 541
   <211> 60
   <212> DNA
   <213> Artificial

<220>
   <223> A_23_P258633
<400> 541
   ctgtccacgc tgtactggtt cacggtggag ttcgggctgt gtaagcagaa cggggaggtg 60
<210> 542
   <211> 60
   <212> DNA
   <213> Artificial
<220>
   <223> A_23_P95891
<400> 542
   ggatgaggaa attgagaagc tgtccacgct gtcatggttc acggtggagt tcgggctgtg 60
<210> 543
   <211> 60
   <212> DNA
   <213> Artificial
<220>
   <223> A_01_P013083
<400> 543
   ttccagactg cttccaattt ttctggaaca cattaaatat ggatcagtta taagtagcag 60
<210> 544
   <211> 60
   <212> DNA
   <213> Artificial
<220>
   <223> A_23_P91390
<400> 544
   acagggtctg caaggtcttt ggttcagcta agctaggaat gaaatcctgc ttcagtgtat 60
<210> 545
   <211> 60
   <212> DNA
   <213> Artificial
<220>
   <223> A_32_P192334
<400> 545
   tctttctgta accataacaa cttcatatat gaggacttgt gtctctgtgc ttttaaatgc 60
<210> 546
   <211> 60
   <212> DNA
   <213> Artificial
<220>
   <223> A_23_P253652
<400> 546
   actttttact tttttgcgtg tggagctgta ttcccgagac caacgaagcg ttgggatact 60
<210> 547
   <211> 60
   <212> DNA
   <213> Artificial
<220>
   <223> A_23_P62021
<400> 547
   tcgttgtgtt gttttgctgc actttttact tttttgcgtg tggagctgta ttcccgagac 60
<210> 548
   <211> 60
   <212> DNA
   <213> Artificial
<220>
   <223> A_24_P605612
<400> 548
   tgtggagtga aaattgggca tgccattaca ttgctttttc ttggtggtta aaaagaatga 60
<210> 549
   <211> 60
   <212> DNA
   <213> Artificial
<220>
   <223> A_23_P399078
<400> 549
   caagatggca cccaagtgtt tggcttctgg ctacctaagg ttaacatgtc actagagtat 60
<210> 550
   <211> 60
   <212> DNA
   <213> Artificial
<220>
   <223> A_01_P005541
<400> 550
   ctgtctagaa ggaatgtatt tgttgctaaa ttttgtagca ctgtttacag ttttcctcca 60
<210> 551
   <211> 60
   <212> DNA
   <213> Artificial
<220>
   <223> A_01_P014722
<400> 551
   ccttttatgg aaaccgtttt ttaaaaaagt gaatgtacac aaatccacag aagactgtgg 60
<210> 552
   <211> 60
   <212> DNA
   <213> Artificial
<220>
   <223> A_24_P87036
<400> 552
   atgagtgatc taaatttgca gcaatgatac taaacaactc tctgaaattt ctcaagcacc 60
<210> 553
   <211> 60
   <212> DNA
   <213> Artificial
<220>
   <223> A_24_P110831
<400> 553
   cttatctggt gtgttcatat agaatcacct agaaggataa agtcgctgta gagttaatga 60
<210> 554
   <211> 60
   <212> DNA
   <213> Artificial
<220>
   <223> A_23_P98304
<400> 554
   tcagggtttc aagaagtctt agggcttcca ggggtcccct ggaagcttta gaatatttat 60
<210> 555
   <211> 60
   <212> DNA
   <213> Artificial
<220>
   <223> A_01_P015348
<400>. 555
   gaagaaactg cttgttgtgt atcagtaatc attagtggca atgatgacat tctgaaaagc 60
<210> 556
   <211> 60
   <212> DNA
   <213> Artificial
<220>
   <223> A_24_P413126
<400> 556
   aagaaactgc ttgttgtgta tcagtaatca ttagtggcaa tgatgacatt ctgaaaagct 60
<210> 557
   <211> 60
   <212> DNA
   <213> Artificial
<220>
   <223> A_23_P57089
<400> 557
   gccgggctgg ggctgcgtag gtgaaaaggc agaacactcc gcgcttctta gaagaggagt 60
<210> 558
   <211> 60
   <212> DNA
   <213> Artificial
<220>
   <223> A_23_P137173
<400> 558
   tgtagttctt gcatcctata ctggataagc ctgttttaac ctgctatgat gggtgcttcc 60
<210> 559
   <211> 60
   <212> DNA
   <213> Artificial
<220>
   <223> A_23P137179
<400> 559
   tagagatttt aggcgtcttc ggatatcttc tcacctatgt tccctggcta agaagtcaga 60
<210> 560
   <211> 60
   <212> DNA
   <213> Artificial
<220>
   <223> A_23_P118834
<400> 560
   tgtgaggcga ttattttaag taattatctt accaagccca agactggttt taaagttacc 60
<210> 561
   <211> 60
   <212> DNA
   <213>' Artificial
<220>
   <223> A_23_P106061
<400> 561
   ctcacctttg ggacaggcac tcagctagaa gtgggactca atatccagaa ccctgaccct 60
<210> 562
   <211> 60
   <212> DNA
   <213> Artificial
<220>
   <223> A_23_P258504
<400> 562
   atcttcagtg ggttctcttg ggctctaggt cctggagaat gttgtgaggg gtttattttt 60
<210> 563
   <211> 60
   <212> DNA
   <213> Artificial
<220>
   <223> A_32_P806170
<400> 563
   tttctttaac catttttgaa acccttcaaa ggcagagact tgtccagcct aacctgcctg 60
<210> 564
   <211> 60
   <212> DNA
   <213> Artificial
<220>
   <223> A_23_P44857
<400> 564
   atcattcatg gtctaaaaaa caatgaaacc aatgaaatgg cctctctgat catcacagaa 60
<210> 565
   <211> 60
   <212> DNA
   <213> Artificial
<220>
   <223> A_23_P258798
<400> 565
   tagtgttcat aaagaaatac atagtattct tcttctcaag acgtgggggg aaattatctc 60
<210> 566
   <211> 60
   <212> DNA
   <213> Artificial
<220>
   <223> A_23_P132760
<400> 566
   tctggctgtg ggataaatgt gtgtgggaat attgaaacat cgcctaggaa ttgtggtttg 60
<210> 567
   <211> 60
   <212> DNA
   <213> Artificial
<220>
   <223> A_01_P011590
<400> 567
   agcccctttc ccttgtcaat gacagtcatc ctaatgataa taaaacctgc atccagataa 60
<210> 568
   <211> 60
   <212> DNA
   <213> Artificial
<220>
   <223> A_23_P36531
<400> 568
   gctgattggg gaaataattt tcaacactat cctgaattat gtgcctgtct agataagcag 60
<210> 569
   <211> 60
   <212> DNA
   <213> Artificial
<220>
   <223> A_23_P36528
<400> 569
   ttgagatact gggtttggtg ttttctatgg tcctgtattg ccagatcggg aacaaatgaa 60
<210> 570
   <211> 60
   <212> DNA
   <213> Artificial
<220>
   <223> A_23_P5422
<400> 570
   tgatgactct gcttctatga ggtcaccagc agtagaacca tatcttgctg gcatacactt 60
<210> 571
   <211> 60
   <212> DNA
   <213> Artificial
<220>
   <223> A_24_P111511
<400> 571
   atccaactta tccaaaaatc agaaaggata tacttgataa ggcccgtgcg tctttaagac 60
<210> 572
   <211> 60
   <212> DNA
   <213> Artificial
<220>
   <223> A_23_P34345
<400> 572
   aaagccaaca tgaaggggtc atatagtctt gtagaagcac agaaatcaaa agtgtagcta 60
<210> 573
   <211> 60
   <212> DNA
   <213> Artificial
<220>
   <223> A_23_P200752
<400> 573
   ctttactgcg aataagcttt taatgctcca aatgctgacc catgcaatat ttcctcatgt 60
<210> 574
   <211> 60
   <212> DNA
   <213> Artificial
<220>
   <223> A_24_P45476
<400> 574
   agtttatact caccttttat gaaagcactg catgaataaa attattcctt tgtattttta 60
<210> 575
   <211> 60
   <212> DNA
   <213> Artificial
<220>
   <223> A_01_P019136
<400> 575
   cactcttaga gtccagcttg taatggttct ttacacatga gtcacaagtt acagctgtga 60
<210> 576
   <211> 60
   <212> DNA
   <213> Artificial
<220>
   <223> A_23_P321431
<400> 576
   tcactgcata cattgtggaa ggtgtaggga gtgaagtctc acataggagg acctgtgtga 60
<210> 577
   <211> 60
   <212> DNA
   <213> Artificial
<220>
   <223> A_01_P017396
<400> 577
   catgaataaa gttattcctt tgtattttta cttttaaatg tcttctgcat tcacttatat 60
<210> 578
   <211> 60
   <212> DNA
   <213> Artificial
<220>
   <223> A_23_P51534
<400> 578
   ccagttagca gaatcaagac ctacaccatc acggaaggct ccttgagagc agtaattttt 60
<210> 579
   <211> 60
   <212> DNA
   <213> Artificial
<220>
   <223> A_23_P51532
<400> 579
   ctcatttcac tttacaccct catggactga gattatactc accttttatg aaagcactgc 60

## Description

### Field of the Art

The present invention relates to determining the receptivity of the human endometrium from a gene expression profile. This can be accomplished by developing a specific expression microarray of endometrial receptivity (Endometrial Receptivity Array or ERA) which allows evaluating the receptive state of a human endometrium, as well as assessing said state for diagnostic and therapeutic purposes.

### Prior Art

The endometrium is the mucosa coating the inside of the uterine cavity. Its function is to house the embryo, allowing its implantation and favoring the development of the placenta. This process requires a receptive endometrium capable of responding to the signals of the blastocyst, which is the stage of development of the embryo when it implants. Human endometrium is a tissue cyclically regulated by hormones, the hormones preparing it to reach said receptivity state are estradiol, which induces cell proliferation, and progesterone which is involved in differentiation, causing a large number of changes in the gene expression profile of the endometrium, which reaches a receptive phenotype for a short time period referred to as "window of implantation". Though there is no consensus as to the implantation period in humans, clinical studies suggest that this process takes place between days 20 and 24 of a normal ovulation cycle (Wilcox *et al*., 1999), day LH+7 (day 20-21) being considered critical.

The evolution of our knowledge about the human endometrium contrasts with the lack of progress in developing new diagnostic methods for the dating and study thereof. The endometrium is still evaluated today by means of histological studies based on in observations described over 50 years ago (Noyes *et al*., 1950) or with macroscopic techniques with little resolution as equally non-objective ultrasound studies which lack specificity and produce widely varying results.

In 1950, Noyes *et al*. described for the first time a method for endometrial dating based exclusively on histological criteria and on the morphological changes of the different compartments of the endometrium in response to the presence of estrogens and progesterone. Noyes *et al*. studied the histological features of endometrial biopsies taken during 8,000 spontaneous cycles in 300 women (Noyes *et al.*, 1950). They were able to relate different histological patterns with particular moments of the menstrual cycle by correlating the histological changes with the basal body temperature. These morphological criteria continue to be used today and are considered the Gold Standard for the study of the endometrium, evaluation of endometrial receptivity and detection of endometrial anomalies.

However, this technique does have its drawbacks. It has been demonstrated that the use of histological features fails when distinguishing the phase of the menstrual cycle, and it also fails as a means to discriminate between fertile and infertile women, concluding that it is not suitable for clinical use. The subjectivity involved in visual observation means that there is an inter-observer, intra-observer and inter-cycle variability altering the consistency of the results obtained. Furthermore, ovarian stimulation typical of assisted reproductive treatments (ART) modifies the endometrial maturation process compared to natural cycles which can barely be explained with Noyes' criteria (Papanikolaou *et al.*, 2005). For this reason there are many works in the literature which question the histological observations interpreted by one or several pathologies both in retrospective clinical studies (Balash *et al*., 1992; Batista *et al.*, 1993; Shoupe *et al*., 1989), prospective clinical studies (Li *et al.*, 1989; Creus *et al.*, 2002; Ordi *et al.*, 2003), and recently in randomized studies (Murray *et al.*, 2004; Coutifaris *et al.*, 2004). The Practice Committee of the American Society for Reproductive Medicine (ASRM) also establishes that even though the classic criterion of the luteal phase defect consists of a delay in the endometrial maturation of > 2 days following the Noyes criteria, this Committee has serious doubts as to the accuracy of said histological criteria and therefore of the prevalence of the luteal phase defect (LPD) and even of its clinical relevance as a cause of infertility (ASRM, 2000).

In this sense, Balasch *et al*., 1992 demonstrated that the incidence of LPD and histological endometrial patterns were similar in fertile and infertile women. Moreover, a suitable endometrial histology in the ovulation cycle or in previous ones was not related to the pregnancy data in infertile women concluding that the histological evaluation of the endometrium in the luteal phase is not useful for predicting or improving the reproductive results (Balasch *et al*., 1992). In other studies of the same group, it was demonstrated that there was a clear dissociation in the temporary expression of a series of markers related to the window of implantation (alpha and beta 3 integrins) and the pinopod expression. They furthermore did not find differences in the expression of these markers between fertile and infertile women (Creus *et al*., 2002). They also demonstrated a high variability between cycles and low reproducibility for these markers (Ordi *et al.*, 2003).

Li *et al.* 1989 dated 63 endometrial biopsies on two different occasions by the same pathologist, demonstrating that there was complete agreement in only 24% of them. In a separate study, they observed that between different cycles in the same woman, there was complete agreement in only 4% of the cases. These data emphasize the lack of precision of traditional dating methods and their lack of any assurances for predicting the development in the following cycles (Li *et al*., 1989).

The differences between pathologists varied depending on the day of the menstrual cycle in which the endometrial biopsy is taken. Over 20% of the endometrial biopsies were dated with a difference of at least two days between pathologists in the early, mid and late luteal phases. Inter-cycle variations reach 60% in the mid luteal phase (Murray *et al*., 2004). It has been demonstrated that during the window of implantation, a very similar percentage of women has the endometrium out of phase, 49.4% fertile versus 43.2% infertile (p = 0.33) and, ultimately, that the histological dating is not related to fertility status (Coutifaris *et al*., 2004). These variations described suggest that the traditional criteria are not precise and that new technologies are required for dating and functionally identifying the endometrial samples.

In the pre-genomic era, only "gene-by-gene" studies could be carried out to select useful candidates for studying uterine receptivity or for determining the endometrial situation in women with or without endometriosis.

Therefore, in the present genomic era objective tools based on molecular criteria which improve the diagnostic capacity of determined techniques such as the histological technique, which is very useful, however, for other types of needs, are sought.

In the mid 1990s (Schena *et al*., 1995), a revolutionary technology was developed for determining and quantifying the expression of messenger RNA (mRNA) in a sample, gene expression microarrays. Their main advantage is that they offer the possibility of simultaneously analyzing thousands of genes in a single experiment. A DNA microarray consists of a large number of DNA molecules arranged on a solid substrate such that they form an array of sequences in two or three dimensions. These fragments of genetic material can be short sequences called oligonucleotides or larger sequences, such as complementary DNA (cDNA) which is synthesized from mRNA, or PCR products (*in vitro* replication of DNA sequences by means of the polymerase chain reaction). These single-strand nucleotides fragments immobilized on the support are referred to as "probes". The nucleic acids of the samples to be analyzed are labeled using different methods (enzymatic, fluorescent methods, etc.) and are incubated on the probe panel, which allows hybridization (recognition and binding between complementary molecules) of homologous sequences. During hybridization, the labeled genetic material samples bind to their complementary samples immobilized on the support of the chip, allowing the identification and quantification of the DNA present in the sample. The suitable bioinformatic tools and scanner then allow interpreting and analyzing the data obtained (Al-Shahrour F *et al*., 2005).

To use a microarray, commercially available microarrays can be used or one can be custom designed.

To design a microarray, the following operations must be performed:
a) Choosing the type of probe, oligos, cDNA,...
b) Labeling probes or samples: enzymatic, fluorescent,...
c) Support material: glass, plastic, membranes, ...
d) Immobilizing probes: active, passive, covalent,...
e) Manufacturing: printing, *in situ* synthesis, ...
f) Detecting hybridization: scanner, fluorometry,...
g) Data processing: software.

This technology is being applied to the analysis of gene expression, sequencing, therapy follow-up, preventive medicine, drug toxicology and molecular diagnosis. The manufacture of microarrays, also referred to as bioarrays or biochips has been described in various patent documents, such as for example WO 2005/018796 A1, US 2005/0048554 A1, and US 2005/0046758 A1. Their use has also been applied to dendrimers (WO 2005/040094 A1) and large biomolecules (US 2005/0042363 A1) or for collecting information on samples, such as for example identifying a carcinogenic or pathogenic cell in an individual (WO 2005/016230 A2). Their use is also known for immobilizing nucleic acids which are complementary to a variety of genes, being applied to the field of chemistry, biology, medicine and medical diagnostics (US 6,821,724 B1). Microarrays are currently being used to make comparisons based on genomic data and to research different systems.

There are different patent and non-patent literature publications on this subject. Microarray technology has allowed globally studying the gene expression of the endometrium under physiological conditions during the different phases of the menstrual cycle in the natural cycle (Ponnampalam *et al.*, 2004, Talbi *et al.*, 2005). With respect to the human window of implantation, gene expression profiles of the endometrium in the natural cycle have been described (Borthwick *et al.*, 2003; Carson *et al*., 2002; Riesewijk *et al*., 2003; Mirkin *et al.*, 2005). The gene expression profile of the endometrium during the window of implantation in stimulated cycles has also been analyzed (Mirkin *et al.*, 2004; Horcajadas *et al.*, 2005 (Provide literature reference in the Literature section); Simón C *et al.*, 2005) and in response to drugs such as RU486 (Catalano *et al.*, 2003 (Provide literature reference in the Literature section); Sharkey *et al*., 2005).

The refractory profile of the human endometrium in the presence of an intrauterine device (IUD) during the window of implantation has also been studied (Horcajadas *et al.* 2006). All these works have recently been reviewed by the authors of the present application (Horcajadas *et al.*, 2007). The conclusion of said study is that even though different genomic studies of the human endometrium in different physiological and pathological conditions have been conducted in the last 4 years, generating a large amount of information on the gene regulation during the window of implantation both in fertile and infertile women, the key molecules and mechanisms have yet to be discovered.

In the field of patents, there are several which try to determine endometrial receptivity/non-receptivity, though neither the genes, nor the technology, nor the predictive systems they postulate coincide with those used in the present invention.

Patent document US 2003/0077589 A1 describes a method for diagnosing endometriosis based on identifying the product of at least one of the genes of the group consisting of fibronectin, PTK7 transmembrane receptor, type XVIII collagen, alpha 1, protein similar to subtilisin (PACE4), laminin M chain (merosin), elastin, type IV collagen, alpha 2, interferon-alpha-inducible gene p27, reticulocalbin, aldehyde dehydrogenase 6, gravin, nidogen and phospholipase C epsilon, in which a small amount of the control gene indicates the presence of endometriosis.

Patent application US 2003/0125282 A1 describes two human MATER proteins (mice MATER proteins were already known) and their relationship and use for fertility disorders.

Document US 2003/0186300 A1 describes methods and commercial compositions for the diagnosis and treatment of reproduction-associated diseases. The invention also relates to methods and compositions for the determination and modulation of endometrial receptivity.

Patent US 2005/0032111 A1 uses the expression of cadherin-11 in endometrial tissue as an indicator of the capacity for establishing or maintaining a pregnancy.

Document US 2005/0106134 A1 relates to the role of the enzyme proprotein convertase 5/6 during pregnancy, and particularly its detection and the detection of its isoforms in the uterus. This enzyme is useful in fertility control for monitoring a premature pregnancy and for detecting the uterine receptivity in mammals. New forms of proprotein convertase 5/6 are also described.

Patent US 2003/0228636 A1 describes a method for detecting endometrial receptivity for embryo implantation, which comprises: obtaining a sample of the endometrium, contacting the endometrium with a monoclonal antibody for β₃, and detecting β₃ in the endometrium. Contraceptives and diagnostic kits useful for carrying out the methods of the invention are also mentioned.

Patent application WO 2005/061725 A1 describes methods for detecting markers associated with endometrial diseases or a determined endometrial phase in a woman, which comprise measuring the peptide endometrial markers or the polynucleotides encoding the markers in the studied sample. The invention also provides methods for detecting endometrial diseases, as well as kits for carrying out the methods of the invention.

Document WO 01/89548 A2 relates to the pharmaceutical use of the fibulin-1 polypeptide and nucleic acid in birth control in women, and for the diagnosis and treatment of the endometriosis.

In patent WO 2004/058999 A2, the invention relates to a method and the means for determining the specific conditions or changes in the uterine mucosa or in the epithelium of other organs. The method allows determining the overexpression of type 1-β (β7,β6,B6e) mRNA subunits of human gonadotropin. The measurements of the expression of β7,β6,β6e are used to indicate the receptivity of the uterine mucosa to implantation of an embryo or to indicate neoplastic changes in epithelia.

Patent US 2004/0005612 A1 identifies genetic sequences with expression levels which are suppressed or induced in the human endometrium during the window of implantation. The genes characterized during the window of implantation provide material for screening tests for the purpose of determining endometrial alterations and fertility disorders, as well as endometrial-based birth control methods.

Patent US 6,733,962 B2 describes a method for diagnosing abnormal endometrial development of a woman based on the expression of cyclin E and p27 in a sample obtained after day 20 of the menstrual cycle of a woman which ideally lasts 28 days.

In summary, for over 50 years the attempt has been made to determine a histological standard for being used in the clinical diagnosis of endometrial receptivity based on morphological observations. Today, with microarray technology, which is much more precise than morphological observations, works have been published relating to different genes present throughout the menstrual cycle, but the results do not coincide because the experimental design, collecting the samples and selecting the genes are crucial for reaching any conclusions.

Therefore, it is still and more than ever necessary to have a microarray which encompasses selecting genes which generate an expression profile that serves to diagnose and determine if the state of a particular endometrium corresponds to the receptivity/non-receptivity state.

Therefore, a list of genes and probes has been determined in this application which, once incorporated to a microarray, by means of analyzing the joint expression of these genes in the sample under study using a defined and trained computational prediction model, is capable of evaluating the receptivity/non-receptivity state of a sample of the endometrium obtained 7 days after the LH surge, as well as situations of sub-fertility of an endometrial origin depending on the gene expression profile of all of them.

Therefore, the method of the present invention uses the joint expression of the process-related mRNA as a whole as an endometrial receptivity marker, unlike the remaining receptivity molecular markers of the prior which are based on studying a molecule or a small group of molecules considered independently.

### Object of the Invention

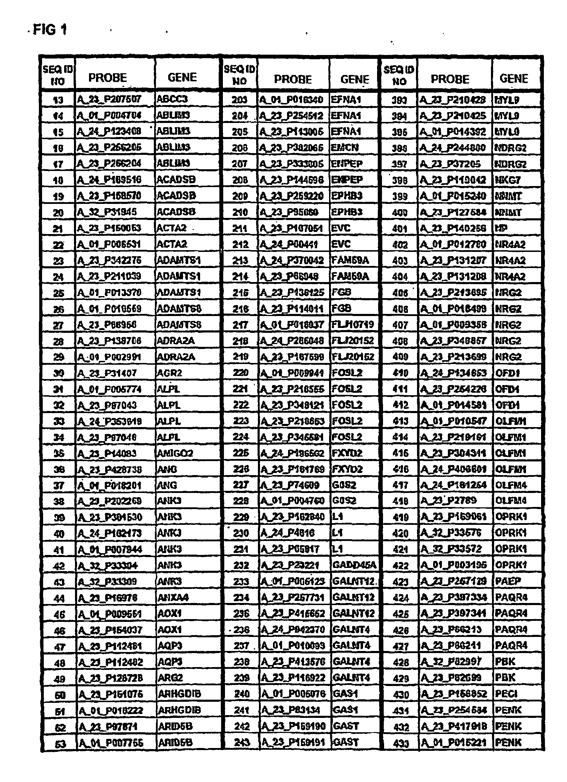

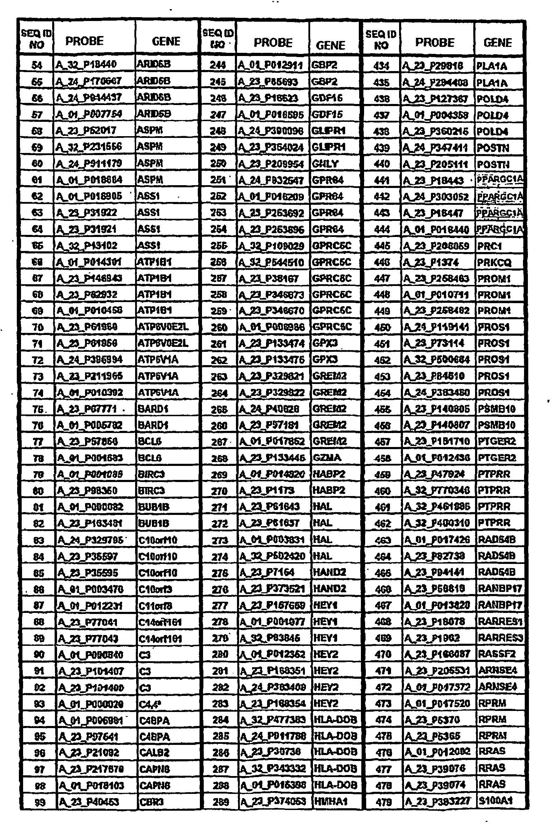

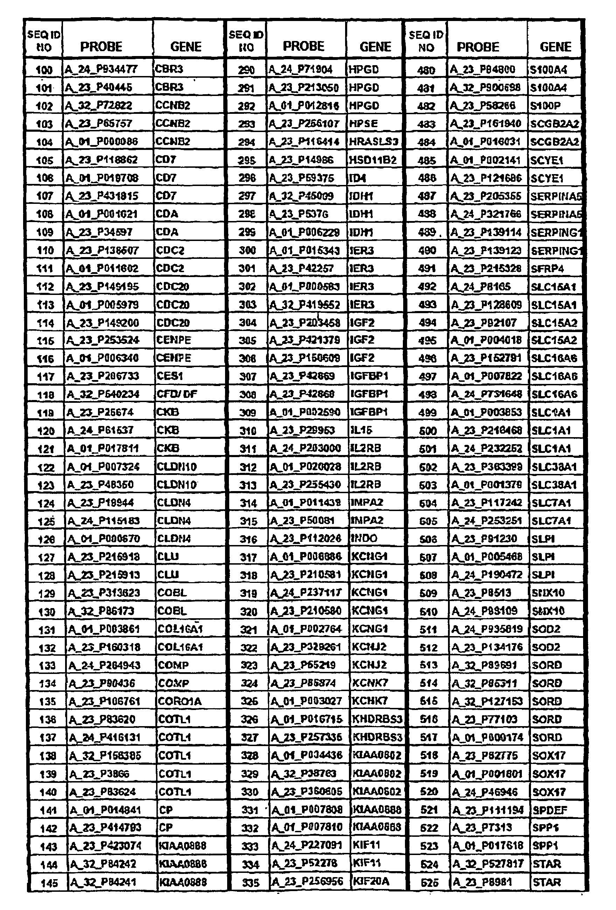

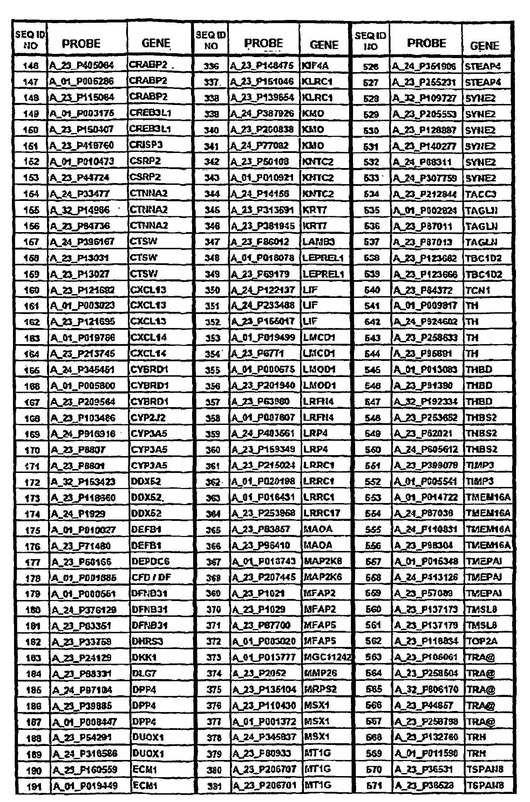

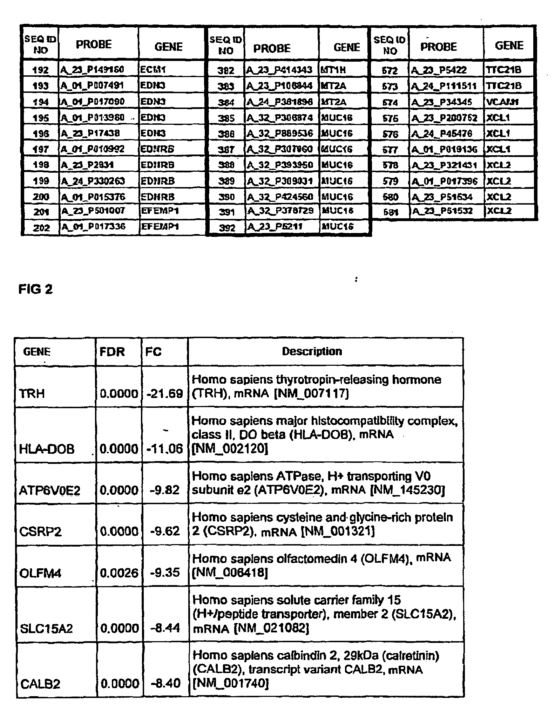

The present invention allows determining the human endometrial receptivity functional state by means of the gene expression profile of the situation of human endometrial receptivity/non-receptivity represented by the set of genes of Fig. 1.

To that end, the steps described below are followed:
1. Identifying a set of genes that are involved in endometrial receptivity for their inclusion in a specific microarray of endometrial receptivity (Endometrial Receptivity Array, ERA).
2. Creating the specific microarray.
3. Analyzing the expression pattern of the ERA during the window of implantation by means of bioinformatic tools, to be able to establish the endometrial receptivity profile and create a prediction model.
4. Developing software which, with this prediction model based on the gene expression profile, allows quantitatively and objectively evaluating and predicting the *in vivo* endometrial receptive state.

The foundation of the microarray is the following: when a gene is active, mRNA molecules which have a base sequence complementary to that of the gene are produced. When a gene is inactive mRNA is not produced. The analysis consists of extracting the total mRNA from two cell populations which vary in the situation to be studied, in this case receptive and non-receptive endometrium, labeling it with a fluorescent substance and hybridizing it on the microarray. Since each mRNA matches up only to the probe of the gene having the same complementary base sequence, those probes which capture the most mRNA -and which therefore shine with more fluorescence - will indicate which genes were the most active. If the fluorescence pattern of the receptive endometrium is compared to that of the non-receptive endometrium, it will be known which genes are differentially expressed in one situation with respect to the other, and that they are therefore process-related.

The probes are designed so that the mRNA of the gene to which they belong bond to them and are fixed in the support of the array. The oligonucleotides forming the probe are inserted in an automated manner in a layer of glass, nylon or plastic, being placed in squares acting like a micro-test tube. The oligonucleotide microarrays are made in an automated manner and inserted by robots by means of photolithography or piezoelectric printing. The result is an automated and normalized process which allows thousands of printings per cm² and minute.

The distribution of the probes in the microarray as a set of probes is generally observed; those having the same sequence are located at the same point in the array. In the ERA of the present invention, the probes are oligos with 60 nucleotides. Therefore, what is labeled and loose in the solution hybridized in the microarray are labeled mRNA fragments, which will bind to the probe fixed to the support as explained, by sequence homology, such that the more labeled mRNA that binds to at one point, which corresponds to the specific probe of a gene, the more light will be detected at that point and it is therefore concluded that said gene is the most active.

Having established the operation of the microarray, and having delimited the receptivity expression pattern for evaluating the receptivity/non-receptivity state of an endometrium by means of bioinformatic methods, the receptivity states of other pathological processes resulting in infertility or subfertility of an endometrial origin, such as implantation failure due to an endometrial cause and hydrosalpinx, can also be evaluated using the same method.

In addition to the use of the microarray for molecular diagnosis, the latter can also be used as a biotechnological tool for studying the possible effect of drugs and/or inert devices in the endometrium, such as for example the response to contraceptive drugs, both in *in vitro* and *in vivo* assays.

More specifically, the microarray is suitable for determining from a biological sample of human endometrium the normalcy/abnormality situation in the receptive profile of said endometrium, because the microarray is a customized expression microarray which analyzes the mRNA set of the biopsy. The receptivity expression profile is defined and classified to that end and using a computational prediction model. It is also capable of defining the normal receptivity state and other situations of receptivity, both subfertility and infertility, as well as the exposure to drugs and/or inert devices, because software is used to analyze the microarray which contains the necessary information so that from an endometrial biopsy obtained during the receptive period and after being analyzed by the ERA, the gene expression data are preprocessed, such that the sample is classified in the class determined by the prediction model.

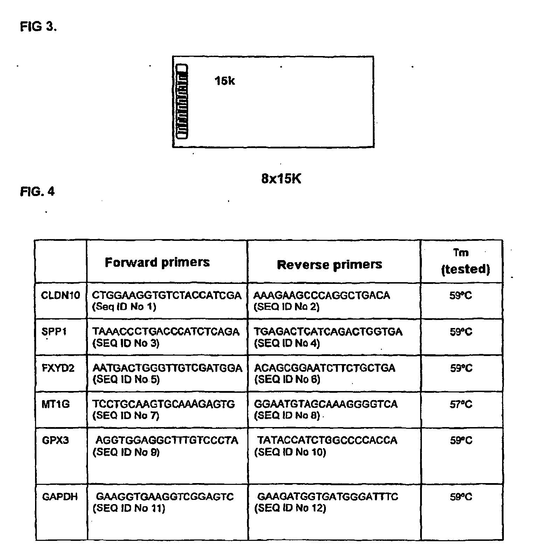

The microarray is an oligo expression microarray with an 8x15K format (8 arrays of 15,000 probes) per slide (Figure 3). Each array contains 15,744 points: 569 probes in which are included the selected genes (8 replicas per probe, 4552 points), 536 control points and 10656 free (empty) points.

### Brief Description of the Figures

Figure 1 shows a list of the 569 probes corresponding to the 238 genes with an FDR < 0.05 and an FC > 3, which are those which have been selected and are specified in Figure 2.
Figure 2 shows a list of the 238 genes selected with an FDR < 0.05 and an FC > 3.
Figure 3 shows a specific microarray (ERA) (Agilent Technologies). The figure shows how the ERA, oligo expression microarray, has a format of 8x15K (8 arrays with 15,000 probes) per slide. Each array contains 15,744 points: 569 probes in which the selected genes are included (8 replicas per probe, 4,552 points), 536 control points and 10656 free (empty) points.
Figure 4 shows a table in which the forward and reverse primers designed from the genes to be amplified by means of quantitative PCR are shown.
Figure 5 shows the mean expression of the probes of each gene in the array compared with the expression in the quantitative PCR.

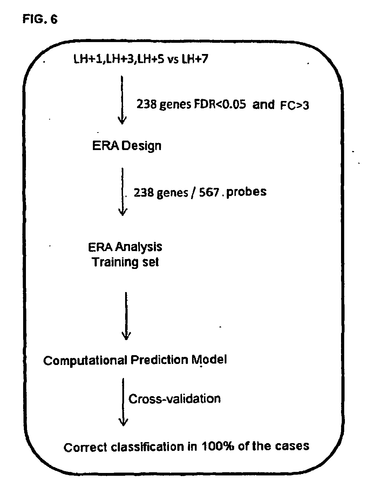

Figure 6 shows a diagram summarizing how the molecular tool and the main components which form it have been designed.

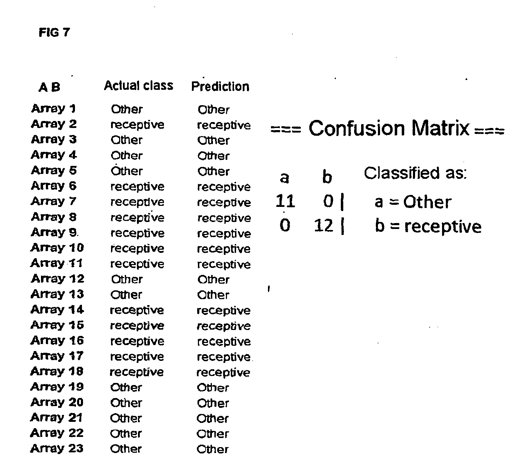

Figure 7 shows the result of a computational prediction model generated with a training set of 23 samples having the described characteristics, which have been analyzed with the ERA. A. The prediction model distinguishes between two classes, Receptive (samples on day 20-21) and Other (samples on days of the cycle outside receptivity). The rows show each of the samples analyzed with the ERA array, and column 1 shows the actual class known *a priori* and column 2 shows the class assigned by the prediction model. It is observed that it predicts with a 100% success rate after calculating the error by cross-validation. B. Confusion matrix in which it is seen that 11 samples are classified as other days of the cycle and 12 samples are classified as receptive, there being no false positives or false negatives.
Figure 8 shows a diagram of the process to be followed for determining the state of endometrial receptivity of a woman.

### Detailed Description of the Invention

Endometrial receptivity is the state in which the endometrium is prepared for embryo implantation. This occurs in all menstrual cycles in a time period referred to as window of implantation, which has a variable duration and opens around day 19 of the cycle and closes on day 24, day 21 being considered a reference day.

Ovulation occurs after the luteinizing hormone (LH) surge, which occurs around day 14. A more exact way to know the actual moment in the menstrual cycle is to measure this LH surge in blood, the day it occurs being considered as day LH 0 and day 15 of the cycle LH+1 and day 21 of the cycle LH+7.

A molecular diagnostic tool allows analyzing the transcriptome of a subset of genes of the genome related to the receptivity status.

After taking an endometrial biopsy on day 21 of the menstrual cycle (receptive phase, LH+7), it can be evaluated whether the woman has a normal receptive endometrium or whether, on contrast, the expected expression pattern is not shown.

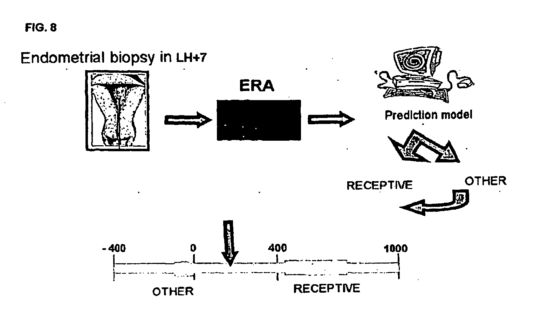

The endometrial biopsy is processed to extract its RNA, and this labeled RNA will hybridize with the probes fixed in the ERA, being able to detect the expression levels of the genes depending on the intensity of each point by means of a scanner. The data of the intensities of each point are analyzed by the prediction model which has previously been trained, and this model, depending on the entire set of points, classifies the samples as normal receptive samples or outside of normalcy samples (Figure 8).

The prediction model is a mathematical system using different algorithms, formulas, to distinguish between classes, and is trained with the training set to define the normal receptivity profile, and to define the receptivity profile of endometrial pathologies or status of subfertility due to endometrial causes which cause implantation failure; such as endometriosis, hydrosalpinx, etc. 1. Identifying the genes involved in endometrial receptivity for generating the specific microarray of endometrial receptivity.

The first phase of the project consists of identifying the genes which are specifically regulated in the endometrium of day LH+7 and which will be part of the customized microarray.

In most published works, the mentioned genes have been selected when they are induced or suppressed two times. Different and stricter selection criteria have been followed in the present invention:

### Gene selection criterion.

The genes have been selected based on the differences of the endometrial gene expression profile represented by LH+1, LH+3 and LH+5 (non-receptive) against LH+7 as the receptive state. The expression levels have been obtained from a whole genome oligo expression microarray. Those genes showing significant differences of expression in these two situations have been chosen using the criteria of FDR < 0.05* and FC > 3**.
* FDR: False Discovery Rate. This parameter corrects the P-value depending on the size of the sample. The value of FDR 0.05 is the significance that is typically taken into account at the statistical level and involves running a 5% risk that the differences are due to chance and not to the biological process in question.
** FC: Fold change. This means the number of times that the expression of a gene changes in one situation with respect to another. With regard to FC > 3, the criterion is to assume that if it changes more than three times, it is sufficient change to consider the gene important for the process.

The possibility that the differences of expression may be due to chance and not to the biological process has been considered with FDR. Furthermore, the genes with an Fc above a threshold value of 3 have been selected so that the final number of genes worked with is feasible. More importance is therefore given to the genes which change the most because a directly proportional ratio between more changes and greater importance for the process is assumed. This strict criterion combines both the statistical and the biological requirement. Furthermore, the functional sense of this gene selection has been corroborated in the biological process of endometrial receptivity. To that end, the genes were ontologically classified by means of bioinformatic tools using FATIGO GEPAS (Al-Shahrour F *et al*., 2005) given that the biological processes represented in a manner exceeding what is expected with a significance of 0.05 are the response to stress, the defense response and cell adhesion, which are fairly relevant processes in preparing an endometrium for the possible implantation of the blastocyst.

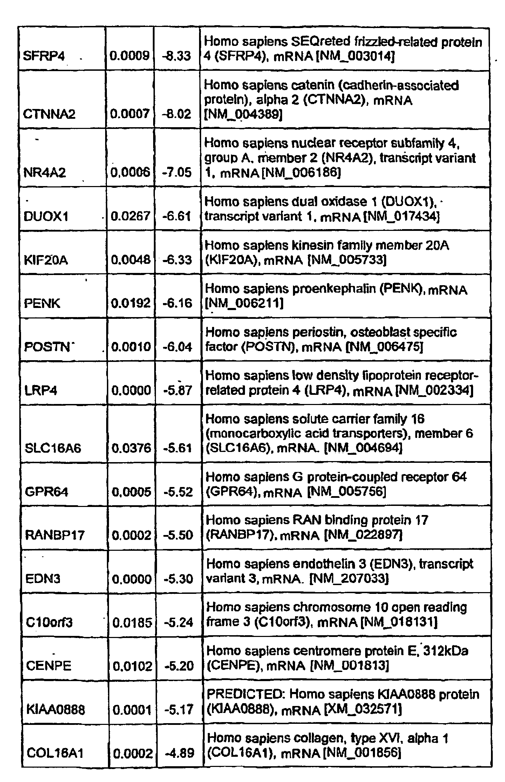

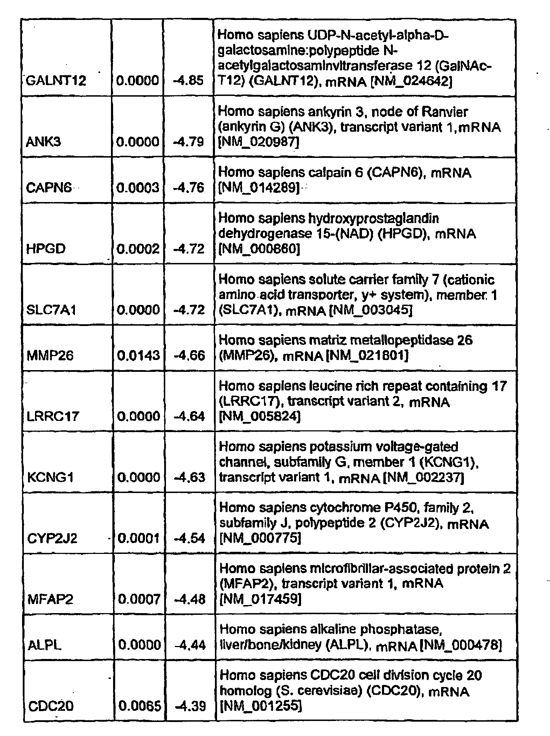

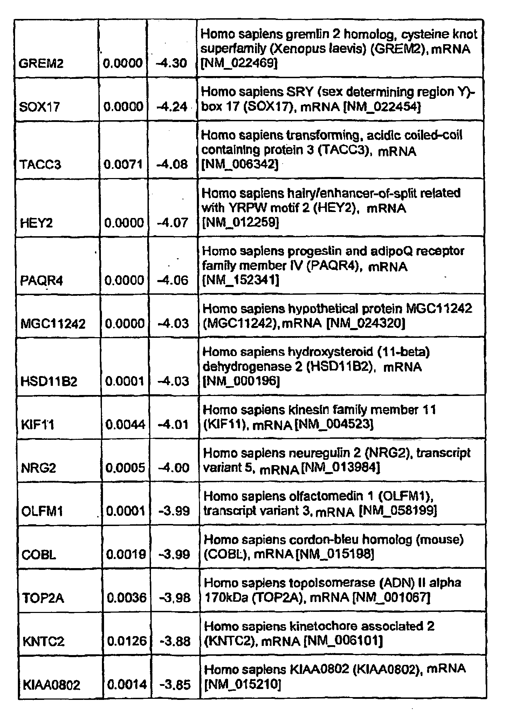

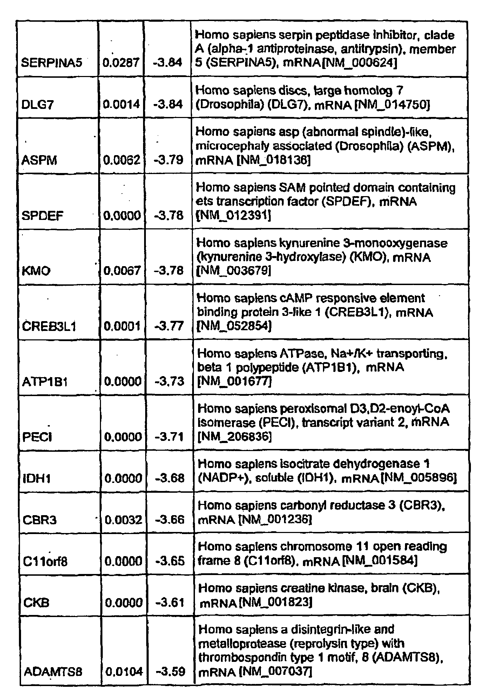

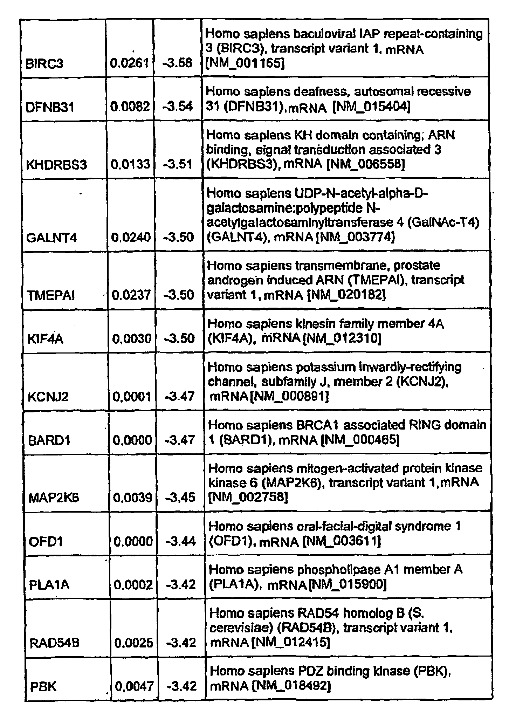

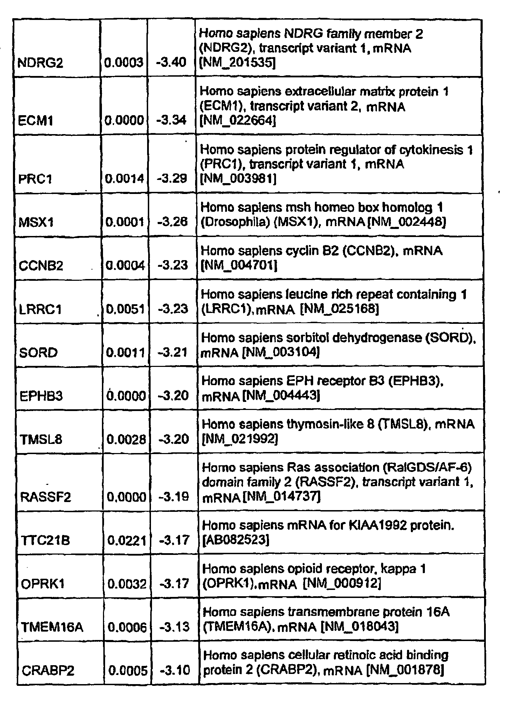

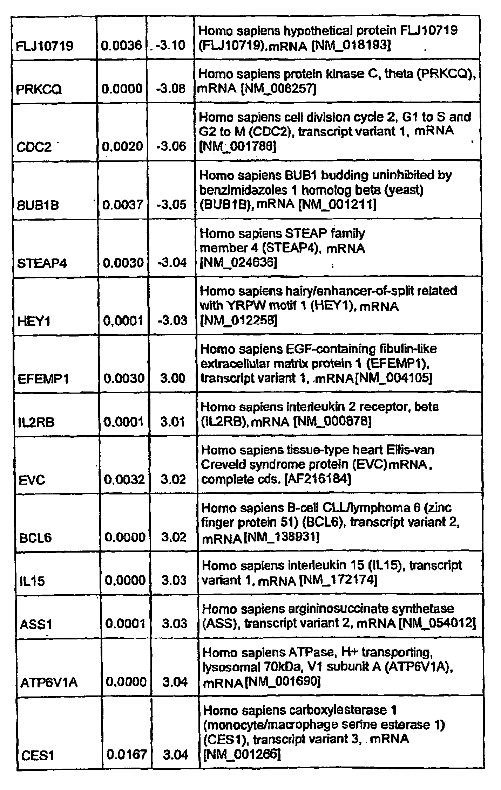

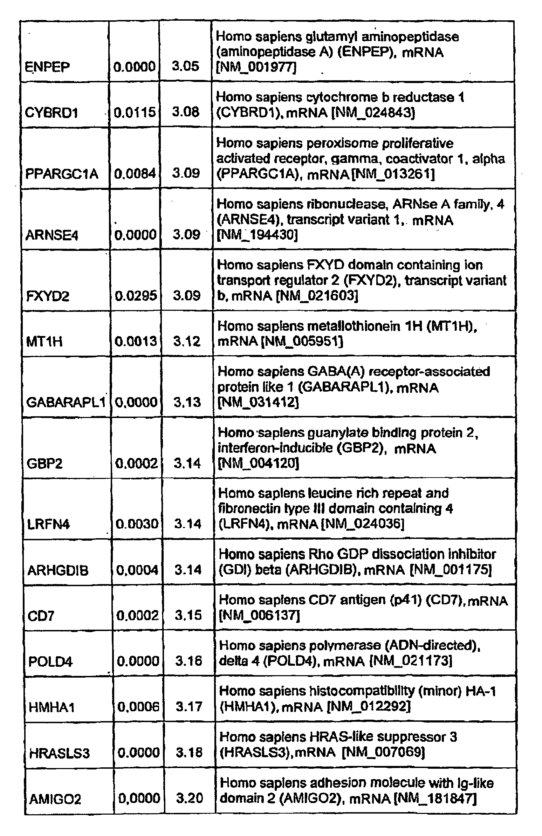

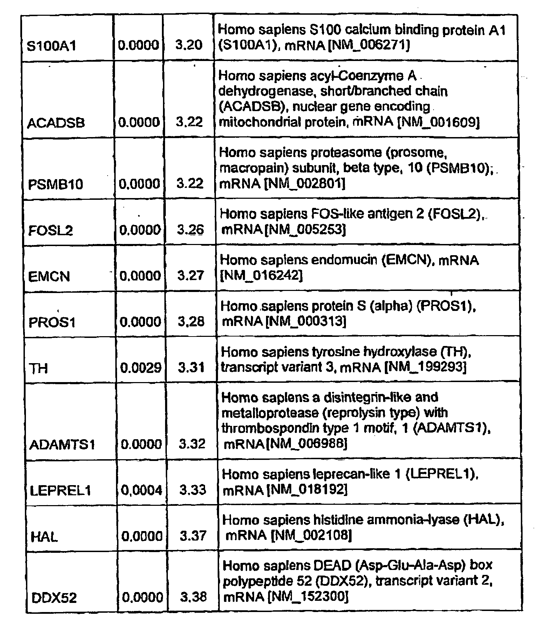

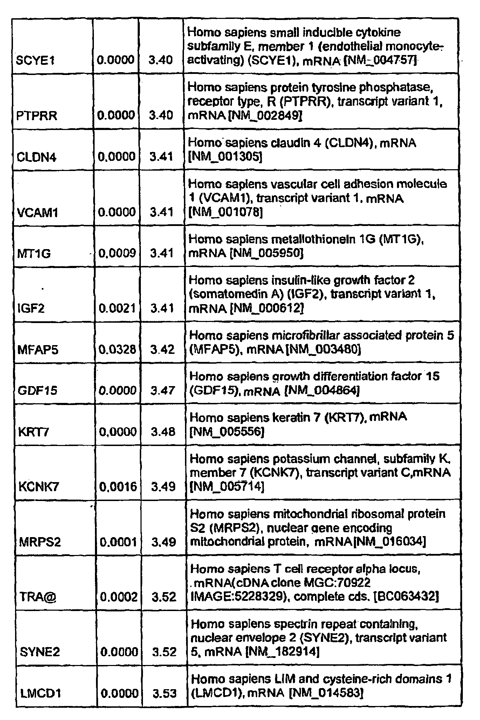

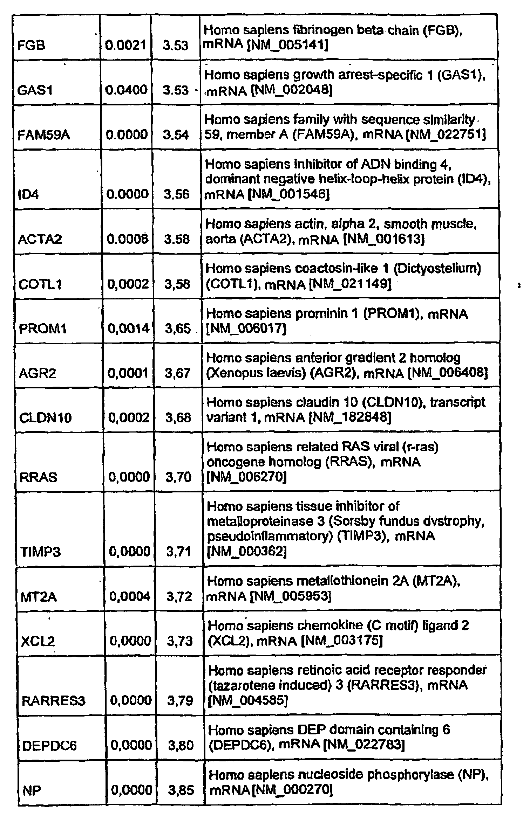

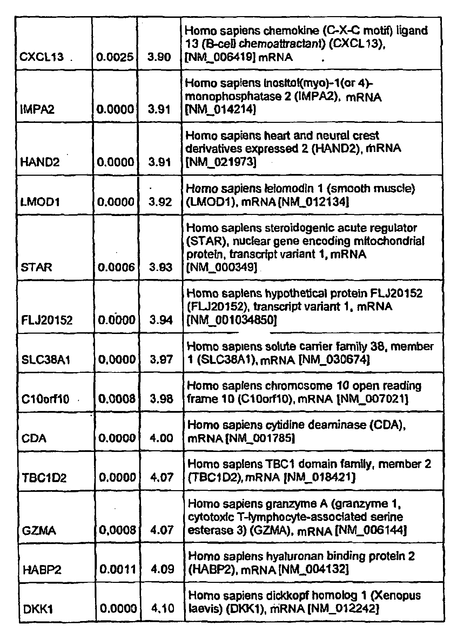

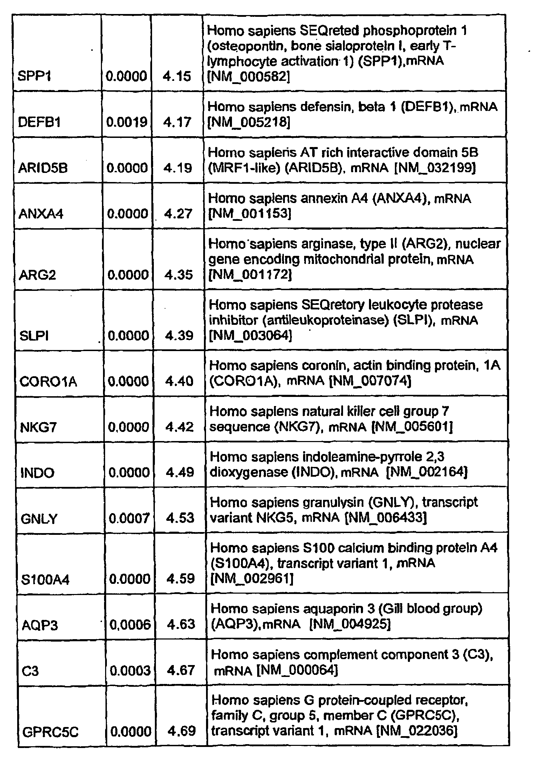

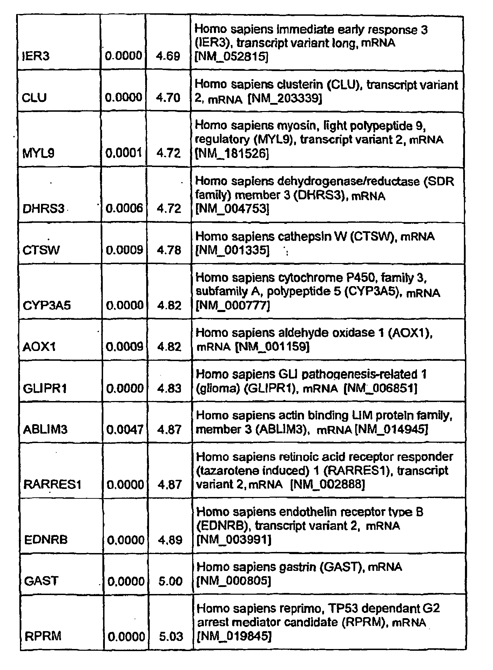

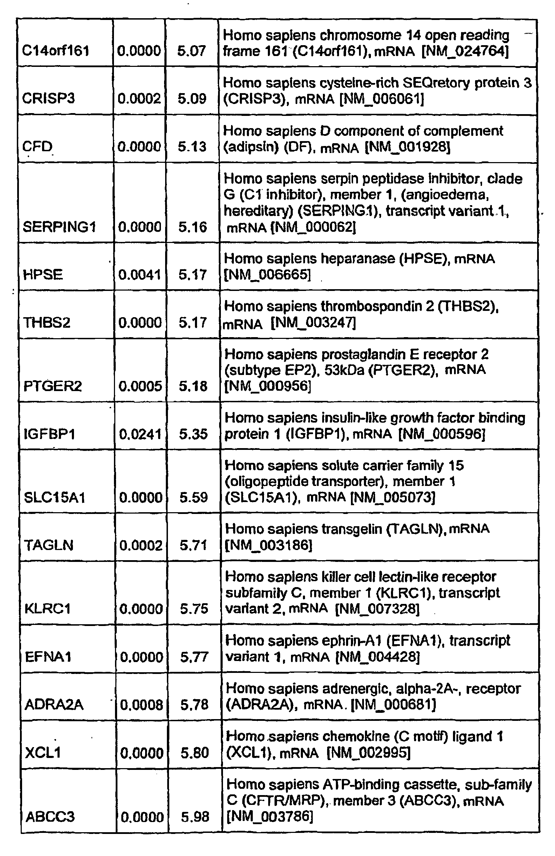

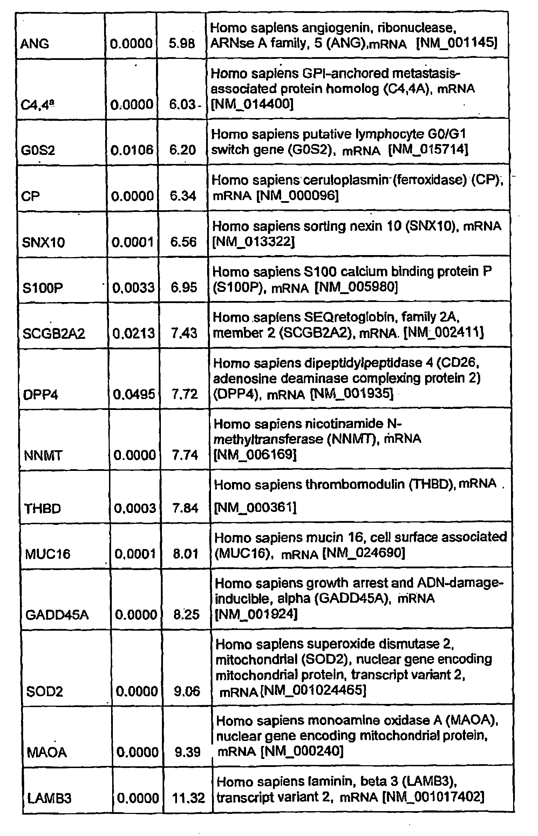

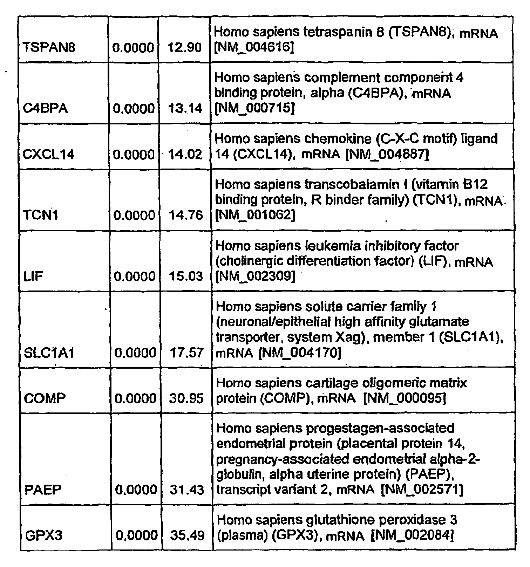

Those genes with these characteristics have been chosen and this has resulted by means of computer programs in a total of 238 genes (Figure 2) represented by 569 probes (Figure 1). 2. Creating the specific microarray (Era) (Agilent Technologies)

The ERA is an oligo expression microarray with a format of 8x15K (8 arrays of 15,000 probes) per slide (Figure 3). Each array contains 15,744 points: 569 probes in which the selected genes are included (8 replicas per probe, 4,552 points), 536 control points and 10,656 free (empty) points. Expression analysis by means of the ERA

In this section, the expression data generated by the ERA for classifying the endometrial samples in two or more classes according to the different receptivity profiles that are generated (normal receptive; pathological receptive; normal non-receptive...) are used to generate the prediction model and to check its efficacy.

To that end, endometrial biopsies of fertile women are selected. All the independent samples are from women with proven fertility on different days of the menstrual cycle. They are Caucasian women with a body mass index between 19 and 25 kg/m² and whose ages range between 18 and 35 years old.

Said samples were used to generate a prediction model.

To that end, the total RNA was extracted using the Trizol protocol (Invitrogen) following the manufacturer's instructions (Life Technologies, Inc., USA). The samples were homogenized using 1 ml of trizol for each 75 mg of tissue, they were incubated at room temperature for 5 minutes, and 200 µl of chloroform were added for the same amount of tissue and were incubated at room temperature for 5 minutes. They were then centrifuged for 15 minutes at 12,000xg (4°C). The aqueous phase was precipitated with an equal volume of 2-propanol (isopropanol), it was incubated on ice for 5 minutes and centrifuged for 30 minutes at 12,000xg (4°C). The precipitate was washed with 70% ethanol in water treated with diethylpyrocarbonate (DEPC) to subsequently resuspend it in water-treated DEPC (15 µl). This protocol usually produces 1-2 µg of total RNA per mg of endometrial tissue. The RNA thus extracted is treated with DNase for 1 hour at 37°C to remove the traces of DNA and purify it again using the Qiagen RNeasy kit following the manufacturer's instructions. The RNA that is obtained after the columns of the RNeasy kit is analyzed to check its quality in the Agilent 2100 bioanalyzer using the Agilent brand RNA specific chips, RNA Nano LabChip.

Only those RNAs having the following characteristics have been used for subsequent analyses:
- They did not have detectable genomic DNA,
- They had a concentration greater than 200 µg/ml,
- The value of the radius of rRNA was 28s/18S > 1.2, and
- The RIN (RNA Integrity Number) value > 7.0.

After the analyses with the samples selected due to their suitable quality, single-stranded complementary DNA (cDNA) is generated from the total RNA by incubating it between one and two hours at 40°C with reverse transcriptase, nucleotides and an oligonucleotide polydT-T7, which has not only the poly T sequence which hybridizes with the polyA tail of messenger RNA, but also the recognition sequence for T7 RNA polymerise.

The cDNA obtained in the previous step is incubated for 2 hours at 40°C in the presence of T7 RNA polymerase and nucleotides, one of which is labeled with Cy3, to produce complementary RNA called cRNA.

That cRNA is purified by means of a purification kit based on affinity chromatography and is quantified.

Once purified, that labeled cRNA is fragmented for 30 minutes at 60°C and hybridized in the microarray for 17 hours at 65°C. Once that time has elapsed, the microarray is washed to remove unspecific hybridizations. Once hybridized and washed, the microarrays are centrifuged at 3,000 rpm for 3 minutes to dry the microarrays and they are then read by means of scanning them in an Axon GenePix 4100A, reading for Cy3 intensities (532 nm).

As a result, after the relevant data processing enclosed below, a gene expression matrix was generated the rows of which correspond to the 569 probes of the 238 genes selected and the columns of which correspond to the different samples.

### Processing the data of the array

The correction of the bottom effect has been done by subtracting half the median of the latter from the intensity of the point. Interarray normalization has been done using the quantile method.

The mean of the eight replicas of each probe is then calculated. The different probes of the same gene (probe set) are analyzed individually and the results are processed by bioinformatic tools.

### Validating the results of the ERA by means of PCR

The results obtained in the ERA have been validated by means of quantitative PCR for the purpose of giving the results greater consistency and checking that the microarray analysis is reliable.

Reverse transcription is performed to obtain RNA in the form of cDNA, to that end 1 µg of total RNA was placed in the presence of 1 µg oligo (dT) (Clontech) until reaching a final volume of 12.5 µl with water treated with DEPC (diethylpyrocarbonate). It was heated for 2 minutes at 70°C so that any possible secondary structure in the mRNA would denature, and it was then kept on ice for 2 minutes.

Then 6.5 µl of a MIX solution with 4 µl of buffer, 1 µl dNTP, 0.5 µl RNase and 1 µl of reverse transcriptase (Rt-PCR Clontech) were added for each of the 30 samples to be validated. The reverse transcription lasted for 1 hour in the thermal cycler. 80 µl of water with DEPC are added and concentration of single-stranded cDNA obtained is measured by spectrophotometry placing 2 µl of sample and 98 µl of DEPC-treated water. The amount of cDNA that has been reverse transcribed must be between 80 to 120 ng/µl to start from similar concentrations, though it is normalized with the internal pattern, in our case GAPDH. In any case, in order for the quantitative PCR to work correctly, the range of cDNA to be amplified must be between 50-500 ng/µl. If any sample is not within those parameters, it is diluted.

The forward and reverse primers were designed for five genes with increased LH+7 (Figure 4). The oligonucleotide sequences of the primers were designed with the GeneFisher bioinformatic program (see Figure 4 and sequence listing). The detection system was performed with SYBR Green I binding to double-stranded DNA (Roche). This detection system establishes a linear dynamic range for detecting specific PCR products. All the Q-PCR experiments were conducted using the SYBR Green PCR Master Mix (Roche) and the universal conditions of the thermal cycle parameters indicated by the manufacturers using the Roche Light Cycler. 40 cycles were performed. The temperatures at which the primers work well can be observed in Figure 4. The relative quantification was performed by means of the standard master curve method.

The expression of GPX3; CLDN10; FXYD2; SPP1; and MT1G, correspond in the ERA to the expression values of the following probes:

| Probe | Gene |
|---|---|
| A_23_P133474 | GPX3 |
| A_23_P133475 | GPX3 |
| A_01_P007324 | CLDN10 |
| A_23_P48350 | CLDN10 |
| A_24_P196562 | FXYD2 |
| A_23_P161769 | FXYD2 |
| A_23_P7313 | SPP1 |
| A_01_P017618 | SPP1 |
| A_23_P60933 | MT1G |
| A_23_P206707 | MT1G |
| A_23_P206701 | MT1G |

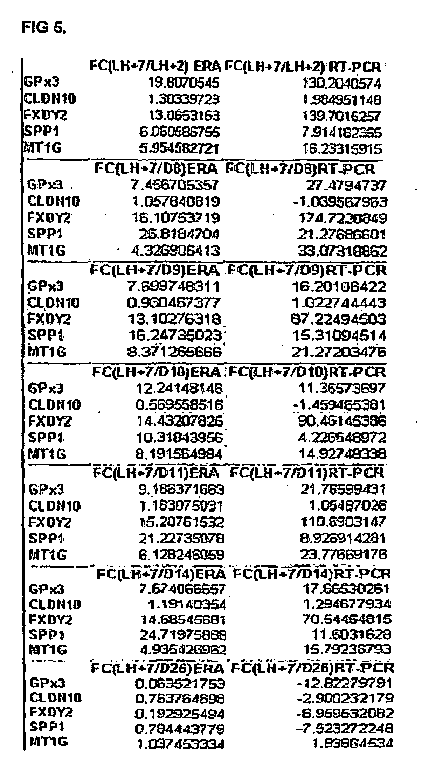

Considering that these are different techniques, quantitative PCR, the sensitivity of which is much higher but it only provides one expression value, and the arrays in the which there is expression of different probes for one and the same gene, in order to make the comparison, the mean expression of the different probes of a gene has been calculated in the array (Figure 5).

Due to the different sensitivity, it is considered that the ratio of the expression value between both techniques would correspond to a correction factor of 10 (augmented expression 10x in the array) it is accepted that they correspond with a maximum of 100x in the quantitative PCR (Figure 5).
3. Analyzing the expression pattern of the ERA during the window of implantation to be able to establish the endometrial receptivity profile. Generating a classifier.

### Training

A predictor is a mathematical tool which uses a data matrix, in this case of the data generated with the ERA, and learns to distinguish classes (Medina I, *et al*., 2007), in this case two or more classes according to the different receptivity profiles that are generated (normal receptive; pathological receptive; normal non-receptive...). The underlying reasoning for this strategy is the following: if it is possible to distinguish among the classes as a consequence of the level of gene expression, it is then in theory possible to find the characteristic gene expression of LH+7 and to use it to assign a class to the expression profile of the test sample analyzed with the customized ERA microarray.

The set of samples which trains the classifier to define the classes is referred to as training set. In other words, the gene expression profiles of these samples, measured with the ERA, are used by the program to know which probes are the most informative and to distinguish between classes (different normal non-receptive and receptivity states). The biopsies used to generate the classification model are carefully chosen and dated in the most reliable manner currently available. This training set will gradually grow as a larger number of samples are tested, but it is made up of receptive samples and on other days of the menstrual cycle. They are all independent samples from different healthy women in the natural cycle and with proven fertility. They are Caucasian women with a body mass index between 19 and 25 kg/m² and between 19 and 34 years old. Only those samples the histological dating of which, by applying Noyes criteria, coincides between the two pathologists and with the day of the menstrual cycle have been chosen.

The classification is done by the bioinformatic program using different mathematical algorithms, there being many available. An algorithm is a well defined, ordered and finite list of operations which allows solving a problem. A final state is reached through successive and well-defined steps given an initial state and an input, obtaining a solution.

The classifier calculates the error committed by means of a process called cross-validation, which consists of leaving a subset of the samples of the training set of a known actual class out of the group for defining the classes, and then testing them with the generated model and seeing if it is right. This is done by making all the possible combinations. The efficacy of the classifier is calculated and prediction models are obtained which correctly classify all the samples of the training set (Figure 5). In other words, all the samples of the training set are classified by the predictor in the assigned actual class known by the inventors.

*A priori*, it is impossible to know how the data are distributed in space, it is only possible to know how they are located in the dimensions that can be distinguished, there being three of them. Therefore, there are different algorithms to be applied which would work better or worse depending on how the entered data are distributed in space. The algorithms most widely used in mathematics for expression matrices generated by microarray analysis are applied, and the one that best separates the defined classes is observed. Therefore, there are algorithms which establish a separation according to a straight line, others do so depending on the closest nearby point, based on distances... and thus each method is based on a mathematical separation criterion which will more or less fit the reality of the samples. 4. Developing a predictor which allows quantitatively and objectively evaluating and predicting the endometrial receptive state based on the gene expression profile.

### Determining the prediction

Depending on all the parameters relating to a computational predictor explained above, a prediction model is generated which classifies all the samples according to the assigned actual class, which in turn was dated by Noyes, there being a 100% coincidence (Figure 7).

The generated prediction model has been trained with a training set of 23 samples, 12 receptive samples and 11 on other days of the menstrual cycle, two classes (receptive/Other) being distinguished. After that, the model will be re-trained as more samples of the same characteristics of the already generated training set are obtained, but also with samples in a receptivity period with pathologies altering the expression pattern of the ERA, as well as the alteration by drugs. Increasingly more classes will thus be gradually defined.

Therefore, the ERA can be used for the positive identification of the endometrial receptivity, as well as for the diagnosis of the alteration thereof associated with endometrial alterations typical of pathologies such as endometriosis, implantation failure, hydrosalpinx, etc. This diagnostic tool would also allow detecting functional modifications induced by interceptive drugs or drugs which intend to improve endometrial receptivity, altering the normalcy/abnormality situation in the receptive profile of the endometrium of a woman.

Therefore, the ERA of the present invention is a customized gene expression microarray. It is a 60-mer oligo array with 8 arrays per slide, with 15K (15744 points) in each array.

It is a customized array with design number 016088 (AMADID). It has 569 probes represented by 238 genes with 8 replicas for each probe, for a total of 4,536 points, 10,672 of which are free points.

Reading the expression profile of the expression data for 238 genes represented by 569 probes (genes with an FDR > 0.05 and an FC > 3) is a prediction model constructed with 23 samples classified with an error of 0, which is capable of classifying the sample as receptive state or other.

The statistical analyses as well as the selection of genes with the indicated characteristics were done using computer programs.

The final list of the ERA includes the 569 probes representing the 238 genes with an FDR < 0.05 and an FC > 3 (Figure 1).

The customized ERA array is hybridized with the messenger RNA of another set of samples different from those used to select the genes to be included, which are used to teach the predictor how to classify between LH+7 or another.

After defining these two classes, receptive or outside, the predictor will be scaled, i.e., it will determine how close or far the profile of a sample is from the receptive profile.

### EXAMPLE

### Obtaining and processing the samples

Biopsies of the endometrium were taken in 30 healthy female donors with proven fertility, and from 10 patients in a clinic with implantation failure due to an endometrial cause, the 4^{th} biopsies being taken on day 21 of the menstrual cycle (receptive phase, LH+7).

The total RNA of each of the biopsies is extracted using the Trizol protocol (Invitrogen) following the manufacturer's instructions (Life Technologies, Inc., USA). The samples are homogenized using 1 ml of Trizol for each 75 mg of tissue, they are incubated at room temperature for 5 minutes, and 200 µl of chloroform are added for the same amount of tissue and are incubated at room temperature for 5 minutes. They are then centrifuged for 15 minutes at 12,000xg (4°C). The aqueous phase is precipitated with an equal volume of 2-propanol (isopropanol), it is incubated on ice for 5 minutes and centrifuged for 30 minutes at 12,000xg (4°C). The precipitate is washed with 70% ethanol in water treated with diethylpyrocarbonate (DEPC) to subsequently resuspend it in DEPC-treated water (15 µl). This protocol usually produces 1-2 µg of total RNA per mg of endometrial tissue. The RNA thus extracted is treated with DNase for 1 hour at 37°C to remove the traces of DNA and purify it again using the Qiagen RNeasy kit following the manufacturer's instructions. The RNA that is obtained after the columns of the RNeasy kit is analyzed to check its quality in the Agilent 2100 bioanalyzer using the Agilent brand RNA specific chips, RNA Nano LabChip.

Only those RNAs having the following characteristics can be used:
- they did not have detectable genomic DNA,
- they had a concentration greater than 200 µg/ml,
- the value of the radius of rRNA was 28s/18S > 1.2, and
- the RIN (RNA Integrity Number) value > 7.0.

After the analyses with the samples selected due to their suitable quality, single-stranded complementary DNA (cDNA) is generated from the total RNA by incubating it between one and two hours at 40°C with reverse transcriptase, nucleotides and an oligonucleotide polydT-T7, which has not only the poly T sequence which hybridizes with the polyA tail of messenger (RNA, but also the recognition sequence for T7 RNA polymerase.

The cDNA obtained in the previous step is incubated for 2 hours at 40°C in the presence of T7 RNA polymerase and nucleotides, one of which is labeled with Cy3, to produce complementary RNA called cRNA.

That cRNA is purified by means of a purification kit based on affinity chromatography and is quantified.

Once purified, that labeled cRNA is fragmented for 30 minutes at 60°C and hybridized in the microarray for 17 hours at 65°C. Once that time has elapsed, the microarray is washed to remove unspecific hybridizations. Once hybridized and washed, the microarrays are centrifuged at 3,000 rpm for 3 minutes to dry the microarrays and they are then read by means of scanning them in an Axon GenePix 4100A, reading for Cy3 intensities (532 nm).

As a result, after the relevant data processing enclosed below, a gene expression matrix is generated the rows of which correspond to the 569 probes of the 238 genes selected and the columns of which correspond to the different samples.

### Processed of the data of the array

The data of the array is processed by a series of bioinformatic commands which are in software designed exclusively for the invention as is explained below.

The correction of the bottom effect in the 40 data matrices due to the labeling process typical of the technique is performed.

The empty points are then removed and the normalization process is performed depending on the 40 samples and depending on the expression profile defined according to the prediction model so that it can be compared.

The mean of the eight replicas of each probe is then calculated. The different probes of the same gene are analyzed individually and the results are analyzed by the computational created prediction model which is also included in the software.

### Prediction

The 40 samples to be tested (test set) are run with the created classification model which analyzes the expression of the ERA and predicts which class they belong to.

### Results

The analysis of the expression data of the array was entered in the software. The obtained result indicated that out of the 30 tested samples from healthy women with proven fertility, 27 corresponded to women with an receptivity expression profile of the endometrium considered as normal and 3 corresponding to women with an receptivity expression profile of the endometrium considered as outside of normalcy. Nine out of the 10 patients with implantation failure were classified as outside of normal receptivity and 1 was classified as within normal receptivity. The molecular tool presented a 90% diagnostic efficacy.

### LITERATURE

- Al Shahrour F and Dopazo J. In Azuaje F and Dopazo J (eds), Data analysis and visualization in genomics and proteomics. Wiley 2005; 99-112.
- Al-Shahrour F, Minguez P, Vaquerizas JM, Conde L and Dopazo J. BABELOMICS: a suite of web tools for functional annotation and analysis of groups of genes in high-throughput experiments. Nucleic Acids Res 2005; 33:460-464,
- Balasch J, Fabregues F, Creus M and Vanrell JA. The usefulness of endometrial biopsy for luteal phase evaluation in infertility. Hum Reprod 1992; 7:973-977.
- Batista MC, Cartledge TP, Merino MJ, Axiotis C, Platia MP, Merriam GR. Midluteal phase endometrial biopsy does not accurately predict luteal function. Fertil Steril 1993; 59:294-300,
- Borthwick J, Charnock-Jones S, Tom BD et al, (2003) Determination of the transcript profile of human endometrium. Mol Hum Reprod 9, 19-33,
- Carson D, Lagow E, Thathiah A et al, (2002) Changes in gene expression during the early to mid-luteal (receptive phase) transition in human endometrium detected by high-density microarray screening. Mol Hum Reprod 8, 971-979.
- Catalano RD, Yanaihara A, Evans AL, Rocha D, Prentice A, Saidi S, Print CG, Charnock-Jones DS, Sharkey AM and Smith SK (2003) The effect of RU486 on the gene expression profile in an endometrial explant model Mol Human Reprod 9,465-473.
- Coutifaris C, Myers ER, Guzick DS, Diamond MP, Carson SA, Legro RS, McGovern PG, Schlaff WD, Carr BR, Steinkampf MP, Silva S, Vogel DL and Leppert PC. Histological dating of timed endometrial biopsy tissue is not related to fertility status. Fertil Steril 2004; 82:1264-72.
- Creus M, Ordi J, Fabregues F, Casamitjana R, Ferrer B, Coll E, Vanrell JA and Balasch J. Alphavbeta 3 integrin expression and pinopod formation in normal and out-of-phase endometria of fertile and infertile women. Hum Reprod 2002; 17:2279-2286,
- Horcajadas JA, Sharkey AM, Catalano RD, Sherwin JRA, Dominguez F, Burgos LA, Castro A, Peraza MR, Pellicer A and Simón C (2006) Use of Gene-Expression Profiling to Identify Human Endometrial Refractoriness. J Clin Endocrinol Metabol.
- Horcajadas JA, Pellicer A and Simón C (2007) Wide Genomic Analysis of Human Endometrial Receptivity. New times, new opportunities. Human Reprod Update 13, 77-86,
- Horcajadas JA, Riesewijk A, Polman J, van Os R, Pellicer A, Mosselman S and Simón, C (2005) Effect of Controlled Ovarian Hyperstimulation in IVF on Endometrial Gene Expression Profiles. Mol Human Reprod 11,195-205.
- Kliman HJ, Honig S, Walls D, Luna M, McSweet JC, Copperman AB. Optimization of endometrial preparation results in a normal endometrial function test (EFT) and good reproductive outcome in donor ovum recipients. J Assist Reprod Genet 2006; 23:299-303.
- Lessey BA, Castelbaum AJ, Sawin SW, Sun J. Integrins as markers of uterine receptivity in women with primary unexplained infertility. Fertil Steril 1995; 63:535-542.
- Li TC, Dockery P, Rogers AW and Cooke ID. (How precise is histologic dating of endometrium using the standard dating criteria?. Fertil Steril 1989; 51:759-763,
- Medina I, Montaner D, Tárraga J, Dopazo J. Prophet, a web-based tool for class prediction using microarray data. Bioinformatics. 2007; 23(3):390-1.
- Mirkin S, Arslan M, Churikov D, Corica A, Diaz Jl, Williams S, Bocca S and Oehninger S (2005) In search of candidate genes critically expressed in the human endometrium during the window of implantation Human Reprod 20:2104-2117.
- Mirkin S, Nikas G, Hsiu JG, Diaz J and Oehninger S (2004) Gene expression profiles and structural/functional features of the peri-implantation endometrium in natural and gonadotropin-stimulated cycles. J Clin Endocrinol Metab 89:5742-5752.
- Montaner D, Tárraga J, Huerta-Cepas J, Burguet J, Vaquerizas JM, Conde L, Minguez P, Vera J, Mukherjee S, Valls J, Pujana MAG, Alloza E, Herrero J, Al-Shahrour F and Dopazo J. Next station in microarray data analysis: GEPAS. Accepted Nucleic Acids Res. 2006,
- Murray MJ, Meyer WR, Zaino RJ, Lessey BA, Novotny DB, Ireland K, Zeng D and Fritz MA. A critical analysis of the accuracy, reproducibility, and clinical utility of histologic endometrial dating in fertile women. Fertil Steril 2004; 81:1333-1343,
- Noyes RW, Hertig AT, and Rock J. Dating the endometrial biopsy. Fertil Steril 1950; 1:3-17.
- Ordi J, Creus M, Quinto L, Casamitjana R, Cardesa A and Balasch J. Within-subject between-cycle variability of histological dating, alpha v beta 3 integrin expression, and pinopod formation in the human endometrium. J Clin Endocrinol Metab 2003; 88:2119-2125,
- Papanikolaou EG, TouARNye H, Verpoest W, Camus M, VeARNeve V, Van Steirteghem A, Devroey P; http://www.ncbi.nlm.nih.gov/sites/entrez?Db=pubmed&Cmd=ShowDet ailView&TermToSearch=15576388&ordinalpos=79&itool=EntrezSystem 2.PEntrez.Pubmed.Pubmed_ResultsPanel.Pubmed_RVDocSum Early and late ovarian hyperstimulation syndrome: early pregnancy outcome and profile. Hum Reprod. 2005; 20(3):'636-641.
- Ponnampalam AP, Weston GC, Trajstman AC. Molecular classification of human endometrial cycle stages by transcriptional profiling. Mol Hum Reprod 2004; 10, 879-893,
- Riesewijk A, Martin J, Horcajadas JA Polman J, Pellicer A, Mosselman S and Simón C (2003) Gene expression profiling of human endometrial receptivity on days LH+2 contra LH+7 by microarray technology. Mol Hum Reprod 9:253-264,
- Schena M, Shalon D, Davis RW and Brown PO. Quantitative monitoring of gene expression patterns with a complementary DNA microarray. Science 1995; 270:467-470,
- Sharkey AM, Catalano R, Evans A, Charnock-Jones DS and Smith SK (2005) Novel antiangiogenic agents for use in contraception. Contraception 71,263-271.
- Shoupe D, Mishell DR Jr, Lacarra M, Lobo RA, Horenstein J, d'Ablaing G. Correlation of endometrial maturation with four methods of estimating day of ovulation. Obstet Gynecol. Obstet Gynecol 1989; 73:88-92.
- Talbi S, Hamilton AE, Vo KC, Tulac S, Overgaard M T, Dosiou C, Le Shay N, Nezhat, CN, Kempson R, Lessey BA, Nayak NR and Giudice LC. Molecular phenotyping of human endometrium distinguishes menstrual cycle phases and underlying biological processes in normo-ovulatory women. Endocrinology 2005; 147:1097-1121.
- Wilcox AJ, Baird DD, Weinberg CR. Time of implantation of the conceptus and loss of pregnancy. N Engl J Med. 1999;340:1796-1799.
   T-REX (http://www.gepas.org/)
   FATIGO(http://babelomics.bioinfo.cipf.es/EntryPoint?load Form=fatigo)
   PROPHET(http://gepas.bioinfo.cipf.es/cgi-bin/loadtool. cgi?tool=prophet)
   Agilent earray 4.5 (https://earray.chem. agilent.com/earray/)

## Claims

1. A method for detecting in a biological sample obtained from human endometrium the normalcy/abnormality situation in the receptive profile of said endometrium, **characterized in that** it comprises:
a) performing the extraction and purification of mRNA of an endometrial biopsy of the fundus of the uterus of a woman 7 days after her endogenous LH surge, which is equivalent to the phase of day 20-21 of the menstrual cycle;
b) determining in said sample the expression profile of the set of the genes involved in endometrial receptivity according to Figure 1, by means of microarray technology;
c) detecting in said biopsy the expression profile of said genes involved in endometrial receptivity; and
d) analyzing said expression profile of the genes by means of a computer software containing a specific prediction model which classifies and determines the state of the endometrium depending on the gene profile with the established criteria.

2. The method according to claim 1, **characterized in that** the endometrial sample is contacted with an oligonucleotide probe which is complementary to a region of the gene the expression of which is quantified.

3. The method according to claims 1-2, **characterized in that** the expression profile fits the one established by the prediction model once the expression pattern of the set of genes has been established during the window of implantation days 20 and 21 and that obtained on other days of the cycle outside of receptivity.

4. The method according to claims 1-3, **characterized in that** the situation of abnormality is caused by subfertility situations or due to an endometrial cause, such as the failure of the implantation or hydrosalpinx.

5. The method according to claims 1-4, **characterized in that** the normalcy/abnormality situation in the receptivity profile of the endometrium is due to the effect of drugs or inert devices, or in combination with drugs which alter the normalcy/abnormality situation.

6. Use of the method according to claims 1-5 for detecting in a biological sample the effect of drugs which alter the normalcy/abnormality situation in the receptive profile of an endometrium.

## Patentansprüche

1. Verfahren, um in einer biologischen, von einem menschlichen Endometrium erhaltenen Probe die Normalitäts- / Abnormalitätssituation in dem Rezeptivitäts-Profil des genannten Endometriums festzustellen, **dadurch gekennzeichnet, dass** es umfasst:
a) Durchführung der Extraktion und der Reinigung des mRNA einer endometrialen Biopsie des Gebärmuttergrunds einer Frau 7 Tage nach der endogenen Freisetzung des LH, was das Äquivalent der Phase des Tages 20-21 des Menstruationszyklus ist;
b) Bestimmung des Expressionsprofils des Satzes der in der endometrialen Rezeptivität involvierten Gene in der genannten Probe gemäß Figur 1 mittels Bio-Chip-Technologie;
c) Feststellung des Expressionsprofils der genannten, in der endometrialen Rezeptivität involvierten Gene in der genannten Biopsie; und
d) Analyse des genannten Expressionsprofils der Gene mittels einer Computer-Software, die ein spezifisches Voraussagemodell enthält, das den Status des Endometriums in Abhängigkeit von dem Genprofil mit den festgelegten Kriterien einordnet und bestimmt.

2. Verfahren gemäß Anspruch 1, **dadurch gekennzeichnet, dass** die endometriale Probe mit einer Oligonucleotid-Sonde kontaktiert ist, die komplementär zu einer Region des Gens ist, dessen Expression quantifiziert ist.

3. Verfahren gemäß Anspruch 1 - 2, **dadurch gekennzeichnet, dass** das Expressionsprofil zu dem, das durch das Voraussagemodell festgelegt ist, sobald das Expressionsprofil des Gensatzes während des Implementierungsfensters der Tage 20 und 21 festgelegt ist, und das, das an anderen Tagen des Zyklus außerhalb der Rezeptivität erhalten wird, passt.

4. Verfahren gemäß Anspruch 1 - 3, **dadurch gekennzeichnet, dass** die Situation der Abnormalität durch Subfertilitätssituationen verursacht wird oder auf eine endometriale Ursache zurückzuführen ist, wie z. B. der Ausfall der Einnistung oder ein Hydrosalpinx.

5. Verfahren gemäß Anspruch 1 - 4, **dadurch gekennzeichnet, dass** die Normalitäts- / Abnormalitätssituation in dem Rezeptivitätsprofil des Endometriums auf die Wirkung von Medikamenten oder inerten Vorrichtungen oder in Kombination mit Medikamenten, die die Normalitäts - / Abnormalitätssituation verändern, zurückzuführen ist.

6. Verwendung des Verfahrens gemäß Anspruch 1 - 5 zur Feststellung der Wirkung von Medikamenten, die die Normalitäts- / Abnormalitätssituation in dem Rezeptivitäts-Profil eines Endometriums verändern, in einer biologischen Probe.

## Revendications

1. Procédé de détection dans un échantillon biologique obtenu à partir d'un endomètre humain de la situation de normalité/d'anormalité dans le profil de réceptivité dudit endomètre, **caractérisé en ce qu'**il comprend les étapes suivantes:
a) procéder à l'extraction et à la purification de l'ARN messager d'une biopsie endométriale du fond de l'utérus d'une femme 7 jours après sa libération de LH endogène, qui est équivalente à la phase des jours 20-21 du cycle menstruel;
b) déterminer dans ledit échantillon le profil d'expression de l'ensemble de gènes impliqués dans la réceptivité endométriale selon la Figure 1, au moyen de la technologie de biopuces;
c) détecter dans ladite biopsie le profil d'expression desdits gènes impliqués dans la réceptivité endométriale; et
d) analyser ledit profil d'expression des gènes au moyen d'un logiciel informatique contenant un modèle de prédiction spécifique qui classifie et détermine l'état de l'endomètre en fonction du profil génétique avec les critères établis.

2. Procédé selon la revendication 1, **caractérisé en ce que** l'échantillon endométrial est mis en contact avec une sonde oligonucléotidique qui est complémentaire à une région du gène dont l'expression est quantifiée.

3. Procédé selon les revendications 1 et 2, **caractérisé en ce que** le profil d'expression coïncide avec celui établi par le modèle de prédiction une fois que le profil d'expression de l'ensemble de gènes a été établi pendant la fenêtre des jours d'implantation 20 et 21 et avec celui obtenu d'autres jours du cycle en dehors de la réceptivité.

4. Procédé selon les revendications 1 à 3, **caractérisé en ce que** la situation d'anormalité est causée par des situations d'hypofertilité ou est due à une cause endométriale, telle que l'échec de l'implantation ou un hydrosalpinx.

5. Procédé selon les revendications 1 à 4, **caractérisé en ce que** la situation de normalité/d'anormalité dans le profil de réceptivité de l'endomètre est due à l'effet de médicaments ou de dispositifs inertes, ou en combinaison avec des médicaments qui altèrent la situation de normalité/d'anormalité.

6. Utilisation du procédé selon les revendications 1 à 5 pour détecter dans un échantillon biologique l'effet de médicaments qui altèrent la situation de normalité/d'anormalité dans le profil de réceptivité d'un endomètre.
